(19) Europäisches Patentamt
European Patent Office
Office européen des brevets

(11) Publication number: **0 680 988 A2**

(12) # EUROPEAN PATENT APPLICATION

(21) Application number: **95110663.2**

(22) Date of filing: **29.01.85**

(51) Int. Cl.[6]: **C08G 71/00**, C09D 201/00, C09D 5/44

This application was filed on 07 - 07 - 1995 as a divisional application to the application mentioned under INID code 60.

(30) Priority: **17.02.84 US 581006**
**17.02.84 US 581015**
**17.02.84 US 581011**
**17.02.84 US 581007**
**17.02.84 US 581005**
**17.02.84 US 581009**
**17.02.84 US 581010**
**17.02.84 US 581012**
**17.02.84 US 581008**
**17.02.84 US 581013**

(43) Date of publication of application:
**08.11.95 Bulletin 95/45**

(60) Publication number of the earlier application in accordance with Art.76 EPC: **0 152 820**

(84) Designated Contracting States:
**AT BE CH DE FR GB IT LI NL SE**

(71) Applicant: **CYTEC TECHNOLOGY CORP.**
**1105 North Market Street,**
**Suite 1300**
**Wilmington,**
**Delaware 19801 (US)**

(72) Inventor: **Parekh, Girish Girdhar**
**11 Four Seasons Road**
**Fairfield,**
**Connecticut 06432 (US)**
Inventor: **Jacobs III, William**
**58 Wilson Street**
**Bridgeport,**
**Connecticut 06605 (US)**
Inventor: **Blank, Werner Josef**
**36 Grey Rocks Road**
**Wilton,**
**Connecticut 06894 (US)**

(74) Representative: **DIEHL GLAESER HILTL & PARTNER**
**Flüggenstrasse 13**
**D-80639 München (DE)**

(54) **Carbamate compounds and coating compositions comprising same.**

(57) Described are carbamates and coating compositions comprising same. Specifically disclosed is a coating composition comprising:

(a) an hydroxyalkyl carbamate compound of the formula

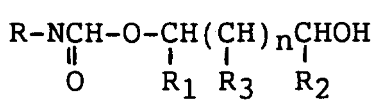

wherein R is a $C_1$ to $C_{20}$ organic moiety which may contain one or more constituents selected from the class consisting of heteroatoms and hydroxyl groups, and each of $R_1$, $R_2$ and $R_3$ is independently H or $CH_3$;

(b) an amino cross-linker;

(c) a polymer containing active sites which, at elevated temperatures, are reactive with the amino cross-linker (b); and

(d) optionally, an acid catalyst;

the compound (a), the cross-linker (b) and the polymer (c) being stable relative to each other in the composition

EP 0 680 988 A2

while at an ambient temperature, and reactive at elevated temperature.

The present invention concerns carbamates and coating compositions comprising same.

The reaction of propylene carbonate with primary and secondary amines to produce corresponding 2-hydroxypropyl carbamates is known in the art (Comp. rend, 1142, 1954). Similar reactions of ethylene carbonate are exemplified by the article, "The Preparation of Polymeric and Cyclic Urethans and Ureas from Ethylene Carbonate and Amines" by Elizabeth Dyer and Harvey Scott, J.A.C.S. (1956) pp. 672 - 675. See also the report "Polyurethane elastomers obtained without the use of diioscyanates" by L. Ya. Rappoport, G.N. Petrov, I.I. Trostyanskaya and O.P.D. Gavrilova in International Polymer Science and Technology, 8, No. 1, 1981. The Dyer-Scott reference discloses that polyurethanes might be prepared from 2-(hydroxyethyl)- carbamate by elimination of ethylene glycol, thereby avoiding the need for using diisocyanates. The Rappoport et al paper discloses generally the reaction of cyclic carbonates with amines to form polyurethane elastomers. Thus, the prior art shows an awareness that amines react with, e.g., propylene carbonate, to yield the corresponding hydroxyalkyl carbamates. The Journel of Polymer Science, Vol. 7, 899 916 (1969), in an article entitled "New Method for Preparing Saturated and Unsaturated Aliphatic Polyurethanes" by Y. Mizake, S. Ozaki and Y. Hirata, at pages 899 - 915, discloses alternate routes to saturated and unsaturated polyurethanes, including polycondensation reaction of glycol bis(chloroformate) with diamine.

An article by Richard D. Cowell entitled: "Thermoplastic Polyurethane Elastosmers: Chemistry Properties and Processing for the 80's in the Journal of Elastomers and Plastics, Vol. 14, (October, 1982) pages 195 - 203, discloses the preparation of bis(2-hydroxyethyl) carbamates by reaction of diamines with ethylene carbonate followed by a catalyzed transesterification reaction with a glycol or macroglycol. The two types of diamines were used, 1, 4 cyclohexane - bis- (methyl-amine) and a product sold under the trademark Jeffamine D2000 comprising a diamine with a polyoxypropylene backbone.

Coating compositions comprising a cross-linker and a backbone polymer containing sites thereon which are reactive with the cross-linker at elevated temperature to form a cross-linked polymeric material, but which are stable relative to each other at ambient temperatures are of course well known in the art. One difficulty with such compositions is that the high viscosity and high softening temperature of backbone polymers typically employed requires the utilization of a solvent to reduce viscosity of the polymer. After application of the coating composition and heating it to cure, volatilization of the solvent produces environmental, health and processing problems.

In order to achieve high solids content coatings compositions, it is known to use high boiling point diols or polyols as reactive diluents in the compositions. However, in acid-catalyzed paint or coating formulations containing amino cross-linkers, the presence of diols or polyols reduces the shelf life of the coating formulation because the hydroxy groups on the polymer react with the amino cross-linkers.

The use of blocked or capped isocyanates as cross-linking agents which are stable or nonreactive with functional groups such as hydroxyl or amine groups at room temperature, but which react to cross-link therewith at elevated temperature, is known in the art. For example, U.S. Patent 3,984,299 of Robert D. Jerabek discloses an electrodepositable composition comprising an amine adduct of an epoxy group containing resin, a capped or blocked organic polyisocyanate and, optionally, a catalyst for urethane formation. The blocked isocyanate is disclosed as being any isocyanate in which the isocyanate groups have been reacted with a compound such as an aliphatic, cycloaliphatic, aromatic alkyl monoalcohol or phenolic compound.

The art shows generally the use of blocked isocyanates as cross-linking agents in coating applications such as powder coatings, electrodepositable coatings, and aqueous and organic solvent-borne coatings. Blocked isocyanates are usually prepared by reacting a blocking agent, such as monoalcohols, phenols, lactams, oximes, beta-dicarbonyl compounds, triazoles, hydroxamic acid esters and the like, with aliphatic or aromatic polyisocyanates. See, for example, Progress in Organic Coatings Review, 9, 1981 Z. Wicks.

One disadvantage associated with the utilization of blocked isocyanate compounds is the necessity of storing and handling these toxic compounds. Another difficulty is the relatively high cost of suitable isocyanate compounds as compared to the compounds utilized in the present invention.

Electrodepositable resin compositions are of course well known in the art. For example, U.S. Patent 4,031,050 discloses cationic electrodepositable compositions of blocked organic polyisocyanates and an amine adduct of an epoxy resin. As disclosed in this patent, electrodeposition of such compounds, which may optionally contain a catalyst for urethane formation, can be carried out to provide coatings on a conductive substrate, which coatings have desirable properties. In this regard, see also U.S. Patents 3,984,299 and 4,031,050. However, isocyanate compounds are toxic and highly reactive, requiring the taking of suitable precautions in handling and storing the same.

U.S. Patent 4,017,438 discloses an epoxy resin- derived, cationic electrodepositable resin enhanced by the incorporation of primary amine groups into the resin molecule, by reacting certain polyamine compounds having primary amine groups blocked by ketimine. The ketimine groups when contacted with water, will decompose to provide primary amine functionality as disclosed in this patent. Capped isocyanates are disclosed in combination with the amine-resin adduct to provide, together with a suitable catalyst a cationically electrodepositable resin system. The electrodeposited coating, upon being heated to an elevated temperature, usually in the presence of a cross-linking catalyst, undergoes cross-linking through urethane, hydroxy and amino groups.

As well known, the "capped" or "blocked" isocyanates react with hydroxyl groups and amino groups under conditions of elevated temperature to form urethane and urea cross linkages.

Coating systems based on organic solvent-based materials, such as isocyanate systems, are available which provide high performance, urethane cross-linked coatings but engender enviornmental and fire hazards because of the use of volatile or toxic organic solvents. Commercially available isocyanate compounds typically are toxic and highly reactive, requiring the taking of suitable precautions in handling and storing the same. Aqueous solutions or dispersions of polyurethanes for coatings are known, but these known systems usually require high curing temperatures on the order of 350 to 600°F(176 to 315° C) in order to obtain cross-linking through the urethane groups. Although other low temperature-, or even room temperature-curable aqueous solutions or dispersions of isocyanate-free polyurethanes are available, such coatings do not cross-link through urethane groups and therefore are not likely to meet the performance standards attainable by urethane cross-linked coatings. Most aqueous dispersions of polyurethanes are usually attained by the addition of acins to form cationic dispersions or by the addition of bases to form anionic dispersions, or by the addition of surfactants, all of which additives can adversely affect the properties of the cured film obtained thereby. For example, the aqueous cationic-, anionic-, or surfactant-dispersed isocyanate-based polyurethanes often suffer from a lack of stability upon aging. If there are -NCO groups present, a reaction between water and the isocyanate will usually take place within about three to twenty hours at room temperature. Thus, isocyanate-based polyelectrolytes which are fully soluble in water either readily hydrolyze in water or, after removal of water, become brittle and hygroscopic. Because of these drawbacks occasioned by the high ion group content of such materials, they are not of significant practical importance in the field of coatings and plastics generally.

One class of non-ionic aqueous solutions of polyurethanes is based upon the incorporation of polyester-glycol or polyether-glycol segments. However, the polyester-glycol types are sensitive to hydrolytic degradation while the water solubility of the polyether-glycol based resins is isocyanate-dependent. Moreover, both types tend to yield cured films with excessive sensitivity to water, i.e., films which are subject to swelling, turbidity (turning white), softening, and variable adhesion upon exposure to water.

It has now been found that multi-functional amines containing at least one primary and at least one hindered secondary amine group are, unexpectedly, selectively reactive with cyclic carbonates at the primary amine groups, leaving one or more secondary amine groups unreacted in the resulting hydroxyalkyl carbamate. The resultant hydroxyalkyl carbamate contains at least one unreacted secondary amine group and is a useful intermediate in the preparation of polymers which are cross-linkable through hydroxyalkyl carbamate groups.

Generally, in accordance with a first aspect of the present invention, there is provided an intermediate compound comprising at least one hydroxyalkyl carbamate group and at least one unreacted secondary amine group, and a method of making the same. The compounds of the invention are further described by the general and specific formulas given in the description below, which should be deemed to include the isomers of the described compounds and mixtures thereof.

A second aspect of the present invention concerns self-cross-linkable polymers which contain at least two hydroxyalkyl carbamate groups per molecule and monomers which contain at least one hydroxyalkyl carbamate group and which are polymerizable to form the self-cross-linkable polymers.

A third aspect of the present invention provides (1) a reactive diluent useful in coating compositions and which is obtained by reacting a cyclic carbonate and a primary amine to provide an hydroxyalkyl carbamate compound, and (2) compositions which contain one or more hydroxyalkyl carbamate compounds as a reactive diluent.

In a fourth aspect the present invention concerns coating compositions comprising hydroxy group-containing polyurethane or polyurea compounds and an amino cross-linking agent.

In a fifth aspect of the present invention, there is provided a thermosettable composition comprising (a) a cross-linking agent containing at least two hydroxyalkyl carbamate groups which may be obtained as the reaction product of an amine with a cyclic carbonate; (b) a polymer containing two or more active functional groups; and (c) optionally, a cross-linking catalyst; the cross-linking agent (a) and the polymer (b) being stable relative to each other in the composition at ambient temperature and reactive with each other at elevated temperature.

In a sixth aspect of the invention, there is provided a cathodically electrodepositable, self-cross-linkable polymer containing hydroxyalkyl carbamate groups and one or more tertiary amine groups per molecule. The polymer may be obtained as the reaction product of (a) an epoxy resin having an average epoxy equivalent weight of from about 300 to about 10,000, preferably from about 1,000 to about 4,000 and (b) one or more amines having at least one secondary amine group and at least one hydroalkyl carbamate or precursor thereof.

In a seventh aspect of the present invention there is provided a cathodic electrocoating composition comprising (a) a hydrophobic cross-linking agent having at least two carbamate groups selected from the class consisting of one or both of (i) hydroxyalkyl carbamate groups, and (ii) alkyl carbamate groups obtained by capping the reaction product of a cyclic carbonate (such as ethylene carbonate or propylene carbonate) and a polyamine; (b) a water-soluble and hydrophobic amino group-containing polymer; and (c) an acid disperser (such as an inorganic acid or hydrophilic organic acid) effective to provide cationic groups in the polymer (b) whereby the polymer is rendered water-dispersible; and (d) optionally, a cross-linking catalyst; the cross-linking agent (a) and the polymer (b) being stable relative to each other in the composition while at ambient temperature, and reactive with each other at elevated temperature.

In an eigth aspect of the present invention, there is provided a hydrophilic, substantially epoxy-free self-cross-linkable polymer which contains hydroxyalkyl carbamate groups and one or more tertiary amine groups.

In accordance with a ninth aspect of the present invention, there is provided a self-cross-linkable acrylic polymer which contains at least two hydroxyalkyl carbamate groups per molecule.

In accordance with a tenth aspect of the invention, there is provided a bonding system for bonding workpiece surfaces to one another comprising a primer coating on the workpiece surfaces and a structural adhesive between the primer coatings, the primer coating comprising a cured polymer having hydroxyalkyl carbamate groups and at least one tertiary amine group thereon and cross-linked by reaction of the hydroxyalkyl carbamate groups.

Generally, compounds of the first aspect of the invention are made by reacting one or more amines and cyclic carbamates to yield compounds having the formula:

(1)

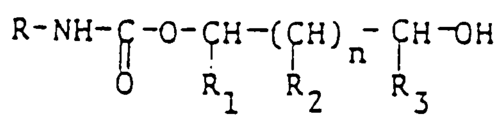

wherein R is an organic moiety having at least one unreacted secondary amine group, each of $R_1$, $R_2$ and $R_3$ is independently H or $C_1$ to $C_{20}$ alkyl, cycloalkyl or alkyl aromatic moiety or any such moiety containing, in addition to at least one carbon atom, one or more heteroatoms, and n is 0 or 1. R may also contain one or more heteroatoms. Such moieties containing one or more heteroatoms include, for example, those containing ether groups, thio groups and organo-silcon moieties.

The cyclic carbonate to be reacted with the multi-functional amine may comprise any suitable cyclic carbonate, including biscarbonates, which is reactive with one or more of the primary amine groups of a multi-functional amine. Generally, five-member ring organic carbonates are preferred as compared to six-member ring organic carbonates, the latter being relatively more expensive and difficult to prepare. Accordingly, a preferred utilizable cyclic carbonate has the formula:

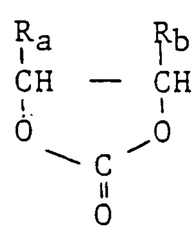

wherein $R_a$ and $R_b$ may be the same or different, and each may comprise H, or a $C_1$ to $C_8$ aliphatic, cycloaliphatic, aromatic or heterocyclic compound. Ethylene carbonate (dioxolane-2-one), both $R_a$ and $R_b$ = H, and propylene carbonate (4-methyldioxolane-2-one), $R_a$ = H and $R_b$ = $CH_3$, are preferred reactants.

The multi-functional amine contains at least one secondary amine group which is hindered with respect to reacting with the cyclic carbonate and at least one primary amine group. As used herein and in the claims, (a) "multi-functional amine" means an amine containing at least one primary and at least one hindered secondary amine group; and (b) "hindered secondary amine group" means a secondary amine group which is inhibited, sterically, electronically or otherwise, with respect to reacting with the cyclic carbonate under conditions at which the primary amine group will react. It has been discovered that, suprisingly, the well known reactivity of primary and secondary amines with cyclic carbonates is highly selective to the primary group for certain multi-functional amines. Stated otherwise, it has been found that certain multi-functional amines have secondary amine groups which are sterically or otherwise inhibited from reacting with a cyclic carbonate, and yet are reactive with, for example, epoxy groups or other functional groups available on backbone polymers. Thus, hindered secondary amine groups contained in multi-functional amines enable the formation, with one or more cyclic carbonates, of hydroxyalkyl carbamates in which secondary amine groups remain unreacted and available to react with epoxy or other active groups. This enables compositions to effectively serve as a means of anchoring hydroxyalkyl carbamate groups by reaction of the compositions with epoxy or other active groups on backbone polymers. For example, when ethylene and propylene carbonates were reacted with diethylenetriamine, they reacted selectively with the primary amine groups of the triamine to form carbamate groups while leaving the secondary amine groups unreacted. Such secondary amine groups can then be reacted with, for example, epoxy groups on backbone polymers, without affecting the carbamate groups.

Further, such compounds can be used to prepare polymers containing hydroxyalkyl carbamate groups which can self-cross-link to yield thermoset polyurethanes suitable for a number of applications in the areas of coatings. The self-cross-linking reaction may be base catalyzed or tin-catalyzed, which offers significant advantages over prior amino resin systems which require acid catalyzation. Thus, the present invention enables the utilization of a system which is free of formaldehyde and in which cure inhibition in the presence of hindered amine ultraviolet stabilizers is avoided.

Before discussing in detail the preparation of the hydroxyalkyl carbamate-containing compounds of the present invention, the use thereof may be illustrated as follows. The hydroxyalkyl carbamate-containing compounds may be reacted with any suitable "backbone" polymer containing active groups, such as, for example, epoxy groups, in which case the reaction may be represented as

(3)

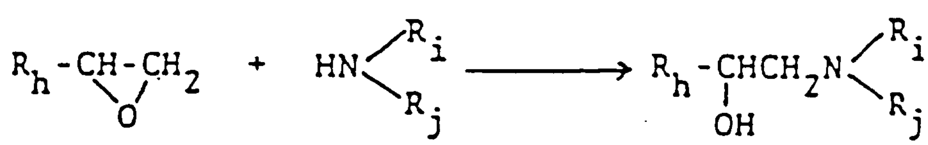

where $R_h$ is a fragment of an epoxy-containing resin and $R_i$ and $R_j$ are fragments of the hydroxyalkyl carbamate-containing amine or polyamine compounds of the invention. The reaction usually occurs at room or slightly elevated temperatures and is often exothermic. The reaction may be performed without a solvent, otherwise aprotic or alcohol solvents may be used. Any of numerous types of backbone polymers having any of a variety of reactive functional groups thereon may also be used, as described in more detail below. For example, a typical polymer having anchored thereon one or more of the compositions of the invention may have the formula

(4)

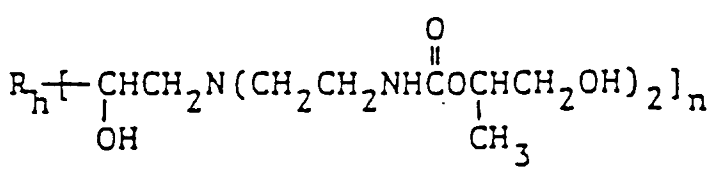

The resultant polymer, upon heating and, optionally, in the presence of a suitable cross-linking catalyst, will cross-link through one or more mechanisms, as follows: by cross-linking through backbone hydroxyl groups, e.g.,

(5) 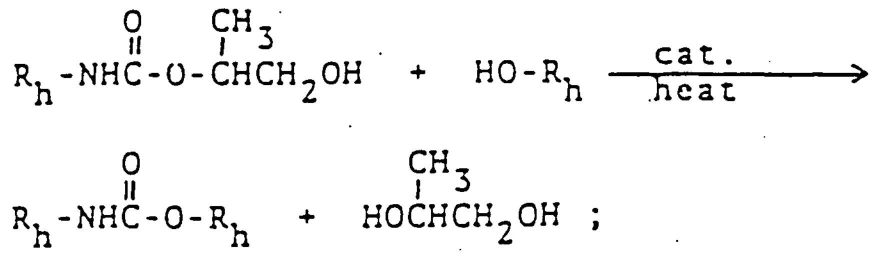

by cross-linking through self-condensation, e.g.,

(6) 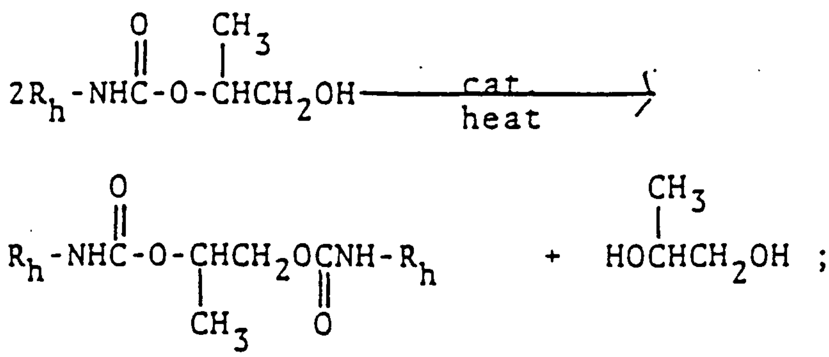

and by cross-linking through backbone amine groups, e.g.,

(7) 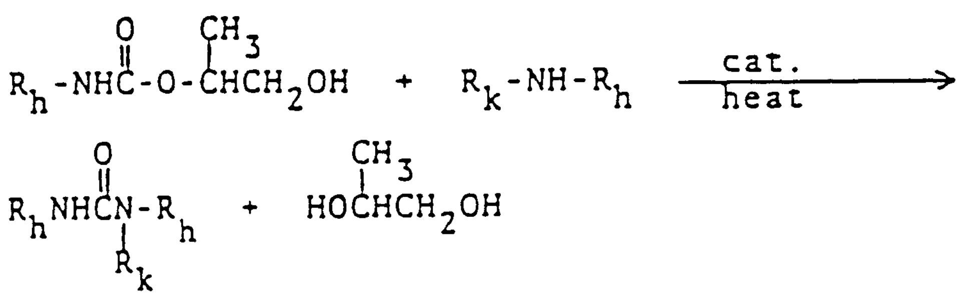

wherein $R_k$ is hydrogen or a fragment of the backbone polymer.

The compounds of the present invention, in addition to having utility as intermediates in the preparation of self-cross-linkable polymers, find utility in their ability to be self-condensed in the absence of backbone polymers to form coherent films which, although but poorly resistant to water, display good resistance to organic solvents.

A wide range of multi-functional amines are utilizable in the present invention to react with the cyclic carbonate inasmuch as, as stated above, it is necessary only that the multi-functional amine contain at least one primary and one hindered secondary amine. For example, one class of multi-functional amines utilizable in the present invention may be represented by the formula:

$$R_c\text{-}(NH\text{-}R_d)_{n_2}\text{-}NH\text{-}R_e$$

wherein $n_2$ is 0 to 5, and each of $R_c$, $R_d$ and $R_e$ is independently a straight chain or branched hydrocarbon fragment having 2 to 6 carbon atoms each and at least one of $R_c$ and $R_e$ contains a primary amine group.

The formulas of suitable classes of amines may be derived simply by replacing with $-NH_2$ the hydroxyalkyl carbamate moieties of the compounds set out in formulas (8) to (11) below.

(8) 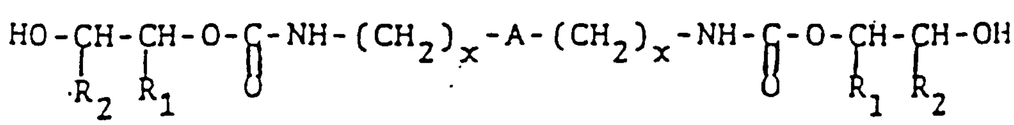

wherein A is

$$[NH\ (CH_2)_n]\ NH,$$

n is 0 to 10, each x is independently 2 to 6, preferably 2; each of $R_1$ and $R_2$ is independently H, or a $C_1$ to $C_{20}$ alkyl or alkyl aromatic moiety, preferably, H or $CH_3$. Preferably, n is not more than 10 and is preferably from 0 to 6, more preferably from 0 to 4. Compositions in which x is 2 or 6 may readily be made from widely available reactants, i.e., diethylenetriamine and dihexamethylenetriamine and to that extent are preferred.

Another suitable class of compounds is represented by the formula:

(9) 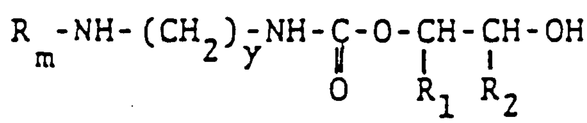

wherein y is 2 or 3, each of $R_1$ and $R_2$ is as defined above, and $R_m$ is a $C_1$ to $C_{20}$ alkyl, cycloalkyl or alkyl aromatic moiety, or any such moiety containing, in addition to at least one carbon atom, one or more heteroatoms. In this class of compounds, starting materials which would provide alkyl, cycloalkyl or alkyl aromatic moieties greater than $C_{20}$ are not readily available. The $C_1$ to $C_{20}$ alkyl, cycloalkyl or alkyl aromatic moieties of all the formulas given herein, and somewhat shorter chains, i.e., $C_1$ to $C_{18}$ alkyl or alkyl aromatic moieties, are to that extent preferred.

Another class of suitable compounds is represented by the formula:

(10) 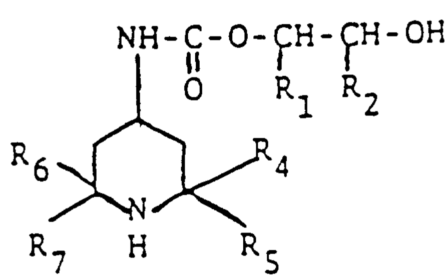

wherein each of $R_1$ and $R_2$ is as defined above, each of $R_4$ and $R_6$ is independently H or a $C_1$ to $C_4$ alkyl moiety and each of $R_5$ and $R_7$ is independently a $C_1$ to $C_4$ alkyl moiety. Preferably, $R_1$ and $R_2$ are independently H or $CH_3$ and each of $R_4$, $R_5$, $R_6$ and $R_7$ is $CH_3$.

Another suitable class of compounds in accordance with the invention has the formula:

(11) 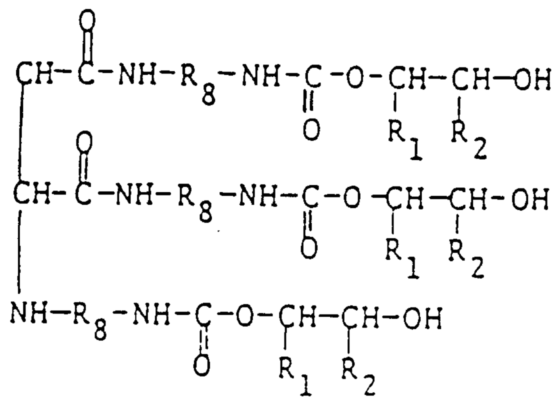

wherein each of $R_1$ and $R_2$ is as defined above and each $R_8$ is independently a $C_2$ to $C_6$ alkylene moiety, preferably $-(CH_2)_2$ - or $-(CH_2)_6$-.

As indicated above, the polymers of the invention can be prepared by reacting either backbone polymers or monomers containing suitable functional groups with primary or secondary amines containing one or more hydroxyalkyl carbamate groups or precursors thereof. When monomers are thus employed, they are polymerized, after reaction with the amines, either by homopolymerization or copolymerization with other monomers, to provide the polymers of the invention. As used herein, "suitable" functional groups simply means those functional groups, such as epoxy, isocyanato, methylol, etc., which are reactive with the primary or secondary amine groups of the hydroxyalkyl carbamate-containing amines. For example, an hydroxyalkyl carbamate-containing monomer may be prepared by reacting glycidyl methacrylate with an hydroxyalkyl carbamate-containing secondary amine. The resulting monomer may then by home- or copolymerized to provide a polymer of the invention.

The repeating units of which the polymer is comprised may fall into two categories, one comprising units containing the suitable amine-reactive functional groups and the other comprising modifying units selected to impart desired film-making or other properties to the polymer and the finished coating or other product made therefrom. Any suitable repeating units may be employed in the polymer in any desired combination provided that there exist in the polymer sufficient suitable reactive functional groups for attachment thereto of the primary or secondary amines utilized in the reaction.

In an alternate method of preparing the polymer of the invention, the monomers or backbone resins are reacted with amines which contain, in addition to one or more secondary amine groups, hydrolyzable blocked primary amine groups, for example, ketimine groups in lieu of some or all of the hydroxyalkyl carbamate groups. After reaction of the secondary amine groups with the reactive functional groups as described above, so that the amine groups are pendant upon the monomer or backbone resin, the hydrolyzable blocked primary amine groups are hydrolyzed to form free amine groups and one or more suitable cyclic carbonates, for example, are then added to the mixture to react with the resultant free amine groups. Thus, the multi-functional amine utilized to form the hydroxyalkyl carbamate will contain either an amine group reactable with a cyclic carbonate or a hydrolyzable blocked primary amine group which is convertible to an amine group reactable with the cyclic carbonate.

A wide variety of suitable monomers containing functional groups or sites which are reactive with primary or secondary amines may be employed to react with the hydroxyalkyl carbamate-containing amines, to form the monomers containing hydroxyalkyl carbamate groups. Such monomers include, by way of example and not limitation, glycidyl methacrylate, glycidyl acrylate, isocyanatoethylmethacrylate, maleic anhydride, methacryloyl chloride, n-methyloylacrylamide, 1-(1-isocyanato -1- methylethyl) -3- (1-methylenethenyl)benzene, 1-(1-isocyanato -1- methylethyl) -4- (1-methylethenyl) benzene, methyl acrylamidoglycolate, methyl acrylamidoglycolate methyl ether, acryloyl chloride and chloromethyl styrene. Thus, the repeating units of the polymer may be derived from one or more of the foregoing monomers to provide one or more functional groups of the polymer as described below.

A wide variety of backbone polymers containing suitable functional groups may also be employed to react with the hydroxyalkyl carbamate-containing amines to provide the polymers of the invention. The choice of suitable monomers or polymers will depend on the characteristics desired for the finished coating or other product. Among suitable backbone polymers are epoxy resins generally, including polybutadiene-modified epoxy resins, acrylic resins, polybutadiene resins and polyester resins.

In order to provide a site on the backbone resin or monomer on which the amines may be anchored, each molecule of such backbone resin or monomer should have at least one, and preferably more, reactive

sites thereon which can react with the secondary amine group of the hydroxyalkyl carbamate-containing amine of the invention. Such reactive sites may include, without limitation, one or more of the following groups:

(12)

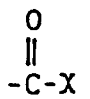

(Acid halides, in which X is a halogen, preferably Cl,Br or I)

(13)

$-CH_2X$

(Halogenated Aliphatics, in which X is a halogen, preferably Cl, Br or I)

(14)

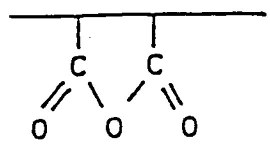

(Anhydrides)

(15)

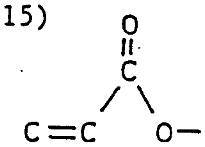

(alpha, beta unsaturated esters)

(16)

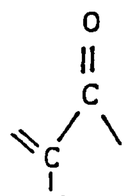

(alpha,beta unsaturated ketones)

(17)

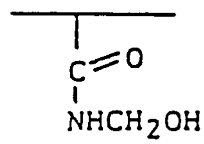

( N-Methylolamides)

(18)

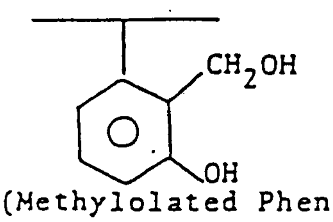

(Methylolated Phenols)

(19)

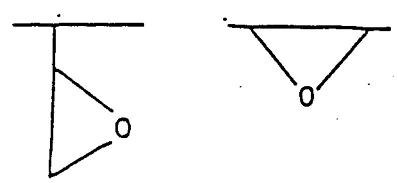

(Epoxies)

(20)

—NCO

(Isocyanates)

(21)

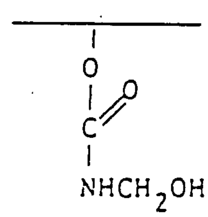

(N-Methylol Carbamates)

(22)

— $SO_2X$

( in which X is a halogen preferably Cl, Br or I)

One suitable class of reactive sites on a monomer or polymer is epoxy groups, for example, suitable resins are acrylic resins with pendant glycidyl ether groups, epoxy resins derived by reaction of epichlorohydrin and bisphenol-A, or epoxy resins derived by reaction of epichlorohydrin with phenol formaldehyde resins. In addition, polybutadiene resins with pendant epoxy groups are also well suited to have hydroxyalkyl carbamate-containing amines anchored thereon to provide the cross-linkable polymer of the present invention.

Reaction of secondary amine groups of the hydroxyalkyl carbamate-containing amines with monomers or polymers containing functional groups as illustrated at (12) - (22) above, will result in the formation of the following groups at the functional group sites.

(a) 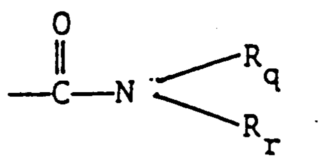

(f) 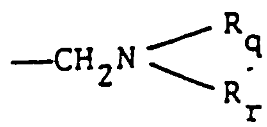

(b) 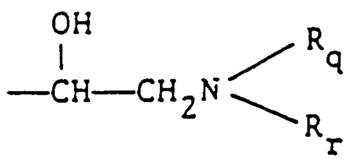

(g) 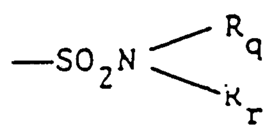

(c) 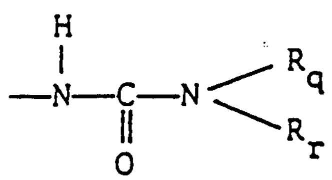

(h) 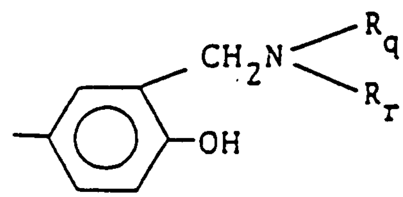

(d) 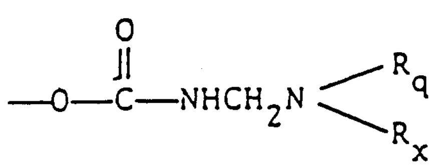

(i) 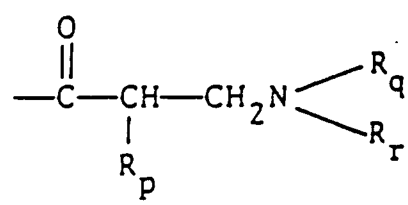

(e) 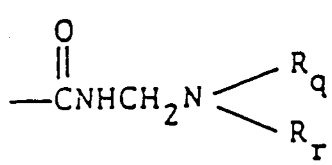

(j) 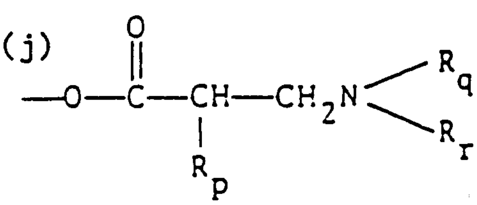

wherein $R_p$ is H or $C_1$ - $C_8$ alkyl; and $R_q$ and $R_r$ may be the same or different and are amine residues containing hydroxyalkyl carbamate moieties.

The polymer containing functional group sites may also include modifying units as mentioned above and such modifying units may be derived from one or more of acrylic acid, methacrylic acid, butadiene, styrene, alpha-methyl styrene, methyl methacrylate, tutyl acrylate, acrylonitrile, hydroxyethyl acrylate, glycidyl methacrylate, acrylamide, methacrylamide, vinyl chloride and vinylidene chloride.

Any suitable epoxide material may be utilized as the backbone resin in accordance with the invention such as a monomeric or polymeric epoxy containing material, preferably a resinous polyepoxide material containing two or more epoxy groups per molecule.

Among the known epoxides which have been found useful as backbone resins in the practice of the present invention are polyglycidyl ethers of polyphenols such as bisphenol-A or, generally, the reaction product of epichlorohydrin with a polyhydric phenol. As used herein, "polyhydric phenol" means and includes compounds such as bisphenol-A, bisphenol-F and bisphenol-S. Such epoxides may also be modified by reaction with carboxylate containing polybutadiene polymers or other modifying materials.

Polypoxides made from polyhydric phenol resins such as novalac resins or the like comprise one suitable class of compounds. Polyglycidyl esters of polycarboxylic acids, such as the reaction products of epichlorohydrin or other similar epoxy compounds with reactants such as terephthalic acid, glucaric acid, succinic acid, oxalic acid and the like may also be employed.

Multi-functional amines as described above may be reacted with, for example, a polyepoxide of one of the following formulas:

(a) 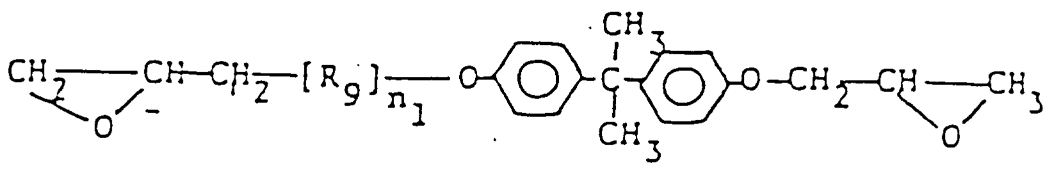

where $R_9$ is the repeating fragment

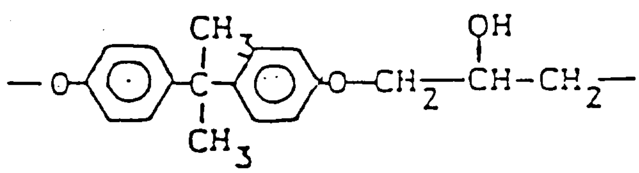

and $n_1$ is from 0 to 12, e.g., 0 to 2;

(b)

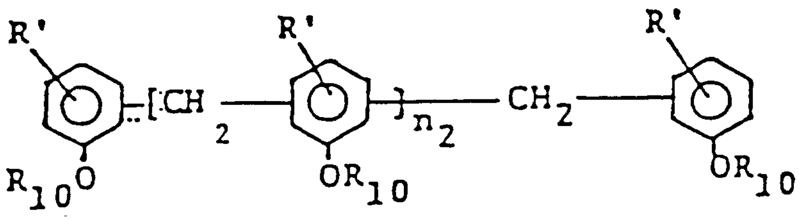

where R' is a hydrogen or a methyl group and $R_{10}$ is a hydrogen atom or a glycidyl group and $n_2$ is from 0 to 12;

(c)

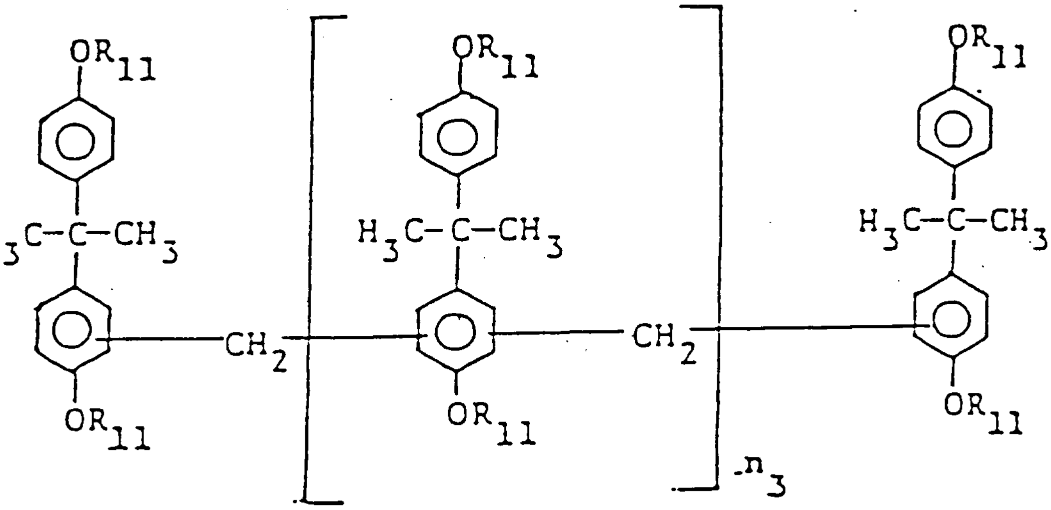

(d)

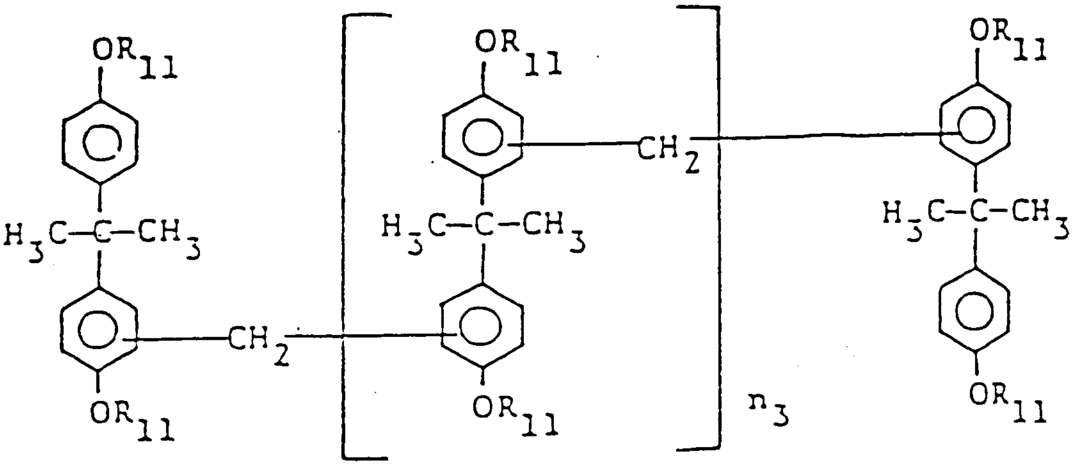

wherein, in (c) and (d), $n_3$ is independently 0 to 4, and $R_{11}$ is a hydrogen atom or a glycidal group.

When it is desired that the polymer of the present invention have good water solubility/reducibility, it is important to select backbone polymers that are of low molecular (equivalent) weight for monofunctional epoxides or of low equivalent weight for di- or polyfunctional epoxides. For high performance water soluble or reducible coatings, di- or polyfunctional epoxides are preferred. The use of such di- or polyfunctional epoxides allows for a high proportion of hydroxyalkyl carbamate groups to be incorporated into the epoxide, thereby providing a hydrophilic resin, e.g., one which is soluble or reducible in water. In addition to the epoxides described above, resins of the following formulas have been found to be well suited for the production of such water soluble/reducible resins. (e) tris (hydroxyphenyl) methane based resins of the formula:

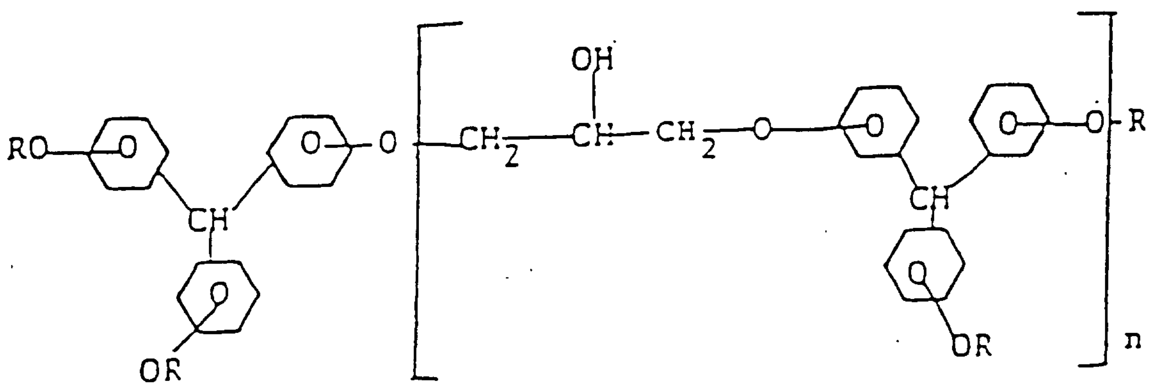

wherein, R = $-CH_2$ -CH- $CH_2$, n is 0 to about 5 and preferably from about 0 to about 0.7, on average; and (f) triglycidyl isocyanurate polyepoxy resins of the general formula:

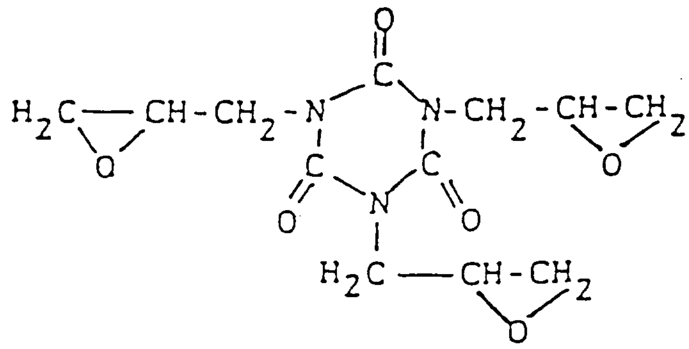

As used herein and in the claims, "tris (hydroxyphenyl) methane based resin" means a resin of formula (e) above wherein n is 0 to 5, and "triglycidyl isocyanurate polyepoxy resin" means a resin of formula (f) above.

Ideally, all reactive epoxy groups will react with a secondary amine group to attach the amine to the epoxide and provide a substantially epoxy-free polymer. However, this is less important for electrodepositable polymers wherein it is feasible to consume a small excess of secondary amine groups by reacting the polymer with mono-epoxides and to consume a small excess of epoxy groups by racting the polymer with suitable amines.

Carboxylic acid-terminated polybutadienes may be reacted with epoxy resins to yield butadiene-modified epoxy resins which may also be employed as backbone resins to prepare the polymers of this invention. Such butadiene-modified epoxy resins are described in SAMPE Quarterly, Vol. 6, No. 4,1975, the disclosure of which is incorporated by reference herein.

Any suitable acrylic resin may be utilized as the backbone resin in accordance with the present invention. Any one of a variety of one or more monomers may be utilized to prepare an acrylic resin, and it is preferred that at least one of the monomers utilized contains at least one epoxy group. Such monomers include, by way of illustration and not limitation, styrene, substituted styrenes, alphamethyl styrene, alkyl acrylates such as butyl acrylate, butyl methacrylate, ethyl acrylate, 2-ethyl hexyl acrylate, hydroxyethyl acrylate, hydroxyethyl methacrylate, acrylamide, methyl methacrylate, acrylonitrile, glycidyl methacrylate, glycidyl acrylate, vinyl acetate, etc.

The backbone resin may have other functional sites, such as, for example, epoxy, hydroxy and amide groups. It is preferred that the polymer of the invention, and hence the backbone resin, have as low an acid content as possible in order to facilitate low temperature cure of the polymer containing hydroxyalkyl, e.g., hydroxyporpyl and/or hydorxyethyl, carbamate groups.

The polymers of the invention can be modified by reacting them with selected organic secondary amines (in addition to the hydroxyalkyl carbamate-containing amines) such as diethylamine, morpholine, n-methylaniline, and the like. The selection and combination of specific modifying secondary amines of course depends on the end use of the polymer. For example, in the preparation of water-reducible polymers one or more hydrophilic secondary amines may be reacted with some of the functional sites on the backbone resin in order to enhance hydrophilicity of the polymer. Conversely, for applications where hydrophobicity of the polymer is desired, for example, for electrodepositable coatings, it may be necessary or desirable to react one or more hydrophobic secondary amines with the backbone resin to impart the desired degree of hydrophobicity to the polymer. Generally, the polymers of the invention are well suited for utilization in the field of coatings and in such case have molecular weights in the range of from about 300 to about 100,000. The urethane cross-linkable polymers of the invention are particularly well suited for a variety of uses in the field of coatings, such as solvent or water based coatings, powder coatings, electrocoating compositions, spray roller and dip type coatings, and the like. Such coatings are normally applied to a substrate such as a metal, textile, plastic or paper. The thermosetting resins of the invention may be used to prepare urethane coatings which are organic solvent, abrasion and water resistant, as well as adhesives and laminating resins.

The compositions of the third aspect of the present invention comprise three essential and one optional components which may be present in a composition which has good shelf life at ambient temperature and which may be applied to a workpiece or substrate by conventional techniques such as brush, spray, roller

or dipping applications. The applied coating is then heated to an elevated temperature for a time sufficient to cure it by a cross-linking reaction between components of the composition.

As mentioned above, the essential components of the coating compositions are (a) an hydroxyalkyl carbamate compound, (b) an amino cross-linker and (c) a polymer containing active sites which are reactive with the amino cross-linker. The optional component is (d) an acid catalyst.

The compounds generally useful in the third aspect of the present invention are hydroxyalkyl carbamate compounds of the formula:

(23)

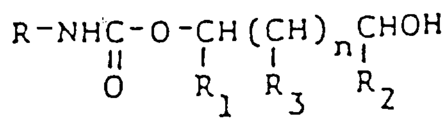

wherein R is a $C_1$ to $C_{20}$ organic moiety which may contain one or more heteroatoms and/or one or more hydroxyl groups and each of $R_1$, $R_2$, and $R_3$ is independently H or $CH_3$. A compound of the type exemplified in equation (23) is obtained by reacting a six-member ring cyclic carbonate with a primary amine.

A preferred class of hydroxyalkyl carbamate compounds useful as reactive diluents in the third aspect of the invention has the formula:

(24)

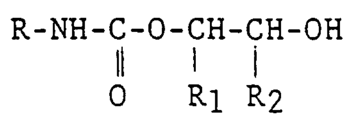

Other suitable classes of hydroxyalkyl carbamate compounds utilizable as reactive diluents in accordance with the present invention are

(25)

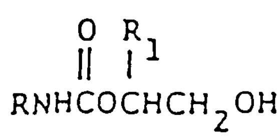

where R is a $C_2$ to $C_{20}$ aliphatic, cycloaliphatic or aromatic moiety, including any one of the foregoing containing heteroatoms, and $R_1$ is H or $CH_3$; and

(26)

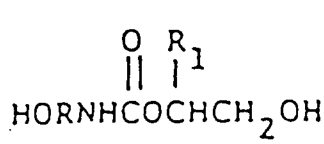

where R is a $C_2$ to $C_6$ alkyl and $R_1$ is H or $CH_3$.

Suitable reactive diluents which have been prepared and utilized in coating compositions in accordance with the invention include the following:

(A)

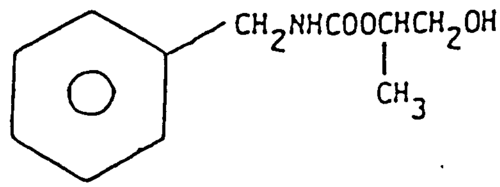

(B)

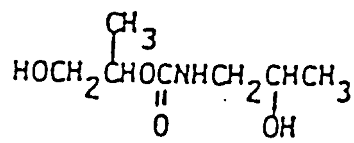

(C)

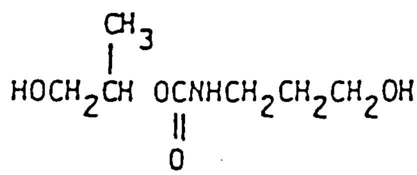

(D)

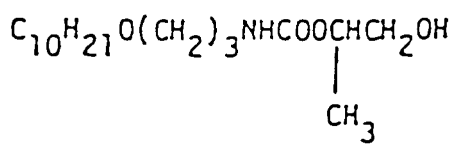

The compounds of formulas (A) - (D) were prepared by reacting suitable amines (the formulas of which are readily discernible from the above) with an appropriate cyclic carbonate.

The amino cross-linker may be any one of a large variety of known amino cross-linking compounds including cross-linkers based on melamine, glycoluril, guanamines such as benzoguanamine, urea, substituted ureas and the like. As used herein, the term "amino cross-linker" is intended to include any suitable aminoplast. One such suitable class of materials is aminoplast resinous compositions, in particular, modified aminoplast resinous compositions such as those disclosed in U.S. Patent 3,082,180, comprising modified amino-triazine-aldehyde resins. Another suitable class of amino cross-linkers useable as a component of the invention comprises fully methylated, fully methylolated melamine compositions, a process for the manufacture of which is described in U.S. Patent 4,293,692. Another suitable group of amino cross-linkers is that described in U.S. Patent 4,105,708 and comprising substantially fully mixed-alkylated, substantially fully methylolated glycoluril derivatives comprising dimethoxymethyl diethoxymethyl glycoluril. Glycoluril is also known as acetylene urea and is obtained by reacting two moles of urea with one of glyoxal. Its proper chemical name is tetrahydroimidazo - (4, 5-d) imidazole 2, 5 (1H, 3H)-dione. The disclosures ot the aforesaid U.S. Patents 3,082,180, 4,293,692 and 4,105,708 are each incorporated by reference herein.

Particularly suitable classes of amino cross-linkers are melamine-formaldehyde resins and glycoluril-formaldehyde resins which, in each case, have been partially or fully, i.e., at least partially, alkylated and methylolated. For example, as disclosed in the aforesaid U.S. Patent 4,293,692, melamine may be methylolated by reaction with formaldehyde. The melamine may be either fully methylolated to produce hexamethylol melamine, or partially methylolated to produce pentamethylol melamine, tetramethylol melamine, etc., or mixtures of two or more of the foregoing. The at least partially methylolated melamine (or glycoluril) may then be reacted with an alcohol, such as methanol, to fully or partially alkylate the fully or partially methylolated melamine or glycoluril. For example, a substantially fully methylolated, fully alkylated melamine (hexamethoxymethyl-melamine) is sold under the trademark CYMEL 303 by American Cyanamid Company. Reference herein and in the claims to "melamine-formaldehyde" and "glycoluril-formaldehyde" resins includes any suitable melamine and any suitable glycoluril derived resin utilizable as a cross-linker in coating compositions. Similarly, reference herein and in the claims to "urea-formaldehyde" and "benzoguanamine-formaldehyde" resins includes corresponding urea and benzoguanamine derived resins.

Any one of a large variety of polymers containing active sites which are reactive at elevated temperature with the amino cross-linker and/or hydroxyalkyl carbamate groups is useable in the present invention. It will be appreciated that a broad spectrum of such compositions exists and can be selected to provide desired qualities in the cured coating. The following classes of such polymers are illustrative of those useable in the invention.

Any suitable acrylic resin may be utilized, i.e., any resin containing at least one acrylic moiety. Generally, acrylic resins comprise interpolymers of esters of unsaturated carboxylic acids, carboxylic acids and one or more ethylenically unsaturated monomers. The acid may be acrylic, methacrylic or other ethylenically unsaturated mono-or dicarboxylic acids. Alkyl acrylates, alkyl-methacrylates and vinyl aromatic hydrocarbons such as styrene, vinyl toluene and the like may be employed as the monomer moiety of the interpolymer. By way of illustration, suitable acrylic resins could comprise interpolymers containing one or more of hydroxyethyl acrylate, meleic anhydride, n-methloylacrylamide, hydroxyporpyl acrylate, acrylamide, acrylic acid, methacrylic acid, methyl acrylamidoglycolate, methyl acrylamidoglycolate methyl ether, etc. The polymeric structure may also include modifying units derived from one or more of butadiene, styrene, alpha-methyl styrene, methylmethacrylate, butyl acrylate, acrylonitrile, hydroxyethyl acrylate; glycidyl methacrylate, acrylamide, methacrylamide, vinyl chloride and vinylidene chloride. For example, commercially available acrylic emulsion polymers obtained by polymerizing a monomer blend of n-butyl acrylate, styrene and acrylic acid may be utilized

Any suitable polyester resin may likewise be utilized as the polymer of the compositions of the invention. Generally, such polymers comprise the reaction product of one or more glycols and dicarboxylic acids. By way of illustration, the polyester resin may be prepared by reacting together glycols such as ethylene glycol, diethylene glycol, triethylene glycol, trimethylene glycol, 1, 2-propylene glycol, tetramethylene glycol, 2, 3-butylene glycol, pentamethylene glycol, and dicarboxylic acids such as malonic maleic, succinic, adipic, pimelic, sebacic, oxalic, phthalic, terephthalic, hexahydroterephthalic, and pare-phenylene-diacetic acids, decamethylene dicarboxylic acid, and the like. Esterification reaction of the multifunctional alcohols and acids results in formation of the polyester. Modified resins such as polyester amide resins having terminal hydroxyl groups may also be utilized. Alkyd resins are similiarly formed by reaction of polybasic acids, amhydrides and polyhydric alcohols, for example, by reaction of isopthalic acid, succinic, maleic or pyromellitic anhydride and ethylene or propylene glycol or pentaerythritol.

Any one of a large number of epoxy resins may be employed as the polymer in the coating composition. For example, polyglycidyl esters of polycarboxylic acids, such as the reaction products of epichlorohydrin or other similar epoxy compounds with aliphatic or aromatic polycarboxylic acids such as cyanuric acid, terephthalic acid, glucaric acid, succinic acid, adipic acid, phthalic acid, oxalic acid, dimerized linolenic acid, phenols such as bisphenol-A, bisphenol-F, bisphenol-S, polyphenolic resins derived from phenol-formaldehyde-acetone condensates, etc. and the like may also be employed.

The acid catalyst optionally employed to reduce the cure temperature, may comprise any suitable acid, as mentioned above. Generally, any substance meeting the definition of a Lewis or Bronsted acid and which does not interfere with the cross-linking reaction or unduly adversely affect ambient temperature stability of the composition may be used. Among suitable acid catalysts are aromatic sulfonic acid compounds such as those disclosed in U.S. Patent 3,960,688, alkyl esters of phosphoric or alkyl phosphonic acids, dinonyl naphthalene sulfonic acid, paratoluene sulfonic acid, n-dodecylbenzenesulfonic acid and the like, as well as inorganic acids such as nitric, sulfuric, phosphoric and hydrohalic acids. Phosphoric acid ester catalysts of the type sold under the trademark CYCAT by American Cyanamid Company may suitably be employed.

The hydroxy group - containing polyurethane polymers of the fourth aspect of the invention may be prepared either by self-condensing the poly-hydroxyalkyl compound or by condensing it with a polyol. The polyol employed in the latter condensation reaction may be any suitable polyol and may be derived from polyols, polyether polyols, polyester polyols, alkyd polyols, phenol formaldehyde condensation products, bisphenol-A, bisphenol-F, polyether resins, bisphenol ethylene oxide or propylene oxide condensation products, polyether resins, dihydroxy polybutadiene, or the like.

Typical polyether diols or polyols which are suitable for use in the present invention are polyethers derived from the reaction of diols, polyols or amines with ethylene oxide, propylene oxide, styrene oxide or the like, or mixtures of other epoxies with a carbon content from $C_2$ to about $C_{18}$ or polyethers derived from tetrahydrofuran.

Typical polyester diols or polyols which are suitable for use in the present invention can be prepared by known methods of condensing diol, triols or polyols with a combination of mono-, di-, tri-, or polybasic carboxylic acids. Typical diols or polyols are ethylene glycol, propylene glycol, diethylene glycol, dipropylene glycol, 1,3 butane diol, 1,6-hexanediol, neopentyl glycol, cyclohexane- dimethanol, trimethyl pentane diol, trimethylol propane, pentaerythritol, etc. Typical carboxylic acids are $C_8$ to $C_{18}$ fatty acids,

dimeric fatty acids, $C_7$ to $C_{10}$ aliphatic dibasic acids, aromatic mono carboxylic and polycarboxylic acid such as benzoic, phthalic and trimellitic acid.

Polyols or a combination of polyols with an average functionality of 2 to 3 are preferred. Although higher functional polyols can be reacted with the polyhydroxyalkyl carbamate compounds, the ratio of polyol to carbamate and the resultant reaction must be controlled to prevent gellation. It will be appreciated that one skilled in the art can readily determine, by conventional techniques, the selection of appropriate reactants and conditions so as to avoid gellation. The condensation of the poly-hydroxyalkyl carbamate compound and a polyol may be represented by the following equation.

(27)

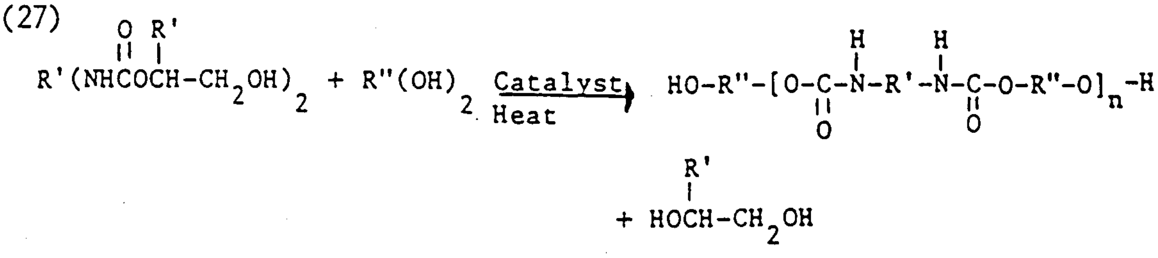


wherein each of R' and R" is the same or different and comprises any suitable organic moiteies.

Reaction (27) illustrates the formation of an hydroxy group-terminated polyurethane polymer and a 1, 2-diol leaving group. The reaction of the poly-hydroxyalkyl carbamate and a polyol can proceed solely by heat or in the presence of a suitable transeterification catalyst such as a tin compound. The reaction can also be run in a solvent medium. The diol which is eliminated during the condensation reaction may be continuously removed by distillation, either using the vacuum or azerotropic distillation with a suitable solvent.

An hydroxy group-containing polyurethane polymer having a high urethane content may be obtained by self-condensation of a poly-hydroxyalkyl carbamate compound as illustrated by

(28)

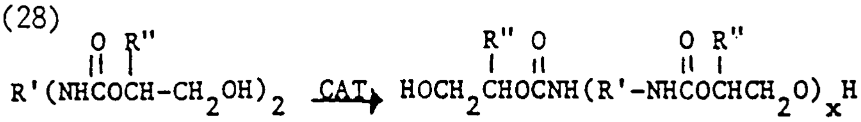


wherein each of R' and R" is the same or different and comprises a suitable organic moiety. The reaction of the poly-hydroxyalkyl carbamate and a polyamine can proceed in the presence of any suitable catalyst. Suitalbe catalysts include organo tin compounds such as dialkytin compounds, e.g., dibutyltindilaurate, organo zinc compounds such as zinc octoate, zinc butyrate, etc., some organo titanium compounds, and, generally, any suitable catalysts as are known in the art.

The hydroxy group-containing polyurea polymer may be obtained by condenstiaon of the poly-hydroxyalkyl carbamate compound with a suitable polyamine, as illustrated by

(29)

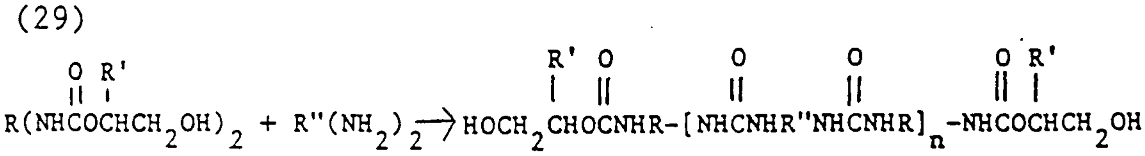

wherein each of R' and R" may be the same or different and comprise any suitable organic moiety.

It should be noted that partial replacement of the polyol with polyamines can be carried out, i.e., a co-condensation of the poly-hydroxyalkyl carbamate compound with one or more polyols and one or more polyamines can be carried out in order to incorporate both urea and urethane linkages in the polymer. For example, the incorporation of urea linkages in the polyurethane polymer is often desired to achieve improved hardness and increased solvent resistance.

While the above-described polyurea and polyurethane polymers may find use as thermoplastics in elastomer applications such as molding compounds, coatings for metals, plastics, textiles or adhesives, it has been recognized that their hydroxy functionality and the urethane linkage or unsaturation on the main chain can be used for further cross-linking. The hydroxy functionality can be cross-linked with amino formaldehyde resins or isocyanates and the unsaturated linkages can be used for air oxidative cure or for sulfur vulcanization. It has been found that a composition well suited to provide a thermosettable coating composition can be prepared by utilizing an amino cross-linker in combination with the polyurethane, polyurea or polyurethane-polyurea polymer.

Any suitable solvent or vehicle may be used to carry the polymer, cross-linker and optional catalyst such as, for example, ethylene glycol monethyl ether (Cellosolve).

The catalyst optionally employed in the composition may comprise any suitable acid catalyst. Generally, any substance meeting the definition of a Lewis or Bronsted acid and which does not interfere with the cross-linking reaction may be used. Among suitable acid catalysts are aromatic sulfonic acid compounds such as those disclosed in U.S. Patent 3,960,688, alkyl esters of phosphoric or alkyl phosphonic acids, dinonyl naphthalene sulfonic acid, paratoluene sulfonic acid, n-dodecylbenzenesulfonic acid and the like, as well as inorganic acids such as nitric, sulfuric, phosphoric and hydrohalic acids. Phosphoric acid ester catalysts of the type sold under the trademark CYCAT by American Cyanamid Company may suitably be employed.

Other additives, such as pigments, modifiers, etc. may also be included in the composition as is well known in the art.

The composition according to the fifth aspect of the invention, containing the cross-linking agent, polymer, and, optionally, catalyst, in use is heated to an elevated temperature at which the hydroxyalkyl carbamate groups of the cross-linker react with active functional groups of the polymer to cross-link the polymer and produce diol leaving groups of low toxicity, such as proplene glycol or ethylene glycol. A tlypical reaction sequence of, for example, a hydroxy functional group containing polymer is shown in equation (30) and that for an amine functional group containing polymer is shown in equation (31).

(30) 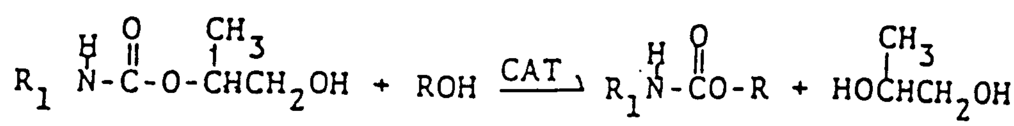


(31) 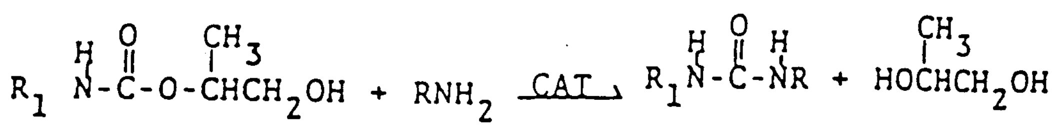


With carboxyl funcational group polymers, amide groups are formed in the reaction and the reaction products of the cross-linking reaction are $CO_2$ and the corresponding 1, 2-diol. Generally, the leaving groups in the cross-linking reaction are, as illustrated above, diols of low toxicity, such as propylene glycol or ethylene glycol. Any attempt to prepare the above described hydroxyalkyl carbamate compounds by reaction of a diisocyanate with a di- or polyol would be difficult or impossible inasmuch as the formation of polyurethane polymers would occur.

As mentioned above, the cross-linking agents may be obtained by reaction of a cyclic carbonate and an amine.

It will be appreciated by those skilled in the art that a wide variety of aliphatic amines may be used in the invention.

Aliphatic and cycloaliphatic amines react readily with cyclic carbonates to provide the hydroxyalkyl carbamate compounds used in the invention.

Aromatic amines usually require an appropriate catalyst to promote the reaction with cyclic carbonates as shown, for example, in U.S. Patent 4,268,684 of Arthur E. Gurgiolo. The aromatic amines disclosed therein are suitable for use in the present invention. These include amines represented by the formulas:

(32) 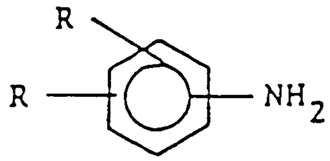 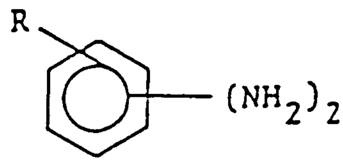

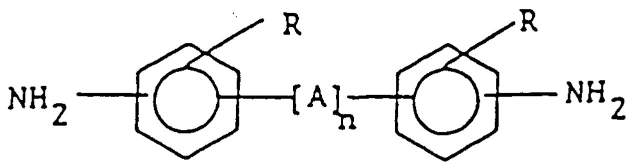

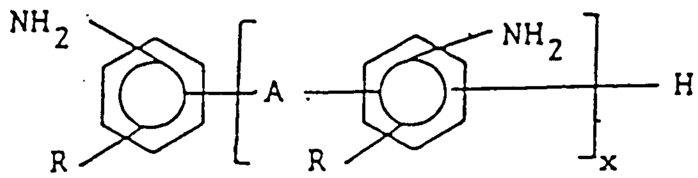

wherein each R is independently hydrogen or a hydrocarbyl or hydrocarboxy group containing up to 8, preferably up to 4, carbon atoms; A is a divalent hydrocarbon group having from 1 to 10 carbon atoms, preferably from 1 to 6 carbon atoms, n has a value of zero to 1, x has an average value of from about 1.1 to about 10, preferably from about 2 to about 4.

Particularly suitable aromatic amines include, by way of example and not limitation, aniline, o-, m-, or p-toluidine, 2,4-xylidine, 3,4-xylidine, 2,5-xylidine, 4-ethylaniline, 3-propylaniline, 1, 3-diaminobenzene, 2,4-diaminotoluene, 2,6-diaminotoluene, 4,4'-diaminodiphenyl methane, 2,4,4'-triamino-diphenylether, 2,6- diaminonaphthalene, 4, 4'-bismethylene diphenlamine, 0-, m-, or p-anisidine mixtures thereof and the like.

In a prepared embodiment of the invention, the cross-linking agent has the formula:

(33) 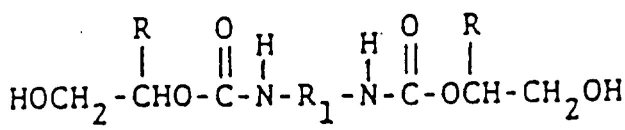

wherein $R_1$ is an aliphatic, cycloaliphatic, heterocylic, polyurethane or polyurea containing moiety of more than two carbon atoms, and R is independently H or a $C_1$ to $C_6$ alkyl or arylalkyl moiety.

Self-condensation of a compound of formula (33) above or condensation of such compound with a polyol and/or a polyamine will yield a di- or polyfunctional hydroxyalkyl carbamate compound containing urethane and/or urea moieties in the chain. For example, condensation with a polyol is illustrated by the following reaction.

(34) 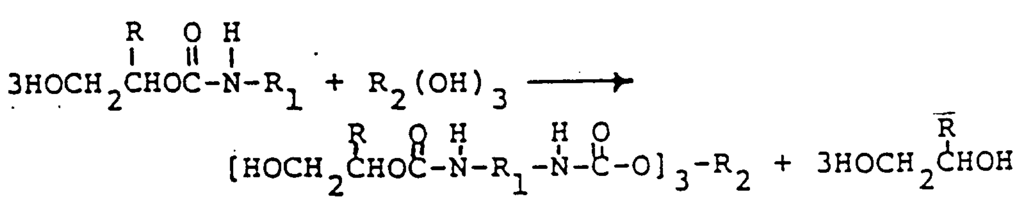

In the above reaction $R_1$ must also contain the following hydroxyalkyl carbamate group.

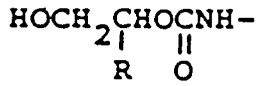

The cross-linker may be suitably modified for a given application by selection of appropriate polyols and/or polyamines in the above-mentioned condensation reactions. For example, physical characteristics such as flow, solubility, and melting point as well as hydrophobicity and functionality can readily be controlled by an appropriate selection.

For example, three moles of biscarbamate prepared by reacting 1,6-hexanediamine and propylene carbonate per reaction 30 above are condensed with one mole of trimethylol propane to prepare a liquid, organic solvent soluble, trifunctional cross-linker for cationic electrocoating. The starting biscarbamate is a water soluble, difunctional crystalline material. Its water solubility prevents its use in cationic electrocoating because it will not codeposit with the resin. The modification of the biscarbamate with the polyol provides a hydrotropic composition well-suited to use in cathodic electrodeposition processes.

The preferred cross-linkers of this invention are at least disfunctional and monomeric or oligomeric in nature. For special purposes, higher molecular weight carbamate-containing cross-linker can be used. The molecular weight can be as low as, for example 236 for the bishydroxyethyl carbamate of ethylenediamine. For polymeric polyurea or polyurethane containing hydroxyalkyl carbamates, the average molecular weight can be greater than 2000. Those skilled in the art will readily select the optimum equivalent weight, functionality, solubility, and melting point required for a given application.

Typical characteristics of an hydroxyalkyl carbamate containing cross-linker for different application areas are listed below:

<u>Typical Characteristics of Carbamate Cross-Linker</u>

| Application | Carbamate Functionality | Melting Point | Solubility |
|---|---|---|---|
| Powder | | | |
| Polyester | 3,>3 | 60-120 °C | not important |
| Acrylic | 2,>2 | 80-120 °C | not important |
| Cationic EC | 2,>2 | preferably liquid at 25 °C, not crystalline | soluble in org. solvents and hydrophobic |
| Solvent borne | >2 | liquid at 25 °C | soluble in org. solvents |
| Water borne | >2 | solid or liquid at 25 °C | water soluble or water dispersible |

The amount of hydroxyalkyl carbamate selected in a typical formulation will of course depend on the cross-linking density desired. Typically, the proportion and compositions of resin and cross-linker are selected to provide from about 0.2 to about 5 moles of hydroxyalkyl carbamate groups per mole of active functional group on the polymer. If larger proportions of cross-linker carbamate groups to functional sites on the polymer are used, the cross-linker will also undergo some self-condensation, as shown in equation (35).

(35) 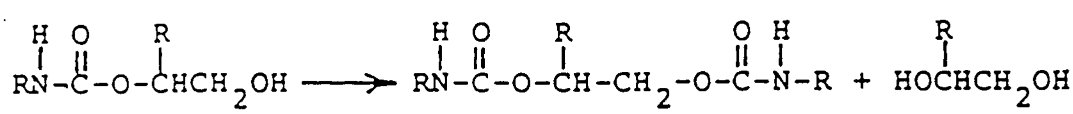

The cross-linkable resins utilizable in the present invention may comprise any suitable polymer containing active functional groups, i.e., suitable functional groups which will react, upon heating, preferably upon heating in the presence of a catalyst, with the hydroxyalkyl carbamate cross-linker of the invention. Such active groups comprise hydroxyl, amine, and carboxyl groups and, accordingly, resins containing such groups are utilizable in the practice of the invention. The functionality of the polymers employed can be as low as 2 but is preferably 3 or higher, and the molecular weight may range, for example, from about 300 to about 100,000. For example, acrylic polymers useful in the invention usually have a molecular weight

range of from about 1,000 to about 50,000.

A typical functional group content of, for example, hydroxyl resins utilizable in the invention is from about 0.5 to about 4 milliequivalents ("meq") hydroxyl per gram of resin solids.

An illustrative, but by no means exhaustive, list of polymers which may be usefully employed in the invention includes acrylic, polyester, vinyl, epoxy, polyurethane, polyamide, cellulosic, alkyd and silicone resins. Acrylic resins useful in the invention can be derived from the acrylic acid or methacrylic acid esters of $C_1$ to $C_{18}$ aliphatic alcohols. Optionally, acrylonitrile, styrene or substituted styrene can be incorporated into the polymer. Additional comonomers suitable for such use are maleic or fumaric acid esters or half esters. Functional groups can be derived from the hydroxyalkyl esters of acrylic, methacrylic, maleic or fumaric acid. Carboxyl functionality can be derived from alpha and beta unsaturated carboxylic acids such as those mentioned below.

Polyester and alkyd resins suitable for use with the hydroxyalkyl carbamate-containing amine cross-linker can be derived from diols, polyols, mono-, di-, and polybasic acids. Examples of such suitable diols or polyols are ethylene glycol, propylene glycol, 1,3-butylene glycol, diethylene glycol, dipropylene glycol, neopentyl glycol, trimethylpentane diol, cyclohexanedimethanol, trimethylolpropane, trimethylolethane and glycerine pentaerythritol. Typical carboxylic acids useful in preparing hydroxy and carboxyl functional polyester and alkyds are $C_8$ to $C_{18}$ aliphatic mono-carboxylic acids, $C_4$ to $C_{10}$ aliphatic dicarboxylic acids, aromatic mono-, di-, and tricarboxylic acids such as benzoic acid, o-, m-, p-phthalic acids, or trimellitic acid, dimeric fatty acids, and hydroxy carboxylic acids such as dimethylol propionic acid or caprolactone.

Vinyl polymers particularly suitable for use in the invention are hydroxy and carboxyl functional group-containing polymers containing either vinyl chloride or vinyl acetate as one of the comonomers.

Epoxy resins particularly suitable for use in the invention are hydroxy or amine functional resins. These are normally derived from bisphenol-A, bisphenol-F, or phenol formaldehyde resins and epichlorohydrin. The epoxy resins may also be formed from cycloaliphatic epoxies.

Polyurethanes particularly suitable for use in the invention may be hydroxyl, carboxyl, or amine functional and may be derived either from polyester or polyether polyols and a polyisocyanate.

Polyamides particularly suitable for use in the invention may be either amine or carboxyl functional and can be obtained by the conventional techniques of condensing polybasic acids with polyamines or by condensing caprolactone.

Cellulose based hydroxyl functional resins such as cellulose acetobutyrate, and hydroxyethyl cellulose can also be reacted with the hydroxyalkyl carbamate-containing amines of the invention. Hydroxy functional silicones can also be cross-linked with the hydroxyalkyl carbamate cross-linker and are therefore well-suited for use in the invention.

All of the above mentioned active functional group-containing resins can be used in either organic solvent solution, as a powdered solid, or as dispersions in water or organic co-solvent aqueous solutions. Depending on resin structure, these uncross-linked polymers will be preferably used in one of the above mentioned forms. Blends of two or more of the above polymers can also be used. Further, the polymer and carbamate cross-linking agent blend may be pigmented, as is known in the art, to achieve a desired appearance of the coating.

Depending on the application process, either a solid powder or a liquid is applied onto the substrate to be coated and after evaporation of any solvent present, the system is cured for a sufficient period of time, e.g., from several minutes to several hours, at temperatures sufficient to affect cure, e.g., from about 200 to about 400°F (about 93 to 204°C).

A cross-linking catalyst may be used to promote cross-linking of the thermosetting composition of the invention. The catalyst may be an external catalyst or it may be incorporated as an internal catalyst during preparation of the functional group-containing resin, as is known in the art. For example, quaternary ammonium hydroxide groups may be incorporated into the resin. Any suitable cross-linking catalyst may be utilized (such as known metal-containing catalysts, e.g., tin, zinc, and titanium compounds) as well as ternary or quaternary compounds as described below. Benzyltrimethyl ammonium hydroxide, dibutyltindilaurate, and similar compounds are good catalysts for achieving cross-linking at elevated temperatures in the range of from about 100 to about 175°C (about 212 to about 347°F) for a period of a few seconds to about 30 minutes. A catalyst may be present in a formulation in the amount of from about 0.1 to about 10% by weight of the polymer, preferably from about 1 to about 5% by weight of the polymer.

The catalyst may comprise ternary or quaternary catalysts such as known compounds of the formula:

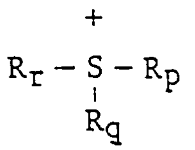 $X^-$ and 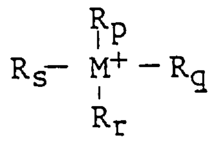 $X^-$,

respectively, where $R_p$, $R_q$, $R_r$ and $R_s$ may be equivalent or different and may be a $C_1$ to $C_{20}$ aliphatic, aromatic, benzylic, cyclic aliphatic and the like, where M may be nitrogen, phosphorus, or arsenic (to provide, respectively, quaternary ammonium, phosphonium or arsonium compounds), where S is sulfur (to provide a ternary sulfonium compound) and where X - may be hydroxide, alkoxide, biscarbonate, carbonate, formate, acetate, lactate, and other carboxylates derived from volatile organic carboxylic acids or the like. Such salts of carboxylic acids are effective to promote the low temperature cure provided that the carboxylic acid portions of the salt are volatile.

The compositions of the present invention are stable at ambient temperature and must be heated to an elevated temperature in order to cause the cross-linking reaction to occur at an appreciable rate. Generally, an elevated temperature of about 200°F (about 93°C) or more is required to effectuate the cross-linking reaction at an appreciable rate. As used herein and in the claims, an "elevated" tem- perature is one which is sufficient to cure the deposited composition by causing the cross-linking reaction to occur at a desired rate, usually a rate sufficient to effectuate cure within a period of 1 to 3 hours or less.

Hydroxyalkyl carbamate amines as illustrated in equations (36) and (37) below are useful for cathodically electrodepositable, self-crosslinkable polymers in the sixth aspect of this invention if, after reaction with suitable epoxy resins and upon acidification, they are cationic and sufficiently hydrophobic to be electro-deposited by conventional techniques. As described in some detail below, in some cases, in order to attain the requisite degree of hydrophobicity of the polymer, some of the available epoxy sites may be used to react with hydrophobic amines to incorporated the latter into the polymer. Useful di- or polyamines for the formation of hydroxyalkyl carbamate amines as illustrated above include those where, in equation (36), each R is independently H or straight chain or branched hydrocarbons from 1 to 50 carbon atoms or such containing either linkages. In equation (37), useful amines include those where n is from zero to about 5, $R_e$, $R_f$ and $R_g$ are straight chained or branched hydrocarbon fragments having one to about six carbon atoms, and where $R_e$, $R_f$ and $R_g$ are straight chained or branched hydrocarbon fragments having one to about six carbon atoms, and where $R_e$, $R_f$ and $R_g$ may also contain an ether group. The Z group may be selected from: hydrogen, hydroxyl, or an alkoxy of from 1 to 20 carbon atoms or a secondary amine of from 1 to 20 carbon atoms, or a primary $-NH_2$ group. In the latter case, the primary amine group Z may be converted to a hydroxyalkyl carbamate group if enough (or excess) cyclic carbonate is utilized to form the hydroxyalkyl carbamate amine (or polyamine) compound.

(36) 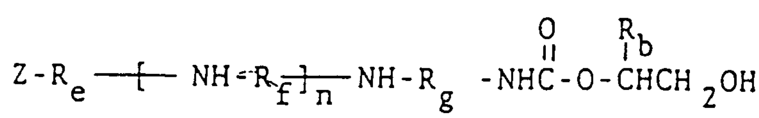

(37) 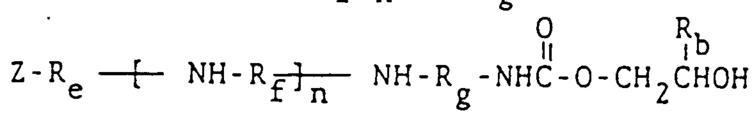

The resulting hydroxyalkyl carbamate-containing amine is reacted with a water-insoluble, epoxide-containing "backbone" compound.

A typical polymer in accordance with the sixth aspect of the invention may have the formula:

(38)

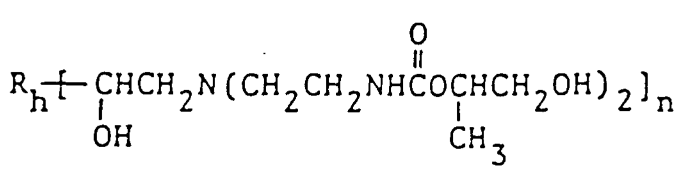

The resultant polymer, upon heating and optionally, in the presence of a suitable cross-linking catalyst, will cross-link through, cross-linking through backbone hydroxyl groups, by cross-linking through self-condensation, and/or, by cross-linking through backbone amine groups.

In all of the cross-linking reactions, the water sensitive hydroxyalkyl portion of the carbamate group is lost as a glycol. The cross-linked films therefore not only develop mechanical properties and solvent resistance during cure but also are water resistant.

Generally, the multi-functional amines utilized in the sixth aspect of the invention contain at least one secondary amine group which is hindered with respect to reacting with the cyclic carbonate and at least one primary amine group. As used herein and in the claims, (a) "multi-functional amine" means an amine containing at least one primary amine group (which may be a blocked primary amine group as described below) and at least one hindered secondary amine group; and (b) "hindered secondary amine group" means a secondary amine group which is inhibited, sterically, electronically or otherwise, with respect to reacting with the cyclic carbonate under conditions at which the primary amine group will react. The secondary amine groups which are sterically or otherwise inhibited from reacting with a cyclic carbonate thus survive formation of the caramate and are reactive with the epoxy groups on the polymer.

The above definition of "multi-functional amine" is intended to include blocked primary amine groups, such as ketimine groups, which can be unblocked to form the primary amine groups. As described in more detail below, the multi- functional amine can optionally be reacted with the epoxide prior to formation of the hydroxyalkyl carbamate groups by having the primary amine groups int he form of blocked primary amines, e.g. ketimine groups. After reaction with the epoxide, the ketimine groups may be hydrolyzed to primary amine groups and then reacted with the cyclic carbonate. Accordingly, any such blocked primary amine group is referred to herein, and in the claims, as a "precursor" of a hydroxyalkyl carbamate group.

The amines utilized in accordance with the sixth aspect of the present invention to react with one or more cyclic carbonates to provide hydroxyalkyl carbamate-containing amine groups may be any one of a large number of compounds and, generally, may comprise multi-functional amines containing straight chain or branched alkyl, cycloalkyl or alkyl aromatic moieties, most preferably $C_1$ to $C_{20}$ alkyl, cycloalkyl or alkyl aromatic moieties and such moieties containing, in addition to at least one carbon atom, one or more heteroatoms. Such moieties containing one or more heteroatoms include, for example, those containing ether groups, thio groups and organo-silicon moieties. General representation of preferred classes of amines are given by the following formulas:

(a) $H_2N(CH_2)_x[NH(CH_2)_x]_n\ NH(CH_2)_xNH_2$

where each x is independently 2 to 6 and n is 0 to 4;

(b) $R_3\ NH(CH_2)_yNH_2$

where $R_3$ is a $C_1$ to $C_{20}$ alkyl, cycloalkyl or alkyl aromatic moiety, and y is 2 or 3; and

(c)

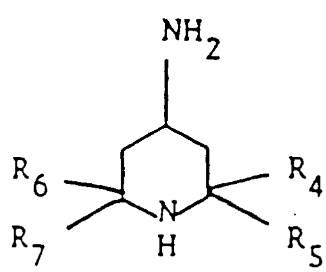

where each of $R_4$ and $R_6$ is independently H or a $C_1$ to $C_4$ moiety and each of $R_5$ and $R_7$ is independently a $C_1$ to $C_4$ alkyl moiety.

Suitable amines include the following fatty acid diamines of the general formula $RNHCH_2CH_2\ CH_2NH_2$ wherein R is a $C_1$ to $C_{20}$ organic moiety, e.g., hydrogenated tallow diamine, tall oil diamine, coco diamine, oleyl diamine and the like; ether diamines of the general formula $R'OCH_2CH_2CH_2NHCH_2CH_2CH_2NH_2$, wherein R' is a $C_1$ to $C_{15}$ organic moiety; and silyl amines of the general formula $(C_2H_5O)_3SiCH_2CH_2CH_2NHCH_2CH_2CH_2NH_2$.

Reaction of a cyclic carbonate with the primary amine groups of one or more amines as indicated above will provide amines containing at least one hydroxyalkyl carbamate group in addition to unreacted

secondary amines. Thus, amine-pendant hydroxyalkyl carbamate resins are obtained having structures in which the $-NH_2$ groups of the above formulas are converted to

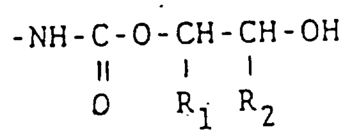

wherein each $R_1$ and $R_2$ is independently H, or a $C_1$ to $C_{20}$ alkyl, cycloalkyl or alkyl aromatic moiety.

For example, a hydroxyalkyl carbamate group-containing amine found to be useful in preparing electrodepositbale polymers in accordance with the invention is obtained by reacting N,N-bis(6-aminohexyl)-2-[(6-aminohexyl)amino] butanediamide with propylene carbonate, and has the formula:

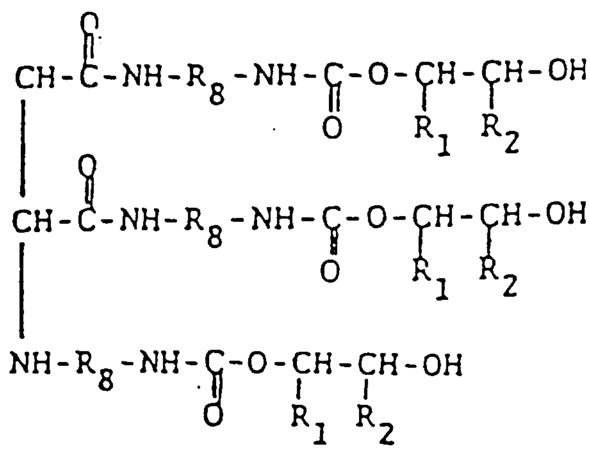

wherein each of $R_1$ and $R_2$ is as defined above and each $R_8$ is $C_6$ alkylene. Other useful amines of this type may be prepared wherein $R_8$ is independently a $C_2$ to $C_6$ alkylene moiety.

Preferred multi-functional amines for reacting with the cyclic carbonate include, for example, diethylenetriamine and triethylenetetramine.

It will be appreciated that those skilled in the art will, when utilizing polyamines, select conditions and reactants so as to avoid gellation in forming the polymer.

A suitable polyepoxide is reacted with approximately one equivalent of the above described amines containing one or more secondary amine groups. The equivalent ratio of amines to epoxy groups should be approximately one to one. Ideally, all reactive epoxy groups will react with a secondary amine group to attach the amine to the epoxy polymer. However, a slight excess or deficiency of epoxy groups after reaction with the secondary amines can be tolerated and accommodated by subsequently adding a small quantity of monoepoxides (in the case of excess secondary amine groups) or simple amines (in the case of excess epoxy groups). Each of the amine groups contains one or more hydroxyalkyl carbamate groups formed thereon by reaction of a cyclic carbonate with a primary amine group of the multi-functional amine, resulting in a self-cross-linkable polymer. Sufficient amine groups are attached to the polymer to render it electrodepositable by cationic deposition.

In an alternate method of manufacturing the depositable polymer of the invention, the epoxides are reacted with amines which contain, in addition to one or more secondary amine groups reactable with the epoxy groups, ketimine groups in lieu of the above described hydroxyalkyl carbamate groups. After reaction of the secondary amine groups with the epoxy groups as described above, so that the amine groups are pendant upon the backbone epoxy polymer, the polymer is acidified and water added thereto. This causes the ketimine groups to react to form free amine groups and one or more suitable cyclic carbonates may then be added to the mixture to react with the resultant free amine groups on the pendant amine moieties. Thus, the multi-functional amine utilized to form the hydroxyalkyl carbamate will contain either an amine group reactable with a cyclic carbonate or a ketimine group convertible to an amine group reactable with the cyclic carbonate. As used herein and in the claims, a "precursor" of a carbamate group means a ketimine group as described in this paragraph.

As will be appreciated by those skilled in the art, a certain degree of hydrophobicity is required of a cathodically electrodepositable polymer. In the case of epoxide polymers which are highly epoxy functional, such as novalac resins, it may be necessary or desirable to coreact the polymer with hydrophobic amines in addition to the amines containing hydroxyalkyl carbamate groups or precursors thereof, i.e., ketimine groups. A quantity of amines of selected hydrophobicity is selected to impart added hydrophobicity to the resultant hydroxyalkyl carbamate-containing electrodepositable resin as required to overcome the hydrophilicity of the hydroxyalkyl carbamate groups.

Preferably, the amount of hydrophobic amines utilized is just sufficient to impart the required degree of hyrophobicity to the carbamate polymer. This is because such hydrophobic amines occupy reactive epoxy sites at the expense of the hydroxyalkyl carbamate -containing amines, thereby limiting the number of cross-linking sites available. Insufficient cross-linking sites would of course result in the cured films obtained from such resins not having the desired properties obtainable by a sufficient degree of cross-linking.

Preferably, secondary hydrophobic amines are utilized to enhance hydrophobicity of the epoxy carbamate resins. Although primary amines and primary/secondary polyamines could be utilized, it will be appreciated by those skilled in the art that reaction of such amines with polyepoxy resins may result in gellation. Those skilled in the art will also appreciate that specific reactants and conditions may be selected to avoid such gellation. However, it is generally preferable to utilize amines of the general structure:

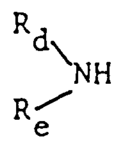

where each of $R_d$ and $R_e$ is independently an aliphatic $C_4$ to $C_{20}$ moiety, a cyclic aliphatic, heterocylic or aromatic moiety or the like, provided that the resulting amine is hydrophobic.

A preferred class of secondary hydrophobic amines is one in which $R_d$ is an aromatic group, $R_e$ is an acrylic aliphatic alkyl, cyclic aliphatic, alkyl aromatic or alkyl heterocyclic group. Such preferred secondary amines include N-methylaniline, N-ethylaniline, N-propylaniline, N-butylaniline, N-benzylaniline, etc.

Preferably, the weight percent of the hydrophobic amine (expressed as the weight of the hydrophobic amine divided by the sum of the weight of the hydrophobic amine plus the carbamate polymer, all based on 100% resin solids) comprises 10 to 80%, preferably, 10 to 40% by weight. Most preferably, the carbamate polymer containing such hydrophobic secondary amines is obtained from a novalac epoxy resin. The utilization of hydrophobic amines to overcome hydrophilic tendencies of the resin is generally not encountered when high equivalent weight bisphenol-A resins or the like are utilized as the backbone resin, inasmuch as only the end segments of the bisphenol-A epoxy resins contain epoxy groups, and intermediate portions are repeating hydrophobic segments.

In preparing electrodeposition baths in accordance with the invention, the polymer is dispersed in water by partial acidification with an acid such as formic acid and, when an external catalyst is utilized, a catalyst such as ditutyltindilaurate or a quaternary ammonium compound is added. Alternatively, the catalyst may be incorporated into the backbone polymer to provide an internal catalyst, as mentioned above. Any suitable acid may be employed to disperse the self-cross-linking resins of the invention, including hydrohalic acids, nitric, sulfuric, phosphoric, or other mineral acids and water soluble organic acids such as formic acid (preferred), acetic, lactic, propionic, butyric, pentanoic and citric acids and the like, as well as polycarboxlic acids such as oxalic, malonic, succinic, maleic, and fumaric acids, and the like.

The cathodic electrocoating compositions of the seventh aspect of the invention comprise three essential and one optional components. These compositions have good bath stability at ambient temperature and may be cathodically electrodeposited upon a substrate by conventional techniques, followed by heating to an elevated temperature for a time sufficient for cure by a cross-linking reaction between components of the composition.

As mentioned above, the essential components of the electrodepositable composition, which is diluted in water to comprise an electrodeposition bath, are (a) a capped or uncapped hydroxyalkyl carbamate cross-linking agent, (b) a cationically-charged, non-gelled polymer and (c) an acid solubilizer or disperser to render the polymer water-dispersible. The optional component is (d), a cross-linking catalyst.

Hydroxyalkyl carbamate-containing compounds are useful in this seventh aspect of the invention if they are sufficiently hydrophobic to be co-electrodeposited with a cationic polymer by conventional techniques. In some cases, in order to attain the requisite degree of hydrophobicity of the cross-linking agent, the hydroxy groups may be reacted with other, hydrophobic capping agents to incorporate the latter into the

cross-linking agent.

Numerous polyamines, including, for example, 4,4'-diamino-dicyclohexyl methane, 4,4'-diaminodicyclohexyl propane, and hexamethylene diamine may be used for the formation of carbamate cross-linking agents by reaction with cyclic carbonates. Suitable polyamines include, by way of example and not limitation, simple diamines such as those of the formula

(39) $R_1HN\text{-}R_2\text{-}NHR_3$

where $R_1$ is independently H, $CH_3$ or $C_2$ to $C_{20}$ alkyl, $R_2$ is independently $-CH_2$ or $C_2$ to $C_{20}$ alkyl fragments which may contain aromatic or saturated rings and wherein $R_1$, $R_2$, and $R_3$ may also contain other functional groups which do not interfere with the amine-carbonate reaction, including, for example, esters, amides, nitriles, ethers, hydroxys, phenolics, ketones, etc., and $R_3$ is independently H, $CH_3$ or $C_2$ to $C_{20}$ alkyl.

A suitable class of monomers/polymers usable to react with a cyclic carbonate to form a carbamate-containing cross-linking agent comprises vinyls/polyvinyls, acrylics/polyacrylics, methacrylics/polymethacrylics, esters/polyesters, amides/ polyamides, imides/polyimides, ethers/polyethers, or mixtures or copolymers of these which contain an average of two or more pendant amine groups per molecule.

Because, as mentioned a certain degree of hydrophobicity is required of a cathodically electrodepositable material such as the cross-linking agent employed in this aspect of the invention, it, is preferred that the polyamines (as used herein, the term "polyamines" is deemed to include diamines) utilized be selected so that after reaction with the cyclic carbonates the resulting carbamate cross-linking agents are water insoluble or only partly water soluble. However, water soluble carbamate cross-linking agents, otherwise suitable, may be modified to render them water insoluble or partly water soluble. This may be accomplished by capping or blocking the hydroxyalkyl carbamate groups by reacting them with a suitable capping agent which, when the deposited composition is heated to cure, will enter into the cross-linking reaction. Generally, the capping agents may be any suitable reagents which will react with the hydroxy groups of the hydroxyalkyl carbamate moiety to form an ester, urethane, thiocarbamate, sulfonic ester, sulfonamate ester, ether, or the like, whereby the cross-linking agent is rendered water-insoluble. Among suitable capping agents are the classes of compounds set forth in the following list, which also shows the class of the moiety resulting from capping the hydroxy moiety of the hydroxyalkyl group of the hydroxyalkyl carbamate.

| Capping Agent | Resultant Capping Moiety |
|---|---|
| 1. Aliphatic and aromatic carboxylic acids | Esters |
| 2. Aliphatic and aromatic carboxylic acid halides | Esters |
| 3. Aliphatic and aromatic carboxylic acid anhydrides | Esters |
| 4. Aliphatic and aromatic carboxylic esters | Esters |
| 5. Isoalkylenyl Esters (e.g., isopropenyl acetate, etc.) | Esters |
| 6. Ketenes (e.g., ketene, mono or disubstituted ketenes, etc.) | Esters |
| 7. Ureas (e.g., urea, mono-,di-, or tri-substituted ureas, etc.) | Urethanes |
| 8. Aliphatic and aromatic isocyanates | Urethanes |
| 9. Cyanic Acid | Urethanes |
| 10. Aliphatic and aromatic isothiocyanates | Thiocarbamates |
| 11. Aliphatic and aromatic sulfonyl halides | Sulfonic Esters |
| 12. Aliphatic and aromatic sulfamoyl halides | Sulfonamate Esters |
| 13. Aliphatic and Alkyl aromatic alkyl halides | Esters |

The above list of capping agents is merely illustrative and not limiting, as will be appreciated by those skilled in the art. For example, alcohols, including diols and polyols (see Example 4, below), mercaptans, amines and polyamines may also be employed as capping agents to render the hydroxyalkyl carbamate cross-linking agent water-insoluble. By way of example, capping the hydroxyalkyl carbamate moiety of the cross-linking agent with a polyol is illustrated the the following reaction.

(40) 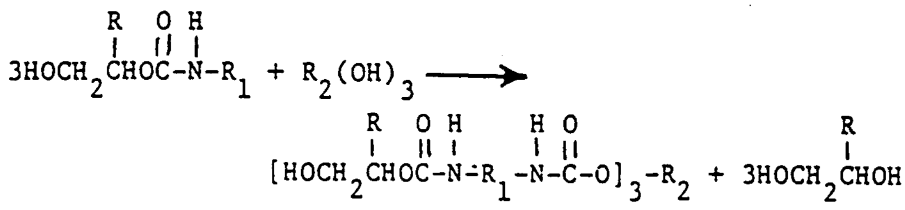

In the above reaction $R_1$ must also contain the following hydroxyalkyl carbamate group.

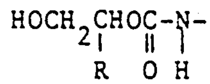

As shown by reaction (40), three moles of a biscarbamate prepared by reacting 1,6-hexanediamine and propylene carbonate are condensed with one mole of trimethylol propane to prepare a liquid, organic solvent-soluble, trifunctional cross-linker suitable for use in a cationic electrocoating composition in accordance with the present invention. The starting biscarbamate is a water soluble, difunctional, crystalline material. Its water solubility prevents its use in cationic electrocoating because it will not codeposit with the resin. The capping of the biscarbamate with the polyol provides a hydrophobic composition well suited for use in this aspect of the invention.

The amino group-containing polymer utilized with the aforementioned hydroxyalkyl carbamate cross-linking agent is a water-dispersible, non-gelled polymeric material carrying a cationic charge. The polymeric material may contain several different types of functional groups. For example, the presence of hydroxy groups in the polymeric material is highly desirable for reaction with the carbamate cross-linking agents. However, the carbamate-containing cross-linking agents will also cross-link through amine groups. The A large number and variety of amino group-containing polymeric materials are suitable for use as the cationic polymeric component so long as such material are non-gelled, water-dispersible, polymeric in nature, and capable of carrying a cationic charge. It is also necessary that these polymeric materials co-deposit with the carbamate- containing cross-linking agent and when used, the optional cross-linking catalyst on the metal surface upon passage of an electric current between a cathode comprising the surface and an anode immersed in the electrode-position bath. In this way, the electrodeposited composition can be converted to the cross-linked state by the application of heat. Cationic polymeric materials such as those identified as cationic Polymeric Materials A through F in U.S. Patent 4,026,855, the disclosure of which is incorporated by reference herein, may be utilized as the amino group-containing polymers invention. Generally, in addition to bisphenol-A based epoxy cationic materials, novalac epoxy based cationic materials may be utilized.

The acid solubilizer or disperser may comprise one or more organic or inorganic acids which are water soluble or at least water dispersible and which will convert the amino group-containing polymer to a water-dispersible material carrying a cationic charge. The acid solubilizer is a water soluble (or at least water dispersible) material, so that the counterion formed when the acid is added to the cationic polymer facilitates the water dispersion or solubilization of the cationic polymer. Generally, the acid solubilizer may be any suitable acid which will impart a cationic charge to the polymer and will not interfere with the stability or cross-linking reaction of the composition.

Among suitable acid solubilizers are inorganic acids such as: hydrohalic acids, nitric, sulfuric, phosphoric, carboxylic acids such as acetic, butyric, pentanoic, formic, lactic and citric acids or the like, or polycarboxylic acids such as: adipic, oxalic, malonic, succinic, maleic, or fumaric acids or the like.

The optional catalyst is effective to lower the cure temperature of the other components of the composition after they have been electrodeposited upon a substrate. The catalyst accordingly must co-electrodeposit with the other ingredients in the cathodic electrodeposition process. Typically, a suitable cross-linking catalyst will lower the cure temperature of the electrodeposited materials from about 400 to 600°F (about 204 to 316°C), the cure temperature without the catalyst, to about 200 to 400°F (about 93 to 204°C). In order to attain sufficient co-deposition of the cross-linking catalysts with the other components of the electrodeposition composition, the cross-linking catalysts are preferably water insoluble or, at most, only partially soluble in water. Further, in order to insure efficient codeposition of the catalyst, it is preferably at

least partially soluble in the cross-linking agent and in the amino group-containing polymer. Typical catalysts include organo tin compounds such as dibutyltindilaurate, organo zinc compounds, organo titanium compounds, quaternary ammonium compounds and the like, including quaternary phosphonium and arsonium compounds and ternary sulfonium compounds. Other suitable catalysts include organo tin compounds such as dialkytin compounds, e.g., dibutyltindilaurate, organo zinc compounds such as zinc octoate, zinc butyrate, etc., some organo titanium compounds, tetraalkyl ammonium compounds where the alkyl groups are selected so that quaternary ammonium compounds are water soluble (or at most, water dispersible) and co-deposit with the other components of the composition. In general, the catalysts are selected so that they are sufficiently hydrophobic to co-deposit with the carbamate cross-linking agent and amino group-containing polymer components at least in amounts sufficient to reduce the cure temperature of the electrodeposited composition. As known in the art, the catalysts may be incorporated into the backbone of the polymer (b) during preparation of the polymer. Usually, sufficient catalyst is co-deposited to reduce the cure temperature from about 400 to about 600°F(about 204 to 316°C) to from about 200 to about 400°F (about 93 to 204°C) or even less, e.g., from about 200 to about 250°F (about 93 to 121°C).

Hydroxalkyl carbamate amines are useful in the eighth aspect of this invention if they impart water solubility/reducibility to normally water insoluble epoxy resins after the resins have been reacted with the hydroxyalkyl carbamate-containing amines.

The epoxide material utilized in accordance with this aspect of the invention may be a monomeric or polymeric epoxy containing material, preferably a resinous polyepoxide material containing two or more epoxy groups per molecule.

A suitable polyepoxide as described above is reacted with approximately one equivalent of the amine compound containing one or more secondary amine groups. The equivalent ratio of amine to epoxy groups should be approximately one to one. Ideally, all reactive epoxy groups will react with a secondary amine group to attach the amine to the epoxide and provide a substantially epoxy-free polymer.

In an alternative method of preparing the polymer, the epoxides are reacted with amines which contain, in addition to one or more secondary amine groups, ketimine groups in lieu of some or all of the hydroxyalkyl carbamate groups. After reaction of the secondary amine groups with the epoxy groups, so that the amine groups are pendant upon the backbone epoxy polymer, the ketimine groups are hydrolyzed to form free amine groups and one or more suitable cyclic carbonates may then be added to the mixture to react with the resultant free amine groups. Thus, the multi-functional amine utilized to form the hydroxyalkyl carbamate will contain either an amine group reactable with a cyclic carbonate or a ketimine group convertible to an amine group reactable with the cyclic carbonate.

The water borne coating compositions of this aspect of the invention are prepared by adding water, and, optionally, a catalyst and/or a cosolvent to the polymers. Depending upon the specific hydroxyalkyl carbamate-containing polymer and amount of cosolvent utilized, the polymers may vary in solubility form completely water soluble to water reducible. Even with the least water reducible resins it is often possible to obtain solids contents as low as 10 to 20% as clear solutions with only 20 to 30% cosolvents, Furthermore, these cosolvents may be the so called "environmentally exempt" solvents such as alcohols and glycols. Coating compositions with high solids contents (60 to 90%) with workable viscosities are attainable in many cases with no cosolvents.

In preparing coating compositions in accordance with the eighth aspect of the invention, the hydrophilic polymer is dissolved or dispersed in an aqueous medium which optionally may contain a suitable organic sosolvent, and, when an external catalyst is utilized, a catalyst such as dibutyltindilaurate or a quaternary ammonium compound is added. Generally, the external quaternary or tenary catalysts are selected so that they are water soluble or dispersible. Typically, not more than up to about 20 to 40% of the composition, sometimes less than 10%, comprises organic cosolvents. Strong bases such as alkali metal hydroxides (KOH, NaOH, LiOH, etc.) may also be included as catalysts in the composition.

Generally, hydroxyalkyl carbamate-containing amines are reacted with suitable acrylic backbone resins which contain sites which are reactive with primary or secondary amines (or both) to provide the acrylic polymers of the ninth aspect of the present invention. The multi-functional amine utilized to make the hydroxyalkyl carbamate contains a secondary amine group which is hindered with respect to reacting with the cyclic carbonate and at least one primary amine group.

Such secondary amine groups can then be reacted with epoxy groups on acrylic polymers, without affecting the carbamate groups. The resultant hydroxyalkyl carbamate- containing acrylic polymers of the invention can self-cross-link to yield thermoset acrylic resins suitable for a number of applications, for example, in the area of coatings. The self-cross-linking reaction may be base catalyzed or tin-catalyzed, which offers significant advantages over prior amino resin systems which require acid catalyzation. Thus, the present invention enables the utilization of a system which is free of formaldehyde and in which cure

inhibition in the presence of hindered amine ultraviolet stabilizers is avoided.

In order to provide a site on the backbone resin on which the amines may be anchored, each molecule of such backbone resin should have at least one, and preferably more, reactive sites thereon which can react with the secondary amine group of the hydroxyalkyl carbamatecontaining amine of the invention. Such reactive sites may include, without limitation, one or more of the following groups:

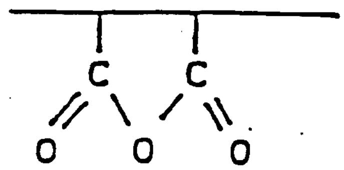

(Anhydrides)

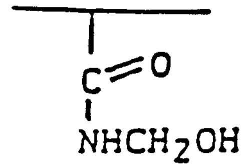

( N-Methylolamides)

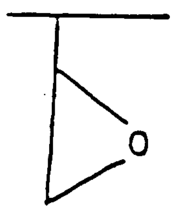

(Epoxies)

—NCO

(Isocyanates)

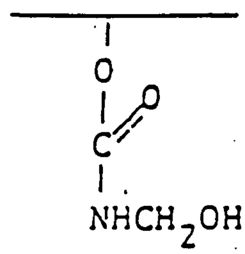

(N-Methylol Carbamates)

Reaction of the secondary amine group of the hydroxyalkyl carbamate-containing amine with acrylic polymers containing functional groups as illustrated above, will result in the formation of the following groups at the functional group sites.

(a)

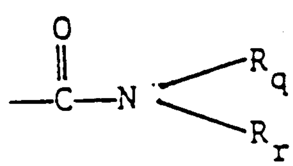

(b)

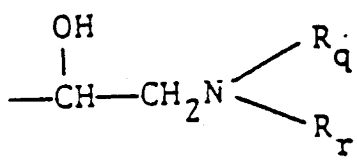

(c)

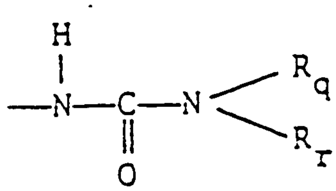

(d)

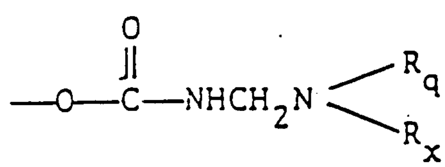

(e)

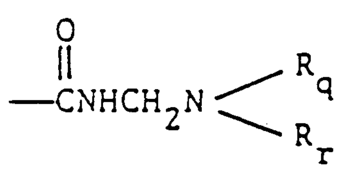

wherein $R_p$ is H or $C_1$ -$C_8$ alkyl; and $R_q$ and $R_r$ may be the same or different and are amine residues containing hydroxyalkyl carbamate moieties.

The repeating units of which the polymer is comprised may fall into two categroies, one comprising units containing the suitable amine-reactive functional groups as described above and the other comprising modifying units selected to impart desired film-making or other properties to the polymer and the finished coating or other product made therefrom. Generally, any suitable repeating units may be employed in the

polymer in any desired combination provided that there exist in the polymer sufficient suitable reactive functional groups for attachment thereto of the primary or secondary amines utilized in the reaction. The polymeric backbone structure may comprise amine-reactive repeating units derived from one or more of glycidyl methacrylate, glycidyl acrylate, isocyanatoethylmethacrylate, maleic anhydride, methacryloyl chloride, n-methloylacrylamide, 1-(1-isocyanato-1- methylethyl) -3- (1-methylenthenyl)benzene, 1-(1-isocyanato -1- methylethyl) -4- (1-methylethenyl) benzene, methyl acrylamido-glycolate, methyl acrylamidoglycolate methyl ether, acryloyl chloride and chloromethyl styrene. The polymeric backbone structure may also include modifying units derived from one or more of acrylic acid, methacrylic acid, butadiene, styrene, alpha-methyl styrene, methyl methacrylate, butyl acrylkate, acrylate, acrylonitrile, hydroxyethyl acrylate, glycidyl methacrylate, acrylamide, methacrylamide, vinyl chloride and vinylidene chloride.

The use of the hydroxyalkyl carbamate-containing amines to prepare the acrylic polymers of the present invention may be illustrated as follows. The hydroxyalkyl carbamate-containing compounds may be reacted with any suitable aclrylic backbone polymer containing, for example, epoxy groups, in which case the reaction may be represented as

(41)

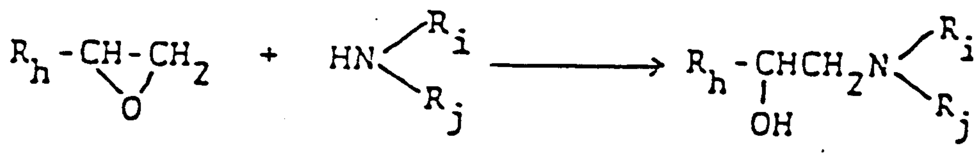

where $R_h$ is a fragment of an epoxy-containing acrylic resin and $R_i$ and $R_j$ are fragments of the hydroxyalkyl carbamate-containing amine or polyamine compounds of the invention. The reaction usually occurs at room or slightly elevated temperatures and is often exothermic. The reaction may be performed without a solvent, otherwise aprotic or alcohol solvents may be used. Any of numerous types of acrylic backbone polymers having any of a variety of reactive functional groups thereon may also be used, as described in more detail below. For example, a typical polymer having anchored thereon one or more of the compositions of the invention may have the formula

(42)

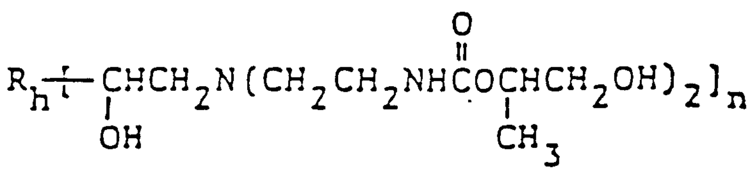

The resultant polymer, upon heating and, optionally, in the presence of a suitable cross-linking catalyst, will cross-link.

In an alternate method of preparing the acrylic polymer, the backbone resins are reacted with amines which contain, in addition to a primary or secondary amine group, hydrolyzable, blocked primary amine groups, e.g., ketimine groups in lieu of some or all of the hydroxyalkyl carbamate groups. After reaction of the primary or secondary amine groups with, e.g., the epoxy groups as described above, so that the amine groups are pendant upon the backbone resin, the ketimine groups are hydrolyzed to form free primary amine groups and one or more suitable cyclic carbonates are then added to the mixture to react with the resultant free amine groups. Thus, the multi-functional amine utilized to form the hydroxyalkyl carbamate-containing amine will contain either amine groups reactable with a cyclic carbonate or groups such as ketimine groups convertible to an amine group reactable with the cyclic carbonate.

A wide variety of acrylic backbone resins containing amine-reactive sites may be employed to react with the hydroxyalkyl carbamate-containing amines, depending on the characteristics desired for the finished coating or other product. The secondary amine-reactive groups of the acrylic resin provide sites on the backbone resin on which the amines may be anchored. The hydroxyalkyl carbamate-containing resin should contain not more than one secondary amine group per molecule inasmuch as the presence of more than one such secondary amine group per molecule will cause gellation.

Any suitable acrylic resin may be utilized as the backbone resin in accordance with the present invention. Any one of a variety of one or more monomers may be utilized to prepare an acrylic resin, and at least one of the monomers utilized contains at least one secondary amine-reactive group. Such monomers

include, by way of illustration and not limitation, alkyl acrylates such as butyl acrylate, butyl methacrylate, ethyl acrylate, 2-ethyl hexyl acrylate, hydroxyethyl acrylate, hydroxyethyl methacrylate, acrylamide, methyl methacrylate, acrylonitrile, glycidyl methacrylate, glcidyl acrylate, vinyl acetate, styrene, substituted styrenes, alpha-methyl styrene, etc. Generally a preferred class of acrylic resins is that containing pendant glycidyl ether groups.

The acrylic resin preferably has an active site content prior to reaction with the secondary amine containing hydroxyalkyl carbamate groups (or precursors thereof, as explained below) of from 0.5 to 7 milliequivalents ("meq") per gram of the acrylic polymer. The backbone resin may also have other functional sites, such as hydroxy and amide groups. It is preferred that the polymer of the invention, and hence the backbone resin, have as low an acid content as possible in order to facilitate low temperature cure of the polymer containing hydroxypropyl, e.g., hydroxypropyl and/or hydroxyethyl, carbamate groups. The monomers from which the acrylic resin is prepared are sometimes contaminated with acids, such as methacrylic or propionic acids, in amounts of, e.g. 0.3 to 1% by weight. It is preferred that the acid content of the polymer of the invention be not more than about 0.1% by weight of the polymer in order to attain relatively low temperature cures. The acrylic polymer itself may also have 0.5 to 10 meq per gram of pendant or terminal hydroxylakyl, e.g., hydroxypropyl and/or hydroxyethyl, carbamate groups.

The acrylic polymers of the invention can be modified by reacting them with selected organic secondary amines (in addition to the hydroxyalkyl carbamate-containing amines) such as diethylamine, dibutylamine, morpholine, n-methylaniline, and the like. The selection and combination of specific modifying secondary amines of course depends on the end use of the polymer. For example, in the preparation of water-reducible polymers one or more hydrophilic secondary amines may be reacted with some of the functional sites on the backbone resin in order to enhance hydrophilicity of the polymer. Conversely, for applications where hydrophobicity of the polymer is desired, for example, for electrodepositable coatings, it may be necessary or desirable to react one or more hydrophobic secondary amines with the backbone resin to impart the desired degree of hydrophobicity to the polymer. Generally, the polymers of the invention are well suited for utilization in the field of coatings and in such case preferably have molecular weights in range of from about 1,000 to about 50,000, more preferably in the range of from about 2,000 to about 20,000. The urethane cross-linkable polymers of the invention are particularly well suited for a variety of uses in the field of coatings, such as solvent or water based coatings, powder coatings, electrocoating compositions, spray roller and dip type coatings, and the like. Such coatings are normally applied to a substrate such as a metal, textile, plastic or paper. The thermosetting resins of the invention may be used to prepare urethane coatings which are resistant to organic solvents and water and are abrasion-resistant, as well as adhesives and laminating resins.

A coating formulation containing the acrylic polymers of the invention may be modified by incorporating into the formulation other additives such as amino formaldehyde resins, reactive diluents, etc. to change the physical, chemical and mechanical properties of the resulting coatings.

The acrylic polymers described in this invention are particularly useful as binders for conventional solvent based coatings, electrocoating, powder coating, etc., and may be formulated with or without a catalyst.

Generally, the primer coatings in the tenth aspect of the invention may be applied to any structural material including aluminum, aluminum alloys, titanium, titanium alloys, steel, glass, natural and synthetic rubbers, synthetic organic polymeric materials, or combinations of the foregoing. As the primer coatings must be heated in order to cure, the structural members to which they are applied must, of course, be able to withstand the temperature necessary to cure the coating deposited thereon. The cure temperature may be as high as 400°F (204°C) or higher although, as described in more detail below, in some applications, lower temperatures, on the order of about 200 to about 250°F (about 93 to about 121°C), and duration of curing, up to about one hour, e.g., about 30 minutes, may be employed.

The adhesives utilized in the bonding means in this aspect of the present invention may comprise any suitable structural adhesive. For a general description of appropriate structural adhesives see, for example, the book Adhesives for Metals - Theory and Technology by Nicholas J. DeLollis, Industrial Press Inc., New York, New York, 1970, at page 81 et seq. Generally, epoxy adhesives or copolymers of phenol with materials such as neoprene, nitrile rubber (Buna N), vinyls, such as polyvinyl butyral, and the like materials, or the new high temperature resistant condensation or addition polyimide adhesives are commonly employed in such use. Significant classes of structural adhesives which are particularly well suited for use in the present invention are epoxy resin adhesives, including phenolic copolymers with epoxy resins. Epoxy phenolics generally have good resistance to high temperature, which makes them well suited for use in aircraft surfaces which are exposed to elevated temperatures at high speed. The epoxy phenolic structural adhesives meet Federal Government specification requirements for exposure to temperatures of about 300

to 500° F (148.9 to 260°C) for extended periods. Generally, such adhesives comprise an epoxy resin, a phenolic resin, and an amine curing agent, with optionally other additives such as aluminum powder to act as an oxygen "getter".

Another well suited class of adhesives are the condensation or addition polyimides. These show outstanding thermal and oxidative stability. For example, see the review article by J.R. Fowler in Materials and Design, Vol. 3, December 1982, pp 602 - 607. At 250° - 300°C a useful life measured in thousands of hours is common for these polyimide adhesives.

The metal or other surfaces on which the primer coating is to be deposited will normally be scrupulously cleaned by known techniques just prior to depositing the primer coating. The coating may then be cured or air or oven dried so that the parts can be handled. Upon assembly, the adhesive, which isusually in the form of a thin film supported on a peelable carrier material, is positioned over the primer coated surfaces and the carrier material is peeled away to leave the adhesive layer. The parts are then clamped together in the proper position with the adhesive layer between the primer coated surfaces and heated to effect a cure of the adhesive (and of the primer coating if it has not previously been cured) into a unitary structural bond. In the aircraft industry, protective primer coatings are widely used with epoxy, nitrile rubber phenolic, nylon epoxy, condensation and addition polyimide and epoxy phenolic adhesives.

The primer coatings for this aspect of the invention are made from polymers comprising hydroxyalkyl carbamate-containing amines which, under curing conditions, cross-link to provide an adherent and stable coating composition. The hydroxyalkyl carbamate-containing polymers may be hydrophobic polymers which are electrodeposited onto the member to be bonded. Alternatively, the coatings may be made of hydrophilic polymers which are applied from an aqueous medium.

The hydroxyalkyl carbamate-containing amine is made and then reacted with a water insoluble, epoxide-containing "backbone" compound, as previously described herein.

The epoxide material utilized may be any suitable monomeric or polymeric epoxy containing material, preferably a resinous polyepoxide material containing two or more epoxy groups per molecule. In addition to the epoxides described previously herein resins of the following formulas have been found to be well suited to the practice of the present invention:

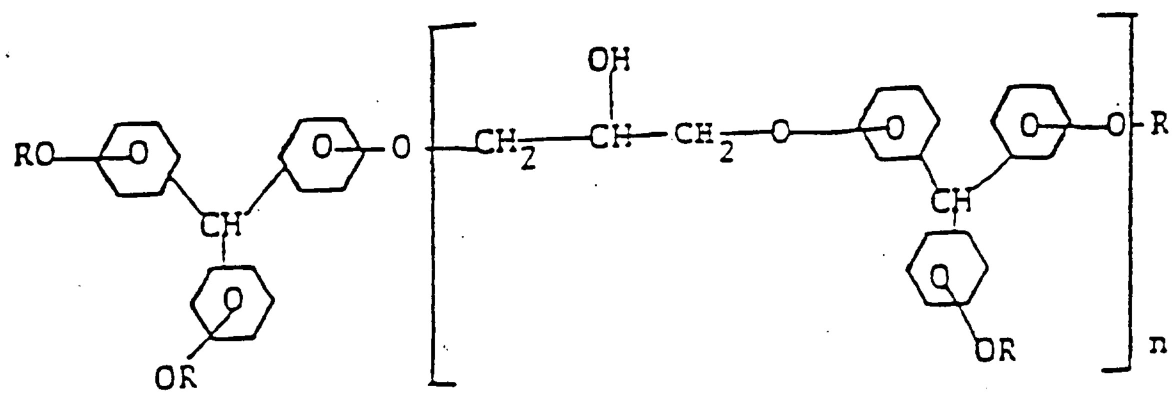

wherein,

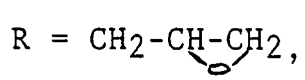

n is 0 to about 5 and preferably about 0 to about 0.7, on average; and

(b) triglycidyl isocyanurate polyepoxy resins general formula:

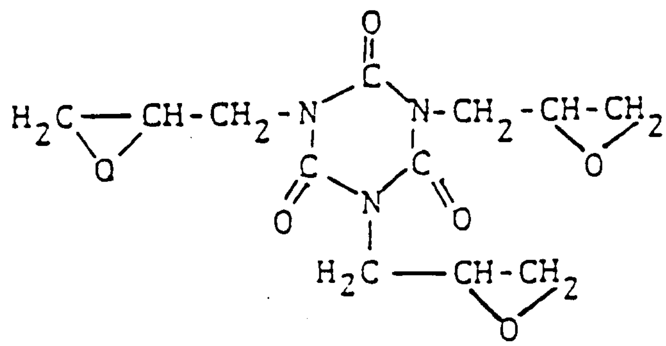

As used herein, "tris (hydroxyphenyl) methane based resins" means resins of formula (a) above wherein n is 0 to 5, and "triglycidyl isocyanurate resin" means a resin of formula (b) above.

The secondary amines which are reacted with the epoxy resin to obtain the polymer of the invention are prepared by reacting a cyclic carbonate with a "multi-functional amine" by which term is meant an amine containing at least one primary amine group (which may be a blocked primary amine groups as described below) and at least one hindered secondary amine group.

In this way, the secondary group survives the carbonate reaction and is available to react with active epoxy sites of the epoxide to form the self-cross-linkable polymer which is deposited on the member and cured to form the primer coating.

The efficacy of the invention is demonstrated by the following examples of specific preferred embodiments thereof. Examples 1 and 2 illustrate the preparation of respectively a carbamate and ketimine group-containing amine and a carbamate group-containing amine.

EXAMPLE I

51 G (0.5 mole) of diethylenetriamine in 150 ml. of methanol were added, at 15° C, to 168 g (1.65 moles) of propylene carbonate. After completion of addition, the materials were allowed to react for 24 hours at 25°C. Potentiometric titration indicated the presence of free secondary amine. The reaction mixture was then heated to 70° C and held at that temperature for 3 hours. Additional potentiometric titration indicated very little change in the free amine content. Methanol was removed from the reaction mixture leaving behind a syrupy liquid comprised of about 72% solids containing bis(2-hydroxy-1-methylethyl)-(imminodiethylene)biscarbamate.

EXAMPLE 2

To a 3-neck flask suitably equipped with a stirrer and a thermometer were added 103 g (1 mole) of diethylenetriamine and 200 g or methanol. To the resulting methanolic solution of diethylenetriamine, 184.8 g (2.1 moles) of ethylene carbonate were added in slow increments. At the completion of the ethylene carbonate addition the reaction temperature had risen from room temperature to 65° C. The reaction mixture was allowed to react for several hours without any external heat being supplied. After removal of methanol by distillation, the residue solidified on standing. The solid residue was recrystalized from ethanol as white solids and isolated in 90% yields. The product was characterized by I.R., N.M.R. and potentiometric titration to be bis(2-hydroxyethyl) (imminodiethylene) biscarbamate, m.p. 98° C.

EXAMPLE 3

408 g (4 moles) of propylene carbonate and 300 g of methanol were placed in a 3-neck flask suitably equipped with a stirrer and a thermometer. Under a nitrogen blanket, 292 g (2 moles) of triethylenetetramine were slowly added, maintaining the temperature between 15 to 30° C by external cooling. After addition was completed, the reaction mixture was heated to 80° C for about 8 hours. After this period almost all propylene carbonate had reacted as indicated by I.R. of the reaction mixture. Methanol was then removed by distillation. On standing at room temperature the product solidified to a low melting waxy solidl The I.R. and potentiometric titration of the product were consistent with the following bishydroxyporpyl carbamate structure and the amine equivalent weight found was 210 (calculated value, 175).

EXAMPLE 4

Diethylenetriamine in the amount of 206 grams (2 moles) and 600 grams of methanol were added to a suitable reactor. 612 Grams (6 moles) of propylene carbonate, which amount comprises 2 moles in excess of the stoichiometric amount, was slowly added to the reactor under a nitrogen blanket while the temperature of the reactants was maintained at 15 to 20° by ice bath cooling. After complete addition, the mixture was stirred 8 hours at room temperature. Methanol was then removed by use of water pump vacuum and with steam bath heating. The resulting product solution comprised diethylenetriamine bishydroxypropyl carbamate and was 73% solids in propylene carbonate (theory 75% solids), had 2.16 meq/g secondary amine (theory 2.37 meq/g at 73% solids), and gave characteristic bands in the infrared for the hydroxypropyl carbamate groups.

EXAMPLE 5

Diethylenetriamine in the amount of 206 grams (2 moles) was added, to a suitable reactor equipped with an inlet for a nitrogen atmosphere and with a decanting trap in the distillate return line. The reactor was cooled in an ice bath and propylene carbonate (306 grams, 3 moles) was slowly added with good stirring, while maintaining the temperature below 40°C. Upon complete addition, the reactor was heated and stirred at 80° C for 2 hours after which time no unreacted proyylene carbonate could be detected by infrared analysis. To the reactor was then added 300 grams (3 moles) of methyl isobutyl ketone (MIBK) and the contents were brought to reflux. After refluxing approximately two hours, the theroetical amount of water was collected in the decanting trap and the reactor was cooled. The resulting product comprising a mixed carbamate/ketimine of diethylenetriamine was 73% solids in MIBK (theory 74.8% solids). Non-aqueous potentiometri titration for secondary amine disclosed 2.58 meq/g amine (theory for 73% solids is 2.46 meq/g) and the infrared showed the characteristic bands for hydroxypropyl carbamate and ketimine groups.

EXAMPLE 6

Preparation of N,N-bis(6-aminohexyl)-2-[(6-aminohexyl) amino]butanediamide

Dimethyl maleate (72 grams, 0.5 moles) was added over a 2 to 3 hour period to a solution of 174 grams (1.5 moles) of 1,6-hexanediamine in 360 grams of toluene at 75 to 80° C. The reaction temperature rose from 80 to 110° C under reflux conditions. After the addition was completed, methanol was distilled at a reaction temperature of 120 to 125° C. Additional toluene (320 grams) was added to maintain reaction volume. The course of the reaction may be followed by amine titration or by disappearance of the methyl ester in the 'H NMR spectrum. After the reaction was complete, toluene was removed under vacuum (50 to 70° C, 15 to 20 mm Hg) to give a viscous liquid which solidified on standing. Potentiometric analysis indicated a 3/1 ratio of primary to secondary amine. NMR and IR spectra was consistent with the desired N, N-bis(6-aminohexyl)-2[6-aminohexyl)amino]butanediamide structure.

The following example illustrates the preparation of a tris(hydroxyalkyl carbamate) compound of the invention utilizing the diamide of Example 6.

EXAMPLE 7

To 100 grams (0.125 mole) of a 50% solution of the diamide of Example 6 in ethanol, were added 36 grams (0.32 mole) of propylene carbonate and 35 grams of ethanol. The reaction mixture was allowed to react at 25° C, overnight. The titration of the reaction mixture indicated that essentially all the primary amino groups had reacted and the secondary amine remained free. After removal of the solvent, a syrupy product was obtained mainly consisting of a compound of the following structure:

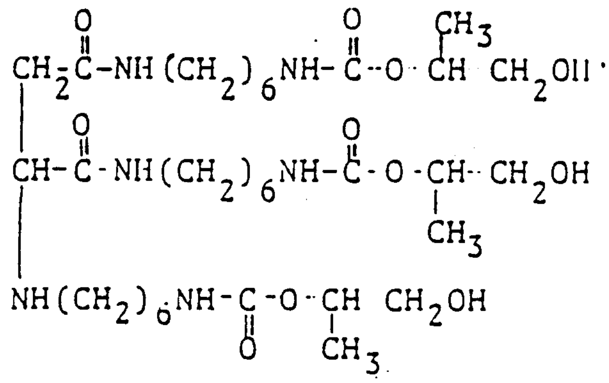

EXAMPLE 8

A. A self-cross-linking novalac hydroxypropyl carbamate-containing resin which can be reduced with water to at least 12% solids as a clear solution with 20% by total weight of cosolvent was prepared from the following ingredients:

| | Parts by Weight | Equivalents | Solids |
|---|---|---|---|
| EPN 1139* | 128.3 | 0.75 | 128.3 |
| Carbamate-containing Amine of Example 4 | 347.3 | 0.75 | 253.5 |

*Ciba Geigy Co. reaction product of phenol-formaldehyde condensate with epichlorohydrin

The EPN 1139 and carbamate-containing amine of Example 4 were added under nitrogen to a suitable reactor equipped with a Cowels high speed stirrer, controlling the exotherm by external cooling when necessary. The final product had a solids content of 80%.

B. A sprayable aqueous composition was prepared by dissolving 100 parts of the novalac-hydroxypropyl carbamate-containing resin of part A of this Example in 100 parts of deionized water and 15.4 parts of 1 Molar tetrabutyl ammonium hydroxide catalyst. The resulting clear solution contained only 9% organic solvent and was 39% soldis. Aluminum panels were sprayed and then baked at 250° F for 20 minutes. The resulting cured coatings were 0.5 - 0.6 mil thick, had 4H pencil hardness, passed 40 in-lb reverse impact, were smooth and glossy, and resisted greater than 300 MEK and water double rubs.

EXAMPLE 9

A. A self-cross-linking cathodic electrocoating composition containing tertiary amine groups and ketimine groups is prepared from the following ingredients:

| | Parts by Weight | Equivalents |
|---|---|---|
| EPON 1004F* | 780.0 | 1.00 |
| MIBK | 176.0 | - |
| Carbamate-containing Amine of Example 5 | 302.0 | 0.78 |
| Propasol P** | 105.0 | - |
| EPON 1001F* | 96.8 | 0.20 |
| Diethylamine | 32.0 | 0.44 |

*A trademark of Shell Chemical Co. for its product comprising the reaction product of epichlorohydrin and bisphenol-A.
**A trademark of Union Carbide Corp. for its propoxypropanol product.

B. The EPON 1004F and MIBK were charged under nitrogen in a suitable reactor and the mixture was heated to reflux with stirring to remove any water present. At 80°C the carbamate-containing amine of

Example 5 was added followed by the EPON 0001F and one-half of the Propasol P. The mixture was stirred and held at 80°C for two hours and then the diethylamine dissolved in the remaining Propasol P was slowly added so as to prevent volatilization. The mixture was then stirred and heated at 85°C for 8 hours. 150 Grams of Propasol P was then added and removed by vacuum distillation (flask temperature 110 to 120°C). This process was repeated with another 150 grams of Propasol P. The resultant product comprised 76% by weight resin solids and 0.91 meq amine per gram; based on 100% resin solids.

C. An electrodeposition bath was prepared by combining 50 grams of the self-cross-linking cathodic electrocoating resin obtained in part B of this Example with 5 grams of hexyl Cellosolve, 5 grams of benzyl hydroxypropyl carbamate (a reactive diluent-flow agent prepared by reacting one equivalent of benzyl amine with one equivalent of propylene carbonate and removing any residual amine with acidic ion-exchange resin); 10 grams of EPON 828 (Shell chemical Co.), 1.54 grams of 89.9% formic acid, and 1.63 grams of dibutyltindilaurate. 376 Grams of deionized water was then slowly added while rapidly mixing with a Cowels stirrer to produce a bath containing approximately 12% solids. The bath had a pH of 4.4, a conductivity of 1300 micromho $cm^{-1}$, and a rupture voltage of 270 volts.

D. The bath composition obtained in part C of this Example was applied, by electrodeposition, to aluminum panels serving as the cathode at 75V for 20 seconds to deposit a thin resin coating on the panels. The panels were then baked at 175°C for 20 minutes and afforded film builds of 0.4 mils. The coatings were smooth, flexible, had 4H pencil hardness, passed 40 in-lb impact tests, and resisted greater than 200 methyl ethyl ketone double rubs.

EXAMPLE 10

A sprayable composition was prepared by dissolving 100 parts of the hydroxyalkyl carbamate-containing amine of Example 3 in 100 parts of deionized water and adding 11.6 parts of 1 Molar tetrabutyl ammonium hydroxide catalyst. The resulting solution, which was 48% solids in water, was sprayed onto aluminum panels to deposit a coating thereon. The thus-coated panesl were cured at 300°F for 20 minutes. The cured film thicknesses were 0.3 to 0.4 mil. The coatings were slightly hazy but smooth, had F pencil hardness, passed 40 in-lb impact and resisted 300 MEK double rubs. However, 40 rubs with water removed the films from the panels.

EXAMPLE 11

To a suitable reactor containing 103 grams (1 mole) of diethylenetriamine and 300 grams of solvent methanol under a nitrogen atmosphere 184.8 grams (2.1 moles) of ethylene carbonate was slowly added. The temperature was maintained at 15 to 20°C by ice bath cooling. After complete addition, the mixture was stirred at room temperature overnight. Methanol was then removed by use of a water pump vacuum and with steam bath heating. The resulting product solidified to a mass of white crystals upon cooling, mp 82 to 88°C. Recrystallization from ethanol afforded a pure product mp 96 to 97°C, in nearly quantitative yield. The product gave completely consistant IR and NMR spectra for the bis-hydroxyethyl carbamate of diethylenetriamine, i.e., diethylenetriamine bis-hydroxyethyl carbamate.

EXAMPLE 12A

To 24 grams of 1,2-dimethoxyethane were added 7.1 grams of glycidyl methacrylate and 13.7 grams of the hydroxyalkyl carbamate of Example 11. The reaction mixture was heated to 85°C for four hours and 1,2-dimethoxyethane was removed by distillation under reduced pressure. The resulting water white product was viscous and glassy and contained 0.3 millimols per gram of free residual epoxy groups. The mass spectrum showed a major peak corresponding to the mass of the desired product of the following structure:

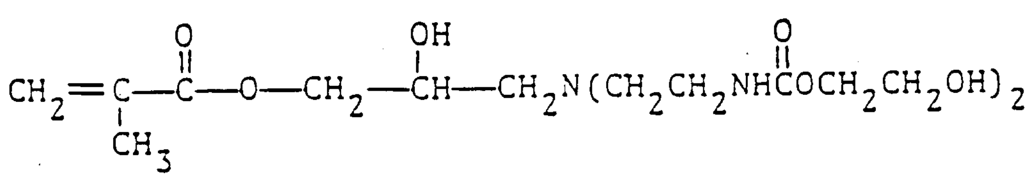

EXAMPLE 12B

To a suspension of 27.9 grams of the hydroxyalkyl carbamate of Example 11 in 100 grams of t-butanol was added 20.8 grams of 1- (1-isocyanato -1-methylethyl) -3-(1-methylethenyl)benzene. The reaction mixture was stirred with a high speed stirrer. Most of the solids dissolved after 2 hours and I.R. showed a small amount of unreacted isocyanate. The reaction mixture was stirred overnight, then filtered to remove trace amount of insolubles, and t-butanol was distilled from the filtrate under reduced pressure. The N.M.R. of the resulting product showed it to be of the following structure:

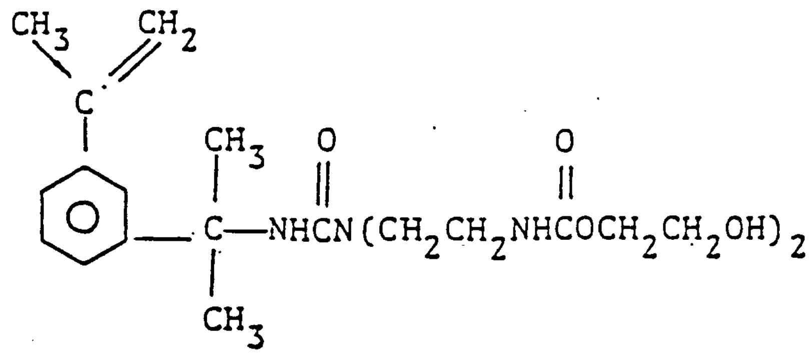

EXAMPLE 12-A-1

To a suitable 3 neck flask equipped with stirrer and a thermometer are added 50 grams of 2-ethoxyethanol. At reflux temperature, a blend of 60 grams of N-butylacrylate, 20 grams of styrene, 59 grams of the monomer of Example 12A 1 gram of N-dodecyl mercaptan and 2 grams of dicumyl peroxide in 50 grams of 2-ethoxyethanol is added to the flask over a period of 2 hours. The reaction mixture is held at 145°C for 2 hours. The resulting resin of 58% solids has a basic nitrogen content of 1 meq per gram of resin solids, and an hydroxyethyl carbamate content of 2 meq per gram of resin solids.

EXAMPLE 12B-1

To a suitable 3 neck flask equipped with a stirrer and a thermometer are added 175 grams of toluene. At reflux temperature a blend of monomers comprising 58.9 grams of N-butylacrylate, 45 grams of methyl methacrylate and 71.2 grams of the hydroxpropyl carbamate-containing monomer of Example 12B is added to the toluene along with 4 grams of t-butylperbenzoate as the initiator. After 4 hours at reflux a resin containing hydroxypropyl carbamate groups is obtained. The resin comprises 50% solids and contains 1.65 meq hydroxypropyl carbamate per gram of resin solids.

EXAMPLE 13

A. A self-cross-linking bisphenol-A hydroxpropyl carbamate- containing resin which can be reduced with water to at least 15% solids as a clear solution with only 20% by total composition weight of cosolvents was prepared from the following ingredients:

| | Parts by Weight | Equivalents | Solids |
|---|---|---|---|
| EPON 828* | 150.9 | 0.82 | 150.9 |
| Carbamate-containing Amine of Example 4 | 380.0 | 0.82 | 277.4 |

*Shell Chemical Co. reaction product of epichlorohydrin and BP-A

The EPON 828 and the carbamate-containing amine of Example 4 were added to a suitable reactor under nitrogen equipped with a Cowels high speed stirrer. Upon stirring, the temperature was allowed to reach 100°C (heat of exotherm) and was then maintained at this temperature by external cooling for one hour. After this, the mixture was stirred and heated at 70°C for 4 hours more. The final product had a solids content of 80%.

B. A sprayable aqueous composition was made up by mixing 191.4 parts of the resin obtained in part A of this Example with 191.4 parts of deionized water and 15.3 parts of ethylene glycol monobutyl ether cosolvent. The resulting clear solution contained only 13.5% by total weight organic cosolvents and was 38.5% solids. To this solution was added 14.5 parts of aqueous 1 Molar tetrabutyl ammonium hydroxide catalyst and the contents were well stirred. This composition was applied, by spraying, to aluminum panels. The panels were baked at 250°F (121° C) for 20 minutes and showed film thickness of 0.3 to 0.4 mil after cure. The coatings were smooth, glossy, had 4H pencil hardness, passed 40 in-lb reverse impact, and resisted greater than 300 water and methyl ethyl ketone (MEK) double rubs.

EXAMPLE 14

A. A self-cross-linking bisphenol-A hydroxyethyl carbamate-containing resin which can be reduced with water to at least 10% solids as a clear solution with 20% by total weight of cosolvent was prepared from the following ingredients:

| | Parts by Weight | Equivalents | Solids |
|---|---|---|---|
| EPON 828 | 85.7 | 0.466 | 85.7 |
| Carbamate-containing Amine of Example 11 | 130.0 | 0.466 | 130.0 |
| Butyl Cellosolve* | 53.9 | - | 0.0 |

*Monobutyl ether of ethylene glycol

The EPON 828, carbamate-containing amine of Example 11, and butyl Cellosolve were mixed and reacted as in Example 13. The final product had a solids content of 80%.

B. A composition for spray application was prepared by dissolving 100 parts of the BP-A hydroxyethyl carbamate of part A of this Example in 100 parts of deionized water and 7.7 parts of 1 Molar tertabutyl ammonium hydroxide catalyst. The resulting clear solution contained only 10% organic cosolvent and was 39% solids. After spraying aluminum panels and then baking at 250°F for 20 minutes, the film thicknesses were 0.3-0.4 mil. The coatings were smooth and glossy had 4H pencil hardness, passed 40 in-lb reverse impact, and resisted greater than 300 MEK and water double rubs.

EXAMPLE 15

A. A self-cross-linking saturated hydroxyethyl carbamate-containing resin which can be reduced to at least 20% solids with 20% by total weight cosolvent was prepared from the following ingredients:

| | Parts by Weight | Equivalents | Solids |
|---|---|---|---|
| Eponex DRH 151* | 100.0 | 0.42 | 100.0 |
| Carbamate-containing Amine of Example 2 | 112.0 | 0.40 | 112.0 |

*Eponex DRH 151 - Shell Chemical Co. Hydrogenated BP-A epichlorohydrin product.

The Eponex DRH 151 and carbamate-containing amine of Example 2 were reacted in the same manner as described in Example 4, carefully controlling the exotherm with external cooling when necessary. The final product was 100% solids. This product was reduced to 80% solids with ethylene glycol monobutyl ether.

B. A sprayable composition was prepared by mixing 100 parts of the saturated hydroxyethyl carbamate of Part A of this Example with 100 parts of deionized water and 15.4 parts of 1 Molar tetrabutyl ammonium hydroxide catalyst. The resulting clear solution at 39% solids and containing 9% organic cosolvents was sprayed onto aluminum panels. The panels were cured at 270°F for 20 minutes and afforded film thicknesses of 0.4 to 0.5 mil. The coatings were smooth, glossy, had 3H pencil hardness, passed 40 in-lb reverse impact and resisted greater than 200 MEK rubs and 300 water rubs.

EXAMPLE 16

A. An acrylic-urethane polymer is prepared as follows. 100 Grams 2-ethoxyethanol was added to a 3-neck flask suitably equipped with stirrer, thermometer, and a condenser and heated to 135°C. Over a two hour period, a blend of 60 grams of n-butyl acrylate, 20 grams of styrene, 20 grams of glycidyl methacrylate, 2 grams of dicumyl peroxide, and 1 gram of n-dodecyl mercaptan was added to the heated 2-ethoxyethanol. After the complete addition of the monomer blend containing an initiator and a chain transfer agent, the reaction temperature was held at 140°C for two hours. Throughout the polymerization reaction a nitrogen blanket was maintained in the reactor. The resultant resin had solids content of 47% and epoxy content of 0.64 meq per gram.

B. To the acrylic resin obtained in part A of this Example was added 36 grams (0.13 mole) of bishydroxyethyl carbamate derivative of diethylenetriamine (the hydroxy-alkyl carbamate of Example 2) and the mixture was heated to 80°C for two hours and then to 120°C for two hours. The resulting resin had the following characteristics. Solids: 56.5%, Viscosity: U-V, Gardner Color: 5-6, Basic Nitrogen Content: 0.51 meq per gram and Epoxy Content: less than 0.05 meq per gram.

Four clear coating formulations were prepared by blending the polymer of Example 16 with various catalysts as shown in Table I. These coating formulations were cast on zinc phosphate pretreated cold rolled steel panels and cured at elevated temperatures. The cure conditions and film properties are shown in Table II. The results in Table II show that the polymer of Example 16 self-cross-links at temperatures as low as 125°C in 20 minutes. Tin catalyzed cross-linked acrylic-urethane films show good humidity and salt spray resistance. All the self-cross-linked films show excellent resistance to methyl ethyl ketone double rubs. Use of a hydrophobic quaternary catalyst such as methyltricaprylyl ammonium hydroxide results in self_cross-linked films which are better in humidity and sale spray resistance than those obtained from the formulation containing a hydrophilic quaternary benzyltrimethyl ammonium hydroxide.

Table I

| | Parts by Weight | | | |
|---|---|---|---|---|
| Formulation: | I | II | III | IV |
| Contents | | | | |
| The Polymer of Example 16 | 10 | 10 | 15 | 23 |
| Dibutyltindilaurate | 0.1 | - | - | - |
| Benzyltrimethylammonium hydroxide (40% in methanol) | - | 0.2 | - | - |
| Tetrabutyldiacetoxy stannoxane | - | - | 0.2 | - |
| Methyltricaprylylammonium hydroxide (72%) | - | - | - | 0.7 |

Table II

| Formulation | I | I | II | II |
| --- | --- | --- | --- | --- |
| Cure Schedule | 175° C 20 min. | 150° C 20 min. | 122° C 20 min. | 150° C 20 min. |
| Film Thickness (micro-meters) | 31 | 25 | 25 | 28 |
| Knoop Hardness | 13.7 | 6.6 | 7.4 | 7.1 |
| Pencil Hardness | H-2H | F-H | H-2H | H-2H |
| Impact (Reverse in. lbs.) | 30-40 | 60 | 80 | 80 |
| MEK Rub Resistance | 100+ | 100+ | 100+ | 100+ |
| Humidity Resistance (65° C) | 21 days | 21 days | 2-3 hours | 2-3 hours |
| Salt Spray Resistance | 200 hours | 200 hours | Less than 8 hrs | Less than 8 hrs |

TABLE II (Continued)

| Formulation | III | III | IV | IV |
|---|---|---|---|---|
| Cure Schedule | 122°C 60 min. | 150°C 20 min. | 125°C 20 min. | 150°C 20 min. |
| Film Thickness (micro-meters) | 22 | 22 | 38 | 33 |
| Knoop Hardness | 3.9 | 7.3 | 7.1 | 8.3 |
| Pencil Hardness | F-H | H-2H | F-H | F-H |
| Impact (Reverse in. lbs.) | 60 | 60 | 30-40 | 20-30 |
| MEK Rub Resistance | 100+ | 100+ | 100+ | 100+ |
| Humidity Resistance (65°C) | - | - | 24 hours | 3 days |
| Salt Spray Resistance | - | - | 60 hours no corrosion. Loss of adhesion at scribe | 60 hours no corrosion. Loss of adhesion at scribe |

EXAMPLE 17

A clear electrocoating system was prepared by emulsification of 70 grams of the polymer of Example 16 with deionized water in the presence of 1 gram of dibutyltindilaurate catalyst and 1.1 grams of acetic acid, to 10% solids. The pH and conductivity of the 10% solids electrocoating emulsion were 4.8 and 175 micromho $cm^{-1}$ respectively. Zinc phosphate pretreated cold rolled steel panels were electrocoated at 100 volts for 30 seconds and later rinsed with deionized water. The electrocoated panels were cured at 150°C and 175°C, respectively, for 20 minutes. The resulting films had good organic solvent resistance and good hardness as shown below.

Film Properties of Electrocoated Films of Part A of this Example

| | | |
|---|---|---|
| Cure Schedule | 150 ° C, 20 min. | 175 ° C, 20 min. |
| Film Thickness (micrometers) | 17.5 | 22.5 |
| Knoop Hardness | 5.8 | 9.3 |
| MEK Rubs | 200 + | 200 + |
| Impact (Rev) in-lbs. | 40 | 30-40 |
| Salt Spray Resistance (120 hrs.) (ASTM D610-68) | 8;5 mm | 8;3 mm |

Generally, the polymers of the invention may be applied in any suitable way to a substrate and may be heat-cured. By utilization of a suitable cross-linking catalyst as described above, heat curing may be attained at temperatures from about 200 to about 400°F (about 93 to about 205°C). The applied composition may comprise the polymer, a suitable catalyst and a liquid vehicle, such as an aqueous or organic solvent vehicle.

EXAMPLES 18 - 22

The coating compositions of the following Examples comprise a formulation prepared by blending the following components and diluting the blend with Cellosolve acetate to Ford Cup #4 viscosity of 41-43 seconds.

TABLE I

The coating compositions of the following Examples comprise a formulation prepared by blending the following components and diluting the blend with Cellosolve acetate to Ford Cup #4 viscosity of 41-43 seconds.

| Component | Parts by Weight |
|---|---|
| Acrylic Resin (75% solids ) (1) | 66.6 |
| Hexamethoxymethylmelamine (2) | 35 |
| Reactive Diluent (3) | 15 |
| PTSA catalyst (4) | 1.0 |
| n-Butanol | 15 |

(1) A commercially available acrylic resin sold under the trademark ACRYLOID AT-400.
(2) A commercially available cross-linker sold by American Cyanamid Company under the trademark CYMEL 303.
(3) See line 1) of Table II, below.
(4) A commercially available 40% solution of PTSA in isopropanol 1% based on TRS, sold by American Cyanamid Company under the trademark CYCAT 4040-A. (PTSA=paratoluene sulfonic acid).

Table II

| Formulation Compositions and Characteristics | | | | | |
|---|---|---|---|---|---|
| Example | 18 | 19 | 20 | 21 | 22 |
| 1) Reactive Diluent of formula (*): | None | (A) | (B) | (C) | (D) |
| 2) Viscosity, FC 4, sec. | 43 | 43 | 41 | 42 | 40 |
| 3) NV %, 45° C/45 min. | 62.1 | 68.2 | 64.7 | 64.8 | 67.2 |
| 4) NV %, 125° C/20 min. | 56.7 | 60.6 | 56.1 | 56.4 | 60.2 |
| 5) % Wt. Retention | 91 | 89 | 87 | 87 | 89.5 |
| 6) % NV Advantage Over Control | -- | 3.9 | 0 | 0 | 3.5 |
| 7) Stability at 50° C (Viscosity, FC 4, after 4 days) | 300 | 130 | 107 | 150 | 101 |

(*) The reactive diluents of formulas (A) - (D) above were used in, respectively, the compositions of Examples 19, 20, 21 and 22. In lines 3), 4) and 6) NV = Non-Volatiles.

The above formulations were applied to a zinc phosphated cold rolled steel substrate and heated to cure at a temperature of 125°C for twenty minutes.

The film coatings obtained from the compositions of the Examples displayed the properties set out in Table III.

Table III

| Film Properties | | | | | |
|---|---|---|---|---|---|
| Example | 18 | 19 | 20 | 21 | 22 |
| Film Thickness (mils) | 1.45 | 2 | 1.1 | 1.1 | 1.0 |
| Knoop Hardness | 10.9 | 13.6 | 14.8 | 15.6 | 9.0 |
| Pencil Hardness | F-H | F-H | 2-3H | 2-3H | F-H |
| Adhesion | 5 | 5 | 4-5 | 4-5 | 5 |
| Impact Resistance (in-lbs) (Rev) | 10 | <10 | <10 | <10 | <10 |

## EXAMPLE 23

A polyether polyurethane diol is prepared by adding to a suitably equipped flask 32 grams (0.10 M) of polytetramethylene glycol (sold under the trademark Teracol 650); 78 grams (0.12 M) of hexamethylene bis-(hydroxypropyl) carbamate, prepared following a literature procedure (Bull. Chim. Soc. Fr. 1142, 1954); and 0.6 grams dibutyltindilaurate. The reaction mixture was heated to 175°C under reduced pressure.

A distillate carried over under reduced pressure was mainly propylene glycol and a small amount of propylene carbonate. About 12 grams of distillate was collected before terminating the reaction after 2 hours. On cooling, 75 grams of Cellosolve was added to the resulting product which by i.r. showed urethane linkages and very few urea linkages. The final resin solids was 57 ± 2% by weight and the resin displayed a very high viscosity.

## EXAMPLE 24

A polyester polyurethane diol was prepared following the procedure of Example 23, except that 53 grams (0.1M) PCP polyol 0200 was used in place of the Teracol 650 polytetramethylene glycol. The i.r. of the product showed the presence of ester and urethane linkages. No urea or amide links were present. The final resinous product was diluted with 25 grams of Cellosolve. The final resin solids and Gardner-Holdt viscosity were 51 ± 2% by weight and A-B, respectively.

## EXAMPLES 25-30

The urethane group-containing resins of Examples 25 - 30 below were formulated with a methylated melamine-formaldehyde resin in the presence of a phosphoric acid ester based catalyst to prepare coating

compositions as set forth in the Table below.

| Example | 25 | 26 | 27 | 28 | 29 | 30 |
|---|---|---|---|---|---|---|
| Weight Ratio (Solids Basis) of Urethane to Amine Resin | 80/20 | 80/20 | 65/35 | 65/35 | 50/50 | 50/50 |
| | Parts by Weight | | | | | |
| Polyurethane of Ex.23 | - | 35.1 | - | 28.5 | - | 21.9 |
| Polyurethane of Ex.24 | 39.2 | - | 31.9 | - | 24.5 | - |
| Methylated Melamine formaldehyde resin* | 6.3 | 6.3 | 10.9 | 10.9 | 15.6 | 15.6 |
| Catalyst** | 0.13 | 0.13 | 0.13 | 0.13 | 0.13 | 0.13 |
| Cellosolve Acetate | - | 9.6 | - | 10.6 | - | 11.5 |

*Sold under the trademark CYMEL 325 by American Cyanamid Company. Solids content is 85 ± 2% by weight
**Sold under the trademark CYCAT 296-9 by American Cyanamid Company

Draw-downs of the above compositions were made on bonderite 100 supports, using a No. 46 wirecater, held-out 10 minutes. The thus applied coatings were cured at 125°C for 20 minutes. The characteristics of the cured coatings thus obtained are shown in the following Table.

Table

| Cured Composition of Example: | 25 | 26 | 27 | 28 | 29 | 30 |
|---|---|---|---|---|---|---|
| Film Thickness Mils | 1.0 | 1.0 | 1.0 | 1.0 | 1.0 | 1.1 |
| Knoop Hardness | - | - | 3.0 | 3.6 | 5.5 | 8.2 |
| Pencil Hardness | 4B | 4B | F-H | HB-F | H-2H | 2H-3H |
| Impact in/lbs, direct | - | - | 80 | 100 | 10 | 10 |
| MEK rubs | 120 | 100 | 200 + | 200 + | 200 + | 200 + |

As indicated by the above Table, the formulations containing urethane/amine resins in a weight ratio of 65/35 (Examples 27 and 28) gave optimum film properties after curing for these specific formulations. All the films had good MEK rub resistance and good flexibility.

EXAMPLE 31

A cationic polymer suitable for cathodic electro- coating was prepared from a bisphenol-A epoxy resin sold under the trademark EPON 1004 by Shell Chemical Co., generally following the directions disclosed at column 7, line 65 to column 8, line 11, of U.S. Patent 3,984,299 as follows. One thousand eight hundred thirty parts (all parts referred to herein are by weight) (2 equivalents) of polyglycidyl ether of bisphenol-A (EPON 1004) having an epoxy equivalent weight of 915 were dissolved in 353.2 parts of methyl butyl ketone by heating to reflux at 130°C with agitation in order to remove any water present by use of a decanting trap in the distillate return line. Upon cooling to 80°C under a dry nitrogen blanket, 52 parts (0.1 equivalent) of a diketimine derived from one mole of diethylenetriamine and 2 moles of methyl isobutyl ketone (1.9 equivalency) and 138.8 parts diethylamine were added and the batch heated to 120°C where it was held approximately 2 hours and then thinned with 326 parts of propylene glycol monomethyl ether. The resultant polytertiary amine cationic resin contains a precursor of primary amine groups attainable from the ketimine moiety upon water addition.

The resultant cationic resin has a solids content of 74.8%, calculated. The t-amine group content is 2 meq per gram of resin solids. Potential primary $NH_2$ is 0.19 meq per gram of resin solids and hydroxy content is 4 meq per gram of resin solids.

EXAMPLE 32

Following a literature procedure reported in Bull. Chim Soc. Fr., 1142(1954), hexamethylene bis-(hydroxypropyl) carbamate was prepared by reacting 2 moles of propylene carbonate with 1 mole of hexamethylene diamine. The hydroxypropyl carbamate has the m.p. 70°C and the following structure:

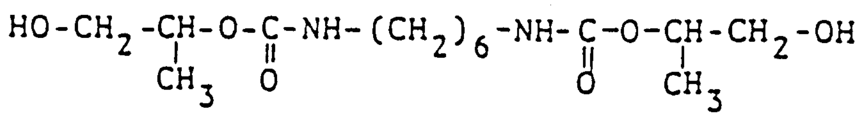

EXAMPLE 33

Twenty-three parts by weight of the cationic resin of Example 31 and 7.5 parts by weight of the hydroxypropyl carbamate of Example 32 were mixed together and warmed to 60°C to make a homogeneous blend. To this mixture, 0.6 parts by weight of dibutyltindilaurate catalyst was added. The formulation was drawn onto an aluminum panel and the resulting films were baked at 175°Cfor 20 minutes. The baked films (approximately 1 mil) were hard and resistant to organic solvents such as methyl ethyl ketone.

EXAMPLE 33A (Comparison)

Example 33 was repeated except that the hydroxypropyl carbamate was omitted so that the baked film was obtained from the cationic resin and catalyst. The resulting film was not solvent-resistant.

EXAMPLE 34

Twenty-six parts by weight of a branched polyester, available commercially as Multron 221-75, from Mobay Chemical Co., has a hydroxy equivalent weight (as is) of 522 and was blended with 8 parts by weight of the hydroxypropyl carbamate of Example 32 and 0.6 parts by weight of dibutyltindilaurate catalyst. The draw down film from the above formulation on aluminum panels was baked at 175°C for 20 minutes. The resulting films were hard and resistant to organic solvents.

EXAMPLE 35

Ten parts by weight (on a solids basis) of the cationic resin of Example 31 were blended with 30 parts by weight of a mixture of isomers of bis(2-hydroxy- 1-methylethyl) (methylenedi-4, 1-1-cylclohexanediyl) biscarbamate, the biscarbamate having been prepared from propylene carbonate and hydrogenated methylene dianiline (4,4'-diaminodicyclohexyl methane). The composition was catalyzed with 2% by weight of a dibutyltindilaurate catalyst and neutralized to 70% with lactic acid. The blend was emulsified in water to 10% solids and electro-deposited on aluminum panels. After a cure time of 20 minutes at 175°C, the film was smooth, demonstrated a pencil hardness greater than 2H and had a reverse impact resistance of greater than 60 in-lbs, and methyl ethyl ketone solvent resistance was greater than 200 MEK rubs.

EXAMPLE 36

A commercially available difunctional terephthalic acid containing powder polyester resin with an average molecular weight of 2800 and a glass transition temperature of 65°C, was melt blended with 13% of the biscarbamate of Example 32 and pigmented with titanium dioxide. The formulation was catalyzed with 3% by weight of dibutyltindilaurate based on the weight of binder. The solidified melt was ground to a powder of 25 to 100 microns and was used for electrostatic spray application. The resulting spray coated film was baked at 200°C for 20 minutes.

The resultant cured film had a thickness of 1.5 mil, a pencil hardness greater than 2H, a reverse impact on steel substrate greater than 140 in-lb. and 20° gloss on untreated steel was greater than 80%. Salt spray resistance on iron phosphated steel was excellent.

EXAMPLE 37

To a suitable reactor containing 408 grams (4 moles) of propylene carbonate and 300 grams of solvent methanol, 292 grams (2 moles) of triethylenetetramine were slowly added while maintaining the temperature at 15 to 30°C by ice bath cooling. Upon complete addition, the mixture was heated to 80°C for approximately 3 hours after which only a trace band in the infrared could be seen for propylene carbonate.

Solvent methanol was then removed by distillation, the last traces of which were removed at 5mm of pressure with steam bath heating. On standing at room temperature, the product, which comprises triethylenetetramine bishydroxypropyl carbamate, solidified to a low melting paste. The product was found to be 98% nonvolatile and titrated in water (phenol red indicator) as though only one amine group per molecule titrated, showing an equivalent weight of 367 (theoretical molecular weight corrected to 98% solids is 357). Potentiometric titration with $HClO_4$ in acetic acid yielded an equivalent weight of 210 which is closer to theory. The infrared spectrum was completely consistent with structure and no problems were subsequently encountered using a theoretical equivalent weight of 175.

EXAMPLE 38

A. A self-cross-linking cathodic electrocoating composition containing tertiary amine groups was prepared from the following ingredients:

| | Parts by Weight | Equivalents |
|---|---|---|
| Epon 1004* | 1017.0 | 1.0 |
| Methyl isobutyl ketone (MIBK) | 176.0 | - |
| Carbamate-containing Amine of Example 4 | 370.4 | 0.8 |
| Diethylamine | 15.0 | 0.2 |
| Propasol P** | 150.0 | - |

*A trademark of Shell Chemical Co. for its product comprising the reaction product of epichlorohydrin and bisphenol-A
**A trademark of Union Carbide Corp. for its propoxypropanol product.

B. The Epon 1004 and MIBK were charged under nitrogen to a suitable reactor as described in Example 5. The mixture was heated to reflux with stirring in order to remove any water present. After cooling to 80°C the carbamate-containing amine of Example 4 was added and the temperature was allowed to rise to 90°C (mild heat of exotherm). Upon complete addition the mixture was heated and held at 100°C for 2 hours. The diethylamine dissolved in the Propasol P was then added slowly so as not to lose diethylamine by volatilization. After addition, the mixture was heated further for 2 hours at 85°C. To remove residual free amine, 250 parts of Propasol P was added and then this same amount was removed by vacuum distillation at 110 to 125°C. The resultant product comprised 76% by weight resin solids and 0.79 meq amine per gram based on 100% resin solids.

C. An electrodeposition bath was prepared by combining 50 grams of the self-cross-linking cathodic electrocoating resin obtained in part B of this Example with 10 grams of hexyl Cellosolve (ethylene glycol monohexyl ether - a flowing agent), 1.2 grams of 89.9% formic acid, and 1.3 grams of dibutyltindilaurate (a urethane catlalyst). 398 Grams of deionized water was slowly added while rapidly mixing with a Cowels stirrer to produce a bath containing approximately 10% solids. The resultant electrodeposition bath had a pH of 4.6, a conductivity of 1800 micromho $cm^{-1}$, and a rupture voltage of 180 volts.

D. The bath composition obtained in part C of this Example was applied, by electrodeposition, to aluminum panels serving as the cathode at 75V for 20 seconds to deposit a thin resin coating on the panels. The panels were then baked at 175°C for 20 minutes and showed film builds of roughly 0.2 - 0.4 mils. All were slightly rough because of minor gassing during electrodeposition as a consequence of emulsion conductivity. This problem could readily be overcome by refining the preparation technique to remove residual free amine. All panels exhibited 4H pencil hardness and resisted greated than 200 methyl ethyl ketone double rubs.

EXAMPLE 39

A. A self-cross-linking cathodic electrocoating composition containing tertiary amine, ketimine, and having on average, a large proportion of carbamate cross-linking groups, was prepared from the following ingredients:

| | Parts by Weight | Equivalents |
|---|---|---|
| EPON 1004F* | 780.0 | 1.00 |
| MIBK* | 176.0 | - |
| Carbamate-containing Amine of Example 37 | 35.0 | 0.20 |
| Carbamate-containing Amine of Example 5 | 277.9 | 0.72 |
| Butyl Cellosolve** | 201.9 | - |
| Styrene Oxide | 20.9 | 0.17 |

*As in Example 38
**Mono butyl ether of ethylene glycol

B. As in Example 38, water was removed from the EPON 1004F after charging with MIBK under nitrogen. At 85°C the carbamate-containing amine of Example 3 dissolved in one third of the butyl Cellosolve was added and the mixture, was stirred for one hour. At this same temperature, the carbamate-containing amine of Example 2 was added and the mixture was further stirred for 8 hours. The styrene oxide was then dissolved in the remaining butyl Cellosolve and the whole was added at 90°C and stirred an additional 4 hours. The resultant product comprised 69% by weight resin solids and 1.10 meq amine per gram based on 100% resin solids.

C. An electrodeposition bath was prepared by combining 50 grams of the self-cross-linking cathodic electrocoating resin obtained in part B of this Example with 3 grams of hexyl Cellosolve, 5 grams of benzyl hydroxypropyl carbamate, 7 grams of EPON 828, 1.26 grams of 89.9% formic acid, and 1.4 grams of dibutyltindilaurate (urethane catalyst). 475 Grams of deionized water was then added slowly while rapidly mixing with a Cowels stirrer to produce a bath containing approximately 10% solids. The electrodeposition bath had a pH of 4.1, a conductivity of 1050 micromho $cm^{-1}$, and a rupture voltage of 400 volts.

D. The bath composition obtained in part C of this Example was electrodeposited on aluminum panels serving as the cathode at 100 V for 20 seconds to deposit a thin resin coating on the panels. After baking the coated panels at 175°C for 20 minutes, the panels showed film builds of 0.3 mils. The coatings were glossy, smooth, flexible, had 4H pencil hardness, passed 40 in-lb impact tests, and resisted greater than 400 methyl ethyl ketone double rubs.

EXAMPLE 40

A. A self-cross-linking cathodic electrocoating composition containing tertiary amine, ketimine, and alkyl groups was prepared from the following ingredients:

| | Parts by Weight | Equivalents |
|---|---|---|
| EPON 834* | 729.0 | 3.00 |
| MIBK* | 176.0 | - |
| Dodecylamine | 166.5 | 1.80 |
| Carbamate-containing Amine of Example 37 | 35.0 | 0.20 |
| Carbamate-containing Amine of Example 5 | 337.5 | 0.87 |
| Butyl Cellosolve | 256.3 | - |
| Styrene Oxide | 24.1 | 0.20 |

*As in Example 38

B. Water was removed from the EPON 834 by charging with MIBK and refluxing under nitrogen as in Example 38. After cooling to 80°C the dodecylamine dissolved in 50 parts of the butyl Cellosolve was slowly added and the temperature allowed to reach 115°C (heat of exotherm). The carbamate-containing amine of Example 37 dissolved in 50 parts of the butyl Cellosolve was then added and the mixture was allowed to slowly cool and was stirred for one hour. After this time, and at 90°C, the carbamate-containing amine of Example 5 dissolved in 100 parts of butyl Cellosolve was added and the mixture was stirred and maintained at 90°C for 5 hours. Following this, the styrene oxide dissolved within the remainder of the butyl Cellosolve was added and the mixture was stirred and heated at 90°C for an additional 12 hours. The resultant product comprised 70% by weight resin solids and 1.89 meq amine

per gram, based on 100% resin solids.

C. An electrodeposition bath was prepared by combining 50 grams of the self-cross-linking cathodic electrocoating resin obtained in part B of this Example with 5 grams of hexyl Cellosolve, 1.37 grams of 89.9% formic acid, and 1.2 grams of dibutyltindilaurate (urethane catalyst). 320 Grams of deionized water was then added slowly while rapidly mixing with a Cowels stirrer to produce a bath containing approximately 10% solids. The electrodeposition bath had a pH of 4.6, a conductivity of 1050 micromho $cm^{-1}$, and a rupture voltage of 300 volts.

D. Electrodeposition of the composition obtained in part C of this Example on aluminum and bare (untreated) steel panels serving as the cathode at 100V for 20 seconds deposited a thin film of resin and afforded, after curing at 175°C for 20 minutes, film thicknesses of 0.4 mil on aluminum and 0.55 mil on bare steel. All coatings were glossy, smooth, flexible, had 4H pencil hardness, passed 40 in-lb impact tests on aluminum and 150 in-lb impact tests on steel, and resisted greater than 100 methyl ethyl ketone double rubs. After exposure to 200 hours in a salt spray cabinet, the coatings on bare steel showed 6 mm pull from the scribe and almost no rusting (rate 9 by ASTM 0610-68).

EXAMPLE 41

A. A one to one mixture of the self-cross-linking resin of Example 39 and the self-cross-linking resin of Example 40 to form an electrocoating composition incorporating the advantages of both was prepared from the following ingredients:

| | Parts by Weight |
|---|---|
| The Resin of Example 39 | 25.00 |
| The Resin of Example 40 | 25.00 |
| Hexyl Cellosolve | 5.00 |
| Formic Acid (89.9%) | 1.38 |
| Dibutyltindilaurate | 1.10 |
| Deionized Water | 315.00 |

B. The electrodeposition bath was prepared by first combining all of the above ingredients except the water. The water was then slowly added while rapidly mixing with a Cowels stirrer producing a bath containing approximately 10% solids. The electrodeposition bath had a pH of 4.3, a conductivity of 1100 micromho $cm^{-1}$, and a rupture voltage of 340 volts.

C. Electrodeposition of the bath composition of part B of this Example at 100 V for 20 seconds on aluminum, bare steel, and zinc phosphate-treated steel panels serving as the cathode deposited thin coatings which yielded film builds of 0.4 mil on aluminum, 0.55 mil on bare steel, and 0.5 mil on zinc phosphated steel after baking at 175°C for 20 minutes. All coatings were glossy, smooth, flexible, and resisted greater than 400 methyl ethyl ketone double rubs. Coatings on aluminum panels had 4H pencil hardness and passed 40 in-lb impact tests, while coatings on steel substrates had 5H pencil hardness and passed 140 in-lb tests. After exposure to 100 hrs. in a salt spray cabinet, the coatings on bare steel showed 7 mm pull from the scribe and no rusting. After exposure to 1000 hours salt spray, the zinc phosphate steel panels showed 3 mm pull from the scribe and only trace rusting (rated 8 by ASTM 0610-68). The coatings on aluminum also passed the 30 day resistance test (Boeing Material Specification 5-89D) for the aerospace fluid Skydrol (BMS 3-11 Fluid with only one unit drop in pencil hardness. After 60 days exposure to salt spray, the coated aluminum panels showed no change in appearance and no loss of adhesion at the scribe line.

EXAMPLE 42

A. A ketimine group-containing electrocoating backbone resin initially having no carbamate groups (and which is to be subsequently converted to a self-cross-linking cathodic electrocoating composition by reaction with an alkylene carbonate) was prepared from the following ingredients:

| | Parts by Weight | Equivalents |
|---|---|---|
| EPON 1004* | 458.0 | 0.50 |
| MIBK* | 95.0 | - |
| Diketimine | 108.0 | 0.40 |
| Diethylamine | 7.0 | 0.08 |
| Propasol P* | 100.0 | - |

*As in Example 38

B. As in Example 38 water was removed from the EPON 1004 by refluxing with MIBK under nitrogen. The mixture was then cooled to 80°C and the diketimine (derived from one mole of diethylenetriamine and 2 moles of MIBK as described in U.S. Patent 3,523,925) and 80 parts of the Propasol P were added. After about one half hour the diethylamine in the remainder of the Propasol P was added and the mixture was allowed to react at 80°C for 1 hour and at 120°C for 2 hours. The resultant product comprises 74.6% by weight resin solids and 2.24 meq amine per gram, based on 100% resin solids.

C. The resin obtained in part B of this Example was then converted into a self-cross-linking cathodic electrocoating composition as follows. To fifty grams of the resin was added 5 grams of hexyl Cellosolve, 1.2 grams of 89.9% formic acid, 1.2 grams of dibutyltindilaurate, and 6.86 grams of propylene carbonate. With rapid mixing using a Cowels stirrer, 403 grams of deionized water was slowly added, forming an electrocoating bath containing approximately 10% solids. The bath was allowed to stir and age for four days and showed no signs of instability. This bath had a pH of 6.6 a conductivity of 1900 micromho $cm^{-1}$, and a rupture voltage of 120 volts.

D. The composition obtained in part C of this Example was applied by electrodeposition to aluminum panels (as the cathode) at 75V for 20 seconds to deposit a thin coating of resin. After curing at 175°C for 20 minutes the panels showed film builds of roughly 0.2 to 0.4 mils. All coatings were rough due to gassing during electrodeposition. This problem could be overcome by refining the preparation technique to remove residual materials such as free residual low molecular weight amine which results in excessively high emulsion conductivity. All panels exhibited 4H pencil hardness and resisted greater than 100 methyl ethyl ketone double rubs.

A significant advantage of the compositions of the present invention is, as mentioned above, the provision of electrodepositable coatings which are curable at low temperatures, i.e., about 200 to 250°F (about 93 to 121°C). The following examples 43 and 44 are exemplary of finished coatings obtained with such low temperature cures.

EXAMPLE 43

A. A self-cross-linking cathodic electrocoating composition was prepared from the following ingredients:

| | Parts by Weight | Equivalents |
|---|---|---|
| EPON 1001F* | 726.0 | 1.50 |
| MIBK* | 132.0 | - |
| Dodecylamine | 55.5 | 0.60 |
| Carbamate-containing Amine of Example 37 | 26.3 | 0.15 |
| Carbamate-containing Amine of Example 5 | 253.1 | 0.65 |
| Butyl Cellosolve** | 240.2 | - |
| Styrene Oxide | 20.4 | 0.17 |

* As in Example 38
**Mono butyl ether of ethylene glycol

B. The EPON 1001F and MIBK were charged under nitrogen into a suitable reactor and water was removed as in Example 38. At 85°C the dodecylamine and the carbamate- containing amine of Example 37 were added in about 20 minutes time followed by 100 parts of the butyl Cellosolve. This mixture was stirred and heated at 85°C for one hour and then the carbamate-containing amine of Example 5 was added with another 100 parts of butyl Cellosolve. After stirring and heating this mixture at 85°C for 8 hours, the styrene oxide in the remainder of the butyl Cellosolve was added and the whole was heated

and stirred at 95°C for four additional hours. The final electrocoating resin was 69% solids and the meq/g total amine corrected to 100% solids was 1.32.

C. An electrodeposition bath suitable for a low temperature cure was prepared by combining 50 grams of the self-cross-linking cathodic electrocoating resin obtained in part B of this Example with 5 grams of hexyl Cellosolve, 1.52 grams of 89.9% formic acid, and 1.94 grams of 72% methyl tricaprylyl ammonium hydroxide catalyst (prepared from the chloride form by ion-exchange in methanol followed by reduced pressure evaporation of the methanol solvent). 314 Grams of deionized water was then added while rapidly mixing with a Cowels Stirrer to produce a bath containing approximately 10% solids. The methyl tricaprylyl ammonium hydroxide catalyst is converted to the formate form in this composition. The resulting electrodeposition bath had a pH of 3.9, a conductivity of 1000 micromho $cm^{-1}$, and a rupture voltage of 220 volts.

D. The composition of part C of this Example was applied, by electrodeposition, to aluminum panels serving as the cathode at 75 V for 30 seconds to deposit a thin film of resin on the panels. The coated panels were baked at 250°F (121°C) for 20 minutes and showed film builds of 0.4 to 0.5 mil. All coatings were smooth, but slightly textured. The coatings were well cured, exhibited 4H pencil hardness, passing 40 in-lb impact tests, and were not removed after 200 rubs with methyl ethyl ketone (MEK).

## EXAMPLE 44

A. The self-cross-linking cathodic electrocoating resin of part B of Example 43 was used for the following 251°F (120°C) curing electrocoating composition.

B. For the preparation of the electrocoating bath, Example 43 was repeated except that in place of the formic acid there was substituted 2.04 grams of 99.8% acetic acid. In this composition the catlyst is methyl tricaprylyl ammonium acetate arising from the replacement of the hydroxide counter ion with acetate ion. The electrodeposition bath had a pH of 4.2, a conductivity of 450 micromho $cm^{-1}$, and a rupture voltage of 280 volts.

C. Electrodeposition of a thin film of the resin of Part B of this Example was attained on aluminum panels serving as the cathode at 100V for 30 seconds. The coated panels were baked at 250°F (121°C) for 20 minutes to afford film builds of 0.35 - 4.0 mils. The coatings were smooth, but slightly textured, had 4H pencil hardness, passed 40 in-lb impact tests, and resisted 100 MEK rubs, 200 MEK rubs just sufficing to remove the films.

Generally, polymers of the invention which comprise the reaction product of bisphenol-A with amines as described above, comprise 0.5 to 4 meq amine, preferably 0.7 to 2.5 meq amine, per gram of resin solids. When novalac epoxy resins are utilized to form the polymers of the invention, in which case hydrophobic amines are often added as described above, the polymers preferably comprise 0.5 to 5 meq amine, more preferably 0.7 to 3 meq amine, per gram of resin solids. When hydrophobic amines as described above are utilized they may comprise 10 to 80%, preferably, 10 to 40% by weight of the total amines (hydrophobic amines and hydroxyalkyl carbamate-containing amines) charged to the reaction. The amines which contain the hydroxyalkyl carbamate group are preferably hydrophilic amines such as diethylenetriamine bishydroxyethyl carbamate, ad the carbamate of Examples 1 and 3. The bishenol-A-derived polymers are hydrophobic enough to overcome the influence of the hydroxyalkyl carbamate groups, provided that the equivalent weight of epoxy is sufficiently high. Low molecular weight bisphenol-A epoxy resins can be chain-extended with primary or di-secondary amines, taking care to select reactants and condtions so as to avoid gellation.

## EXAMPLE 45

Diethylenetriamine in the amount of 618 grams (6 moles) was added to a suitable reactor. 1836 Grams (18 moles) of propylene carbonate, which amount comprises 6 moles in excess of the stoichiometric amount, was slowly added to the reactor under a nitrogen blanket while the temperature of the reactants was maintained at 15 to 20°Cby ice bath cooling. After complete addition, the mixture was stirred 8 hours at room temperature. The resulting product solution comprised diethylenetriamine bishydroxypropyl carbamate and was 75.2% solids in propylene carbonate (theory 75% solids), had 2.51 meq/g secondary amine (theory 2.45 meq/g at 75.2% solids), and gave characteristic bands inthe infrared for the hydroxypropyl carbamate group.

EXAMPLE 46

Novalac Based Hydroxyalkyl Carbamate Electroating Resin

A. A self-cross-linking cathodic electrocoating composition based upon a novalac-hydroxyalkyl carbamate resin was prepared from the following ingredients:

| | Parts by Weight | Equivalents | Solids |
|---|---|---|---|
| DEN$^R$ 485* | 528.9 | 3.00 | 528.9 |
| Methyl isobutyl ketone (MIBK) | 100.0 | - | - |
| N-methylaniline | 175.4 | 1.64 | 175.4 |
| Carbamate-containing Amine of Example 45 | 484.8 | 1.23 | 364.6 |
| Butyl Cellosolve** | 143.2 | - | - |
| Styrene Oxide | 21.4 | 0.18 | 21.4 |

*Dow Chemical Co. Epoxy Novalac Resin
**Monobutyl ether of ethylene glycol

B. The DEN 485 and MIBK were charged under nitrogen into a suitable reactor having a decanting trap in the distillate return line. The mixture was heated to reflux with stirring in order to remove any water present. After cooling to 100°C, the N-methylaniline was charged in 40 parts of the butyl Cellosolve and the stirred mixture was heated and held at 140 to 145°C for 2 hours. After this period the mixture was cooled to 90°C and the carbamate-containing amine of Example 45 and the remainder of the butyl Cellosolve was added. This mixture was stirred and heated at 90°C for 2.5 hours and then the styrene oxide was added and the temperature held at 90 to 100°C for 2 hours further. The analysis for the final electrocoating resin showed 1.81 meq/g amine. The resin was 75% solids.

C. An electrocoating bath was prepared by combining 300 parts of the novalac-hydroxypropyl carbamate electrocoating resin of part B of this Example with 6.83 parts of 90.8% formic acid and 4.62 parts of dibutyltindilaurate (a urethane catalyst). 2046.7 parts of deionized water was slowly added while rapidly mixing with a Cowels stirrer to produce a bath containing approximately 10% solids. The electrocoating bath had a pH of 4.4, a conductivity of 600 micromho $cm^{-1}$, and a rupture voltage of 210 volts.

EXAMPLE 47

The electrodeposition bath of Example 46 was applied to a substrate comprising a 2024T3 Phosanodized bare aluminum member at a deposition voltage of 75 V applied for 20 seconds. The resulting deposited coating was cured by heating for 5 minutes to 347°F (175°C) then maintaining 175°C for an additional 20 minutes. Film builds of 0.35 to 0.4 mils were attained. The resultant coatings were glossy, showed a slight orange peel texture, exhibited 7H pencil hardness, passed 40+ in-lb impact tests on 0.02 inch thick substrate and were not removed after 300+ rubs with methyl ethyl ketone.

Generally, utilization of a suitable ternary or quaternary ammonium catalyst provides an electrodeposited coating which can be cured at low temperature, e.g., 250°F(121°C). Temperatures as low as 200°F (93°C) can successfully be utilized when an appropriate amount of such catalyst is employed. The amount of such catalyst employed to effectuate a low temperature cure is generally about 0.1 to 10%, preferably 1 to 5% by weight of the weight of resin solids.

EXAMPLE 48

Carbamate Cross-Linking Agent I

To a suitably equipped flask under a nitrogen atmosphere was charged 436.8 grams (2.1 moles) of 4, 4'-diaminodicyclohexyl methane (mixture of isomers), 435.4 grams (4.3 moles) of propylene carbonate, and 262 grams of tert-butyl alcohol. The mixture was bought to reflux and the progress of the reaction was followed by titrating the remaining amine with 0.1N HCl using phenol red indicator. After 3 days at reflux 95% conversion was reached and the mixture was cooled to 50°C and 500 ml of acetone was added. After cooling to room temperature the mixture converted to a slurry of white crystals. The mixture was then warmed to 60°C and filtered. To the filtrate was added small portions of ethylacetate-heptane (2 to 1 by

volume) until no more solid separated. Refiltration, combining the collected solid with that obtained previously, and drying at 40°C overnight afforded 125 grams (14.5%) of high melting (192 to 198°C) isomers of bis(2-hydroxy-1- methylethyl) (methylenedi-4, 1-cyclohexanediyl)biscarbamate, herein referred to as "Carbamate Cross-Linking Agent I" which gave satisfactory spectral analyses but were not deemed suitable for use in cathodic electrocoating compositions due to their high melting range. Accordingly, to the clear filtrate was added enough cationic exchange resin (Dowex 50W-X8 manufactured by Dow Chemical Co.) to remove all residual free amine as determined by checking with phenol red indicator from time to time. After all free amine had been removed, the ion exchange resin was filtered off and the solvents were vacuum distilled with steam bath heating, the last traces of which were removed at 5 mm of pressure. The resulting clear, light yellow, low melting semi-solid gave satisfactory spectral analyses for a mixture of isomers of Carbamate Cross-Linking Agent I. The yield of Carbamate Cross-Linking Agent I was 683.1 grams (80% of theory; the total yield of all dicarbamates was 93.7% of theory).

EXAMPLE 49

Carbamate Cross-Linking Agent II

To a suitable reactor under a nitrogen atmosphere was charged 95.36 grams (0.4 moles) of 2,2'-Bis(4-amino cyclohexyl) propane, 83.72 grams (0.82 moles) of propylene carbonate, and 53.72 grams of tert-butyl alcohol and the whole was brought to reflux. The progress of the reaction was followed by titrating the remaining amine with 0.1N HCl using phenol red indicator. 95% Conversion was obtained after three days of refluxing after which the viscous reaction mixture was cooled to 50°C and 54 grams of methanol was added. Enough of the same cationic exchange resin as used in Example 48 was added to remove free amine and the solution was filtered. Evaporation of all solvents under vacuum using steam bath heating afforded 163 grams (91% of theory) of a mixture of isomers of bis(2-hydroxy-1-methylethyl), [(1-methylethylidene)di-4,1-cyclohexanediyl]biscarbamate, herein referred to as "Carbamate Cross-Linking Agent II". The structure was confirmed by spectral analyses. Carbamate Cross-Linking Agent II softens by about 60°C and begins to melt at about 80°C.

Hexane-1,6-bis(hydroxypropyl)carbamate as a starting material for Cathodic Electrocoating Compositions

Hexane-1,6-bis(hydroxypropyl) carbamate is completely water soluble and therefore unsuitable as a carbamate cross-linking agent for cathodic electrocoating. Hexane-1,6-bis(hydroxypropyl) carbamate was prepared as described in the literature (Najer, H., Chabrier, P., and Guidicelli, R., Compt. rend. 238 690 (1954) ) by the reaction of hexamethylene diamine, and propylene carbonate and subsequently fully purified. This bishydroxypropyl carbamate was then chemically modified by "capping" or "blocking" the hydroxyalkyl carbamate groups in order to produce water insoluble carbamate cross-linking agents for cathodic electrocoating as described below.

EXAMPLE 50

Carbamate Cross-Linking Agent III

To a suitable reactor under a nitrogen atmosphere was charged 50.0 grams of dry pyridine and 8.72 grams (0.04 moles) of hexane-1,6-bis-(hydroxypropyl) carbamate prepared as described above. The well stirred solution was then cooled to a 3°C and acetic anhydride (8.98 grams; 0.88 moles) was slowly added keeping the temperature below 10°C while efficiently stirring. After complete addition the mixture was stirred overnight at room temperature. At this point thin layer chromatography (TLC-Analtech precoated Silica Gel GF 250 micron plates 2.5 x 10 cm; eluent 10/90 V/V MeOH/$CHCl_3$ followed by oven drying at 105°C for 30 minutes to remove pyridine and pyridine acetate; visualization with iodine) showed only one product spot at Rf 0.73 (pure hexane-1,6-bis (hydroxypropyl) carbamate has Rf 0.53 under the same conditions. Most of the solvent pyridine was then removed at about 10 mm of pressure with a flask temperature of 40 to 80°C. The reaction residue containing product was then dissolved in $CHCl_3$ (100 ml) and washed with three 25 ml portions of 25% w/w $NH_4Cl$ solution, then with two 25 ml portions of water, followed by a 25 ml portion of brine. The $CHCl_3$ solution of product was then dried ($Na_2SO_4$), filtered, and evaporated under vacuum to afford 10.77 grams (99%) of a light yellow liquid which solidified to a low melting solid on standing. This product was insoluble in water, homogeneous by TLC and gave IR and PMR spectra consistent with the diacetate of hexane-1,6-bis (hydroxypropyl) carbamate, hereinafter referred to as "Carbamate Cross-linking

Agent III".

EXAMPLE 51

Carbamate Cross-linking Agent IV

In a suitably equipped flask was charged trimethylol propane (13.4 grams) hexane-1,6-bis-(hydroxypropyl) carbamate prepared as described above (96 grams), and dibutyltindilaurate catalyst (0.6 grams). The reaction mixture was stirred and heated under reduced pressure (approximately 20 mm of Hg) to 175 to 190°C for a period of about one hour. During this period about 10 grams of distillate was collected which by GPC proved to mostly propylene gylcol. The clear resinous product in the flask was soluble in alcohol, Cellosolve, and insoluble in water. The resulting condensation product of hexane-1,6-bis-(hydroxypropyl) carbamate with trimethylol propane is herein referred to as "Carbamate Cross-Linking Agent IV". The product was diluted with 20 grams of Cellosolve to 77% solids.

EXAMPLE 52

Cationic Polymeric Material V

Cationic polymeric material V is prepared by reacting n-butyl acrylate, styrene, N,N-dimethyl amino ethyl methacrylate, 2-hydroxyethyl acrylate, the reaction product of acrylic acid and a methoxypolyethyleneglycol having a molecular weight of 550, N-dodecyl mercaptan, and azobisisobutyronitrile in the amounts and according to the procedure described in U.S. Patent 4,026,855 for the preparation of the material therein described as "Polymeric Material E". The disclosure of U.S. Patent 4,026,855 is incorporated by reference herein. The final resin is about 71% solids and has an hydroxy number of about 90 and an amine number of about 45.

EXAMPLE 53

Cationic Polymeric Material VI

Cationic polymeric material VI, a cationic epoxy resin, was prepared by reacting EPON 1004 (a product of Shell Chemical Co. comprising the reaction product of bisphenol-A and epichlorohydrin) with the diketimine of the diethylenetriamine (prepared as described in U.S. Patent 3,523,925) and diethylamine according to the procedure described in U.S. Patent 3,984,299 for the preparation of the material therein described as adduct C. The disclosures of U.S. Patents 3,523,925 and 3,984,299 are incorporated by reference herein. The final cationic resin was 75% solids. The analysis of the resin corrected to 100% solids showed the following: 5-amine: 1 meq per gram, primary amine (after hydrolysis): 0.2 meq per gram, and calculated hydroxy content 3.7 to 4 meq per gram.

EXAMPLE 54

Cationic Polymeric Material VII

Cationic Polymeric Material VII, an epoxy material, was prepared from the ingredients and processes described below:

| | Parts by Weight | Equivalents |
|---|---|---|
| EPON 1004* | 1017.0 | 1.0 |
| Methyl isobutyl ketone (MIBK) | 176.0 | -- |
| Diketimine | 69.4 | 0.26 |
| Diethylamine | 57.5 | 0.79 |
| Propasol P** | 163.0 | -- |

*Shell Chemical Co. reaction product of epichlorohydrin and bisphenol-A
**Union Carbide Corp. propoxypropanol

The EPON 1004 and MIBK were charged to a suitable reactor under nitrogen having a decanting trap in the distillate return line. The mixture was heated to reflux with stirring in order to remove any water present. After cooling to 80°C the Propasol P was added followed by the diketimine (derived from one mole of diethylenetriamine and 2 moles of MIBK as described in U.S. Patent 3,523,925). The mixture was held at 80°C for one hour and then cooled to 60°C and the diethylamine was slowly added keeping the temperature below 65°C (exotherm) so that no diethylamine was lost by volatilization. After complete addition the mixture was refluxed for one hour and then 100 parts more of Propasol P was added. The same amount of Propasol P was then removed by two vacuum distillations at 100 to 125°C in order to remove any residual free diethylamine. The final cationic resin was 78% solids. The analysis of the resin corrected to 100% solids showed the following: t-amine, 0.84 meq per gram; primary amine (after hydrolysis): 0.45 meq per gram and calculated hydroxy content: 3.6 to 3.8 meq per gram.

EXAMPLE 55

A cathodic electrocoating bath is prepared by combining 50 parts of the Cationic Polymeric Material V, 15 parts of the Carbamate Cross-Linking Agent 1, 1.7 parts of 88% lactic acid, and 1.5 parts of dibutyltindilaurate in a suitable mixing vessel equipped with a Cowels stirrer. These ingredients are rapidly mixed while 466 parts of the deionized water is slowly added to produce a bath containing approximately 10% solids. The final cathodic electrocoating bath has a pH of 4.8. After aging the bath overnight, electrodeposition of the composition on aluminum panels serving as the cathode at 100V for 60 seconds afforded, after rinsing and curing at 175°C for 20 minutes, cured films which are glossy, smooth, and have very good solvent resistance and mechanical properties.

EXAMPLE 56

The procedure of Example 55 was repeated in all essential respects using the following ingredients:

| | Parts by Weight |
|---|---|
| Cationic Polymeric Material VII | 49.0 |
| Carbamate Cross-Linking Agent I | 14.4 |
| Hexyl Cellosolve* | 5.0 |
| Butyl Cellosolve** | 5.0 |
| Lactic Acid (88%) | 2.8 |
| Dibutyltindilaurate | 1.2 |
| Deionized Water | 485.0 |

*Monohexyl ether of ethylene glycol - a flowing agent
**Monobutyl ether of ethylene glycol - a co-solvent

The final cathodic electrocoating bath was 10% solids and had a pH of 5.8 and a conductivity of 700 micro mho $cm^{-1}$. After aging the bath overnight, electrodeposition of the composition on aluminum panels serving as a cathode at 75V for 20 seconds produced, after rinsing and thencuring at 175°C for 20 minutes, films with thicknesses of 0.2 to 0.25 mil. All panels were smooth, glossy, had 4H pencil hardness, passed 40 in-lb impact tests, and resisted greater than 200 methyl ethyl ketone double rubs.

EXAMPLE 57

The procedure of Example 55 and the ingredients of Example 56 were repeated, except that 15.3 parts of the Carbamate Cross-linking Agent II was substituted in place of the 14.4 parts of Carbamate Cross-Linking Agent I. The resulting cathodic electrocoating bath had a pH of 5.8, a conductivity of 700 micro mho $cm^{-1}$, and was approximately 10% solids. Aging the bath overnight followed by electrodeposition on aluminum panels serving as the cathode at 75V for 20 seconds afforded, after rinsing and curing at 175°C for 20 minutes, film builds of 0.2 to 0.5 mil. The coatings on all panels were smooth, glossy, had 4H pencil hardness, passed 40 in-lb impact tests and resisted greater than 200 methyl ethyl ketone double rubs. The electrocoating bath was stable for more than three months in storage at ambient temperatures.

EXAMPLE 58

The procedure of Example 55 and the ingredients of Example 56 were repeated except that 9.4 parts of Carbamate Cross-linking Agent III was substituted for Carbamate Cross-Linking Agent I and 440 parts of deionized water was used. The resulting cathodic electrocoating bath was 10% solids, had a pH of 5.5, and a conductivity of 780 micro mho $cm^{-1}$. The bath was then aged overnight and aluminum panels serving as the cathode were electrocoated at 75V for 20 seconds. The electrocoated panels were then rinsed and cured at 175°C for 20 minutes. The cured films were 0.2 to 0.25 mil thick, had 4H pencil hardness, passed 40 in-lb impact tests, were smooth and glossy, and resisted greater than 200 methyl ethyl ketone double rubs. The bath was still stable after three months aging at ambient temperature.

EXAMPLE 59

Using the mixing procedure of Example 55, 47 parts of Cationic Polymeric Material VI, 19.5 parts of Carbamate Cross-Linking Agent IV, 3 parts of hexyl Cellosolve, 1.2 parts of dibutyltindilaurate, and 3.6 parts of 88% lactic acid were emulsified to 10% solids with 470 parts of deionized water. A milky white emulsion was obtained having a pH of 5.8 and a conductivity of 1225 micro mho $cm^{-1}$. After aging the cathodic electrocoating bath overnight, aluminum panels serving as the cathode were electrocoated at 50V and 100V for 30 seconds. The electrocoated panels were rinsed with water and then cured at 175°C for 20 minutes. After the cure, the electrocoated films were solvent resistant, had thickness of 0.6 to 0.7 mil, passed 40 in-lb impact tests, and had pencil hardness of 4H to 5H.

EXAMPLE 60

To a suitable reactor containing 103 grams (1 mole) of diethylenetriamine and 300 grams of solvent methanol under a nitrogen atmosphere 184.8 (2.1 moles) of ethylene carbonate was slowly added. The temperature was maintained at 15 to 20°C by ice bath cooling. After complete addition, the mixture was stirred at room temperature overnight. Methanol was then removed by use of a water pump vacuum and with steam bath heating. The resulting product solidified to a mass of white crystals upon cooling, mp 82 to 88°C. Recrystallization from ethanol afforded a pure product, mp 96 to 97°C, in nearly quantitative yield. The product gave completely consistant IR and NMR spectra for the bis-hydroxyethyl carbamate of diethylenetriamine, i.e., diethylenetriamine bis-hydroxyethyl carbamate.

EXAMPLE 61

To a suitable reactor containing 408 grams (4 moles) of propylene carbonate and 300 grams of solvent methanol, 292 grams (2 moles) of triethylenetetramine was slowly added while maintaining the temperature at 15 to 30°C by ice bath cooling. Upon complete addition, the mixture was heated to 80°C for approximately 3 hours after which only a trace band in the infrared could be seen for propylene carbonate. Solvent methanol was then removed by distillation, the last traces being removed at 5mm of pressure with steam bath heating. On standing at room temperature, the product, which comprises triethylene-tetramine bishydroxypropyl carbamate, solidified to a low melting paste. The product was found to be 98% nonvolatile.

EXAMPLE 62

Example 13 was repeated, except that 29 parts of the catalyst (equivalent to 5% by weight solid catalyst on a solid catalyst to resin solids basis) was added. Resulting films cured at 220°F (104.4°C) for 20 minutes showed properties similar to those of the cured films of Example 13. Resulting films cured at 212°F (100°C) resisted over 200 water rubs but only 100 MEK rubs. Based on the foregoing, it appears that a somewhat longer cure time at temperatures as low as 200°F (93.3°C) would provide satisfactory film properties with properly catalyzed polymers of the invention.

EXAMPLE 63

A. A self-cross-linking novalac hydroxyethyl carbamate- containing resin which can be reduced with water to 10% solids with only 10% by total weight cosolvent was prepared by replacing the EPON 828 in Example 14 with 79.7 parts of EPN 1139 and using 52.4 parts of butyl Cellosolve. After following the

preparation procedure of Example 4, the resulting product was 80% solids.

B. A sprayable composition was prepared by dissolving 100 parts of the novalac hydroxyethyl carbamate-containing resin obtained in part A of this Example in 100 parts of deionized water and 15.4 parts of 1 Molar tetrabutyl ammonium hydroxide catalyst. The resulting clear solution contained only 9% organic cosolvent and was 39% solids. The composition was applied, by spraying, onto aluminum panels which were cured at 250°F for 20 minutes. The resulting smooth, glossy coatings had 4H pencil hardness, were 0.3-0.4 mil thick, passed 40 in-lb reverse impact, and resisted greater than 300 MEK and water double rubs.

EXAMPLE 64

A. A more polymeric self-cross-linking BP-A hydroxypropyl carbamate-containing resin, water reducible to at least 35% solids with 25% cosolvent, was prepared from the following ingredients:

| | Parts by Weight | Equivalents | Solids |
|---|---|---|---|
| EPON 828 | 110.4 | 0.6 | 110.4 |
| Carbamate-containing Amine of Example 61 | 35.7 | 0.2 | 35.0 |
| Carbamate-containing Amine of Example 4 | 185.2 | 0.4 | 135.2 |
| Butyl Cellosolve | 19.5 | - | - |

The EPON 828, the carbamate-containing amines of Example 4 and Example 61 and the butyl Cellosolve were mixed and reacted in the same manner as described in Example 61. The final very viscous product was 80% solids.

B. A sprayable composition was prepared by mixing 100 parts of the polymeric BP-A hydroxypropyl carbamate product of part A with 100 parts of 140°F deionized water until a milky suspension was obatined. The stirred suspension was cooled to room temperature and 30 parts of butyl Cellosolve followed by 9.3 parts of tetrabutyl ammonium hydroxide catalyst was added. The resulting clear solution was 34% solids and contained 25% organic cosolvents. This composition was applied, by spraying, onto aluminum panels which were then cured at 250°F for 20 minutes. The cured coatings were 0.4 to 0.5 mil thick, were smooth and glossy, had 4H pencil hardness, passed 40 in-lb reverse impact, and resisted greater than 300 MEK and water double rubs.

EXAMPLE 65

Example 13 was repeated in all essential details, except that in place of the tetrabutyl ammonium hydroxide an equivalent amount of benzyltrimethyl ammonium hydroxide was substituted as catalyst. The cured coatings were similar inall film properties tested.

EXAMPLE 66

Example 13 was repeated in all essential details except that in place of the tetrabutyl ammonium hydroxide an equivalent amount of tetramethyl ammonium acetate was substitited as catalyst. Some precipitate formed and solution was re-effected by adjusting the solids to 30% with elthylene glycol monobutyl ether. After spraying and curing panels at 250°F, the coatings had similar film properties as those obtained in Example 4.

EXAMPLE 67

Example 13 was repeated in all essential details except that in place of the tetrabutyl ammonium hydroxide catalyst an equivalent amount of dibutyltindilaurate (a common commercially utilized urethane catalyst) was substituted as catalyst. The dibutyltindilaurate catalyst was not fully compatible with the water based system but for the short term formed a relatively stable suspension. Cure was not effected at 250°F after 20 minutes and in fact 330 to 350°F for 20 minutes was necessary. Furthermore, the coatings cured ar 350°F were poor in appearance and very brittle.

The polymers of the invention, while having the great advantage from the point of view of environmental and safety considerations of being water soluble/reducible, can also be employed with organic solvents, as shown in Example 13.

EXAMPLE 68

All essential details of Examples 63 were repeated, except that butyl Cellosolve was substituted in place of all the water solvent and the aqueous tetrabutyl ammonium hydroxide catalyst was replaced by an equivalent amount of methanolic benzyltrimethyl ammonium hydroxide. When baked at 250°F for 20 minutes the film from this nonaqueous composition was similar in properties to those obtained from aqueous systems.

EXAMPLE 69

A. An isocyanurate hydroxypropyl carbamate-containing resin was prepared from the following ingredients:

| | Parts by Weight | Equivalents | Solids |
|---|---|---|---|
| ARALDITE PT 810* | 125.4 | 1.0 | 125.4 |
| Carbamate-containing Amine of Example 4 | 463.1 | 1.0 | 338.1 |

*Ciba Geigy Co. reaction product of isocyanuric acid with epichlorohydrin

B. The ARALDITE PT 810 and the carbamate-containing amine of Example 4 were added to a suitable reactor under nitrogen equipped with a Cowels high speed stirrer. Upon heating to 80°C the suspension of reactants began to exotherm mildly and the temperature reached 113°C. After the exotherm subsided, the nearly homogenous solution was heated further at 115°C for 4 hours. The final product had a solids content of 79%.

C. A sprayable aqueous composition was prepared by dissolving 50.0 parts of the isocyanurate hydroxypropyl carbamate-containing resin of part B of this Example in 207.3 parts of deionized water, followed by adding 6.0 parts of ethylene glycol monobutyl ether and 8.5 parts of diethylene glycol monobutyl ether flow-promoting solvents. This solution which contains a total of only 9.2% organic solvents by weight, was filtered and 4.9 parts of 40% aqueous tetrabutyl ammonium hydroxide catalyst was added. The resulting clear solution was 14.8% solids. This composition was applied by spraying onto aluminum panels. The coatings were cured at 250°F for 60 minutes and afforded film thicknesses of 0.15 to 0.20 mil. The coatings were smooth, glossy, had 4H pencil hardness, passed 40 in-lb impact tests, and resisted greater than 300 MEK rubs and 200 water rubs.

EXAMPLE 70

To 168 grams (1.65 moles) of propylene carbonate was added 51 grams (0.5 mole) of diethylenetriamine in 150 ml. of methanol at 15°C. After complete addition the mixture was allowed to react for 24 hours to 25°C. Potentiometric titration indicated the presence of free secondary amine. The reaction mixture was heated to 70°Cfor 3 hours. After this period there was very little change in the free amine content as indicated by titration. Methanol was removed from the mixture to obtain a syrupy liquid at about 72% solids containing bis(2-hydroxy-1-methylethyl) (imminodiethylene)biscarbamate. The analysis of the product by FAB mass spectroscopy also confirmed the composition to consist mainly of the above compound.

EXAMPLE 71

To a neck flask suitably equipped with stirrer and a thermometer was added 62.4 grams (0.4 mole) of 4-amino-2,2,6,6-tetramethylpiperidine (ATP) and 150 grams methanol. To this solution was added 52.8 grams (0.6 mole) of ethylene carbonate and the mixture was heated to 70°C for two hours. The separated white solids were filtered at 25°C and washed with methanol to yield 63.5 grams (65% of theory) of a product of mp 190 to 192°C. I.R., N.M.R. and potentiometric titration confirmed the structure to be 2-hydroxyethyl (2,2,6,6-tetramethyl-4-piperidinyl) carbamate.

EXAMPLE 72

To a 3-neck flask suitably equipped with stirrer and a thermometer was added 62.4 grams (0.4 mole) of ATP (see Example 71 and 25 grams of methanol, followed by 61.2 grams (0.6 mole) of propylene carbonate. The reaction mixture was heated and allowed to reflux for five hours. On standing overnight at 25°C, white crystalline solids had separated. The crystalline solids were filtered and washed with a small amount of methanol to yeild 82 grams (79.4% theoretical) of a product of m.p. 135°C. The N.M.R. analysis indicated the product to be a mixture of isomeric hydroxypropyl carbamates containing primary and secondary hydroxy groups in 2:1 mole ratios.

EXAMPLE 73

The procedure of Example 16 was repeated except that instead of the carbamate of Example 4, 30.2 grams of the hydroxypropyl carbamate of ATP of Example 77 was reacted with 93.75 grams of the acrylic resin of Part A of Example 16 (on solids basis). The reaction was carried out in the presence of 53 grams of 2-ethoxyethanol at 145°C for 18 hours. The resultant acrylic polymer had the following characteristics. Solids: 70%, Basic Nitrogen Content: 0.69 meq per gram, and Epoxy Content: 0.1 meq per gram.

EXAMPLE 74 (COMPARATIVE EXAMPLE)

In a 3-neck flask suitably equipped with stirrer, a thermometer, and a condenser 33 grams of 2-ethoxyethanol was heated to 135°C. To this was added slowly over a two hour period a blend of 60 grams of n-butyl acrylate, 20 grams styrene, 20 grams of 2-hydroxyethylmethacrylate of very low acid content, 1 gram of dicumyl peroxide, and 1 gram of n-dodecyl mercaptan. After complete addition of the monomer blend containing the initiator and the chain transfer agent, the reaction temperature was held at 140°C for two hours. Throughout the polymerization reaction a nitrogen blanket was maintained in the reactor. The resultant resin had the following characteristics: Solids: 75%, Acid number: 0.4, and Hydroxy number: 85 (on solids basis).

EXAMPLE 75

Cast films obtained from the polymer of Example 73 catalyzed with 1% tetrabutyldiacetoxy stannoxane, were cured at 150°C for 20 minutes. The cured films had good MEK rub resistance, however, the films were soft. The polymer of Example 73 can potentially be also useful as a hindered amine light stabilizer for thermoset and thermoplastic materials.

EXAMPLE 76

74.4 grams of the polymer of Example 73, 1.5 grams of benzyltrimethylammcnium hydroxide (40% in methanol), and 30 grams of ORR 650 rutile titanium dioxide pigment were dispersed in a sand mill. The filtered white enamel was cast on zinc phosphate pretreated cold rolled steel panels by a wirecater. The coated panels were cured at elevated temperatures to yield hard, flexible and organic solvent resistant, white glossy coatings. Cure schedule and film properties are shown below.

| | I | II |
|---|---|---|
| Cure Schedule, 20 min. | 122 °C | 150 °C |
| Film Thickness (micro-meters) | 37 | 30 |
| Knoop Hardness | 3.1 | 9.3 |
| Pencil Hardness | F-H | H-2H |
| Impact (Reverse) | 60 in-lbs | 60 in-lbs |
| MEK Rubs | 100+ | 100+ |

EXAMPLE 77 (COMPARATIVE EXAMPLE)

A. Three formulations were prepared using, respectively, the polymers of Examples 16 and 74 in combination with 2-ethyl hexanol blocked toluene diisocyanate (TDI-EHA) prepared according to U.S.

Patent 3,984,299, and benzyl trimethyl ammonium hydroxide (BTMA) as the catalyst.

| Formulation | I | II | III |
|---|---|---|---|
| Polymer of Example 16 | 12.3 | - | - |
| Polymer of Example 74 | - | 23.3 | 20.0 |
| TDI-EHA | 3.2 | 7.9 | 10.2 |
| BTMA (40%) | 0.25 | 0.6 | 0.6 |

The films were cast on zinc phosphate pretreated cold rolled steel and cured at 125°C for 20 minutes. Films obtained from formulation I had good MEK rub resistance, indicating that film was cross-linked. Formulations II and III had no MEK rub resistance, indicating that films were not cross-linked. This shows that hydroxyethyl carbamate behaves chemically differently from hydroxyethyl esters.

B. Three formulations as shown below were prepared using the polymer of Example 74 in combination with BTMA, dibutyltindilaurate (DBTL) and tetrabutyldiacetoxy stannoxane (TDS) as catalysts. The films were cast on zinc phosphate pretreated cold rolled steel and baked at 150°C for 20 minutes. None of the films had good MEK rub resistance after the bake, indicating poor cross-linking. The polymer of Example 16 under similar conditions gave well cross- linked films.

| Formulation | I | II | III |
|---|---|---|---|
| Polymer of Example 74 | 10 | 10 | 10 |
| BTMA (40%) | 0.2 | - | - |
| DBTL | - | 0.2 | - |
| TDS | - | - | 0.2 |

## EXAMPLE 78

A. To a reactor suitably equipped and under a nitrogen blanket was added 131 grams of anhydrous toluene. To this a blend of 59 grams of n-butyalacrylate, 45 grams of methylmethacrylate and 30.1 grams of 1-(1-isocyanato - 1- methylethyl) -3- (1-methylethenyl) benzene was added at 105°C followed by 2.1 grams of t-butylperbenzoate initiator. The reaction temperature was maintained at 105°C for 3 hours. An additional 2 grams of the initiator was added and the reaction temperature was raised to 116°Cand this temperature matintained for 5 hours. The resulting polymer had 50% solids and an NCO content of 2.5% by weight.

B. In a suitable reactor under a nitrogen blanket was added 44.5 grams of isocyanate group containing polymer of part A of this Example, followed by addition of 10 grams of an 80% solution of bis(2-hydroxy -1-methylethyl) (imminodiethylene) biscarbamate in isobutanol, and 10 grams of isobutanol at ambient temperature. There was a slight exotherm initially; the reaction mixture was held at 30°C for 2 hours. After this period I.R. of the reaction mixture showed absence of the isocyanate band and presence of the band for substituted urea. Titration of the resulting product showed no free amine.

## Coating Formulations

The polymer of Example 78 was formulated into a clear coating formulation using dibutyltindilaurate (DBTL), tetrabutyldiacetoxy stannoxane, (TDS), and benzyltri- methylammonium hydroxide (BTMA), catalysts as shown below.

| Formulation | I | II | III |
|---|---|---|---|
| Polymer of Example 78 | 10 | 10 | 10 |
| DBTL | 0.1 | - | - |
| TDS | - | 0.1 | - |
| BTMA (40%) | - | - | 0.2 |

The cure schedule and film properties of the above formulations are shown below.

| Formulation | I | II | III |
|---|---|---|---|
| Cure Schedule: | 150°C (20 min.) | 150°C (20 min.) | 125°C (20 min.) |
| Film Thickness (micro-meters): | 18 | 15 | 22 |
| Pencil Hardness: | 3H-4H | 3H-4H | H-2H |
| Impact(reverse) in/lbs: | 20-30 | 20-30 | 5-10 |
| MEK Rub Resistance: | 100+ | 100+ | 100+ |

EXAMPLE 79

A. A self-cross-linking cathodic electrocoating composition containing tertiary amine, ketimine, and having hydroxyalkyl carbamate cross-linking groups, was prepared from the following ingredients:

| | Parts by Weight | Equivalents |
|---|---|---|
| EPON 1004F* | 780.0 | 1.00 |
| MIBK** | 176.0 | - |
| Carbamate-containing Amine of Example 37 | 35.0 | 0.20 |
| Carbamate-containing Amine of Example 5 | 277.9 | 0.72 |
| Butyl Cellosolve*** | 201.9 | - |
| Styrene Oxide | 20.9 | 0.17 |

*Shell Chemical Co. reaction product of epichlorohydrin and BP-A
**Methyl Isobutyl Ketone
***Mono butyl ether of ethylene glycol

B. Water was removed at reflux from the EPON 1004F after charging with MIBK under nitrogen to a suitable reactor having a decanting trap in the distillate return line. At 85°C the carbamate-containing amine of Example 37 dissolved in one third of the butyl Cellosolve was added and the mixture was stirred for one hour. At this same temperature, the carbamate-containing amine of Example 5 was added and the mixture was further stirred for 8 hours. The styrene oxide was then dissolved in the remaining butyl Cellosolve and the whole was added at 90°Cand stirred an additional 4 hours. The resultant product comprised 69% by weight resin solids and 1.10 meq amine per gram based on 100% resin solids.

EXAMPLE 80

A. A one to one mixture of the self-cross-linking resin of Example 79 and of the self-cross-linking resin of Example 40 to form an electrocoating composition incorporating the advantageous of both was prepared from the following ingredients:

| | Parts by Weight |
|---|---|
| The Resin of Example 79 | 25.00 |
| The Resin of Example 40 | 25.00 |
| Hexyl Cellosolve* | 5.00 |
| Formic Acid (89.9%) | 1.38 |
| Dibutyltindilaurate (urethane catalyst) | 1.10 |
| Deionized Water | 315.00 |

*Mono hexyl ether of ethylene glycol

B. The electrodeposition bath was prepared by first combining all of the above ingredients except the water. The water was then slowly added while rapidly mixing with a Cowels stirrer producing a bath containing approximately 10% solids. The electrodeposition bath had a pH of 4.3, a conductivity of 1100 micromho $cm^{-1}$, and a rupture voltage of 340 volts.

C. Electrodeposition of the bath composition of part B of this Example at 100 V for 20 seconds on aluminum, bare steel, and zinc phosphate-treated steel panels serving as the cathode deposited thin

coatings which yielded film builds of 0.4 mil on aluminum, 0.55 mil on bare steel, and 0.5 mil on zinc phosphated steel after baking at 175°C for 20 minutes. All coatings were glossy, smooth, flexible, and resisted greater than 400 methyl ethyl ketone double rubs. Coatings on aluminum panels had 4H pencil hardness and passed 40 in-lb impact tests, while coatings on steel substrates had 5H pencil hardness and passed 140 in-lb impact tests. After exposure to 100 hours in a salt spray cabinet, the coatings on bare steel showed 7mm pull from the scribe and no rusting. After exposure to 1,000 hours salt spray, the zinc phosphate steel panels showed 3mm pull from the scribe and only trace rusting (rated 8 by ASTM 0610-68).

## EXAMPLE 81

The electrodeposition bath of Example 80 was applied to a substrate comprising a 2024T3 Phosanodized bare aluminum member at a deposition voltage of 100 V applied for 20 seconds. The resulting deposited coating was cured by heating at a temperature of 347°F(175°C), for 20 minutes. The resultant cured film had the following properties:

| | |
|---|---|
| Film Thickness, mil: | 0.35-0.4 |
| Appearance: | Smooth, glossy |
| Pencil Hardness: | 4H |
| MEK rubs: | 300 + |
| Reverse Impact, in-lbs: | 40 + on 0.02 inch thick substrate |
| Skydrol Resistance: | Pass; 30 days immersion one unit drop in pencil hardness |
| Salt Spray Resistance: | Pass; 60 days 5% NaCl no corrosion, no loss of adhesion at the scribe line |

The above film properties demonstrated sufficient coatings performance for aerospace applications as a primer. The following Table I indicates that the coating of Example 81 is useful in some bonding applications. Deficiencies only appear in the higher temperature bonded applications and to a lesser degree in metal to metal peel tests.

The primer coating of Example 81 was used with structural adhesive films comprising modified epoxy adhesives sold under the trademark FM by American Cyanamid Company. These adhesive films are available on polyester carriers which may be peeled from the adhesive to deposit the latter on the primer-coated substrate. The primer coatings of the invention were compared against a known corrosion-inhibiting, organic solvent based spray primer sold under the trademark BR-127 by American Cyanamid Company. The bonded structures were prepared as in Example 81 that, for the control samples, the known primer coating was subsittuted for the primer coating in accordance with the invention, and the samples were then subjected to identical tests. The results are summarized in Table I.

TABLE I

Bonded Properties - Adhesive and Primer System

| Adhesive: | FM[R] 73 Adhesive Film (1) | | FM[R] 300 Adhesive Film (2) | |
|---|---|---|---|---|
| Primer Coating: | Example 81 | Control BR[R] 127 (3) | Example 81 | Control BR[R] 127 (3) |
| **Test: Lap Shear** | | | | |
| Temperature | PSI at Failure (Failure Mode (4)) | | | |
| Room Temp. | 5910 (C) | 5693 (C) | 4950 (C) | 5350 (C) |
| 180°F | 3975 (C/A) | 4180 (C) | - | - |
| 250°F | 785 (A) | 1900 (C) | - | - |
| 300°F | - | - | 1290 (C/A) | 2600 (C) |
| Room Temp. after 80 hrs. @ 350°F. | - | - | 910 (C/A) | 3300 (C) |
| **Test: Boeing Peel Test** | | | | |
| Temperature | In.-Lb./In.-Width at Failure (Failure Mode (4)) | | | |
| Room Temp. | 50 (C/A) | 69 (C) | 44 (TC/A) | 54 (C) |
| -67°F | 34 (C/A) | 58 (C) | 22 (C/A) | 35 (C/A) |

(1) FM-73 Adhesive Film is a 250°F (121.1°C) curing aerospace adhesive.
(2) FM-300 Adhesive Film is a 350°F (176.7°C) curing aerospace adhesive.
(3) BR-127 primer is a 250°F (121.1°C) curing organic solvent based spray primer for aerospace applications.
(4) Failure Mode: C=Cohesive, A=Adhesive to metal; TC=Thin Cohesive.

NOTE: Values in psi are averages of two or more test specimens, in all Tables.

EXAMPLE 82

Diethylenetriamine in the amount of 618 grams (6 moles) was added to a suitable reactor. 1836 grams (18 moles) of propylene carbonate, which amount comprises 6 moles in excess of the stoichimoetric amount, was slowly added to the reactor under a nitrogen blanket while the temperature of the reactants was maintained at 15 to 20°Cby ice bath cooling. After complete addition, the mixture was stirred 8 hours at room temperature. The resulting product solution comprised diethylenetriamine bishydroxypropyl carbamate and was 75.2% solids in propylene carbonate (theory 75% solids), had 2.51 meq/g secondary amine

(theory 2.45 meq/g at 75.2% solids), and gave characteristic bands in the infrared for the hydroxypropyl carbamate group.

EXAMPLE 83

Novalac Based Hydroxyalkyl Carbamate Electrocoating Resin

A. A self-cross-linking cathodic electrocoating composition based upon a novalac-hydroxyalkyl carbamate resin which shows good good high temperature bonded properties was prepared from the following ingredients:

| | Parts by Weight | Equivalents | Solids |
|---|---|---|---|
| $DEN^R$ 485* | 528.9 | 3.00 | 528.9 |
| Methyl isobutyl ketone (MIBK) | 100.0 | - | - |
| N-methylaniline | 175.4 | 1.64 | 175.4 |
| Carbamate-containing Amine of Example 82 | 484.8 | 1.22 | 364.6 |
| Butyl Cellosolve** | 143.2 | - | - |
| Styrene Oxide | 21.4 | 0.18 | 21.4 |

*Dow Chemical Co. Epoxy Novalac Resin
**Monobutyl ether of ethylene glycol

B. The DEN 485 and MIBK were charged under nitrogen into a suitable reactor having a decanting trap in the distillate return line. The mixture was heated to reflux with stirring in order to remove any water present. After cooling to 100°C, the N-methylaniline was charged in 40 parts of the butyl Cellosolve and the stirred mixture was heated and held at 140 to 145°C for 2 hours. After this period the mixture was cooled to 90°C and the carbamate-containing amine of Example 82 and the remainder of the butyl Cellosolve was added. This mixture was stirred and heated at 90°C for 2.5 hours and then the styrene oxide was added and the temperature held at 90 to 100°C for 2 hours further. The analysis for the final electrocoating resin showed 1.81 meq/g amine. The resin was 75% solids.

C. An electrocoating bath was prepared by combining 300 parts of the novalac-hydroxypropyl carbamate electrocoating resin of part B of this Example with 6.83 parts of 90.8% formic acid and 4.62 parts of dibutyltindilaurate (a urethane catalyst). 2046 Parts of deionized water was slowly added while rapidly mixing with a Cowels stirrer to produce a bath containing approximately 10% solids. The electrocoating bath had a pH of 4.4, a conductivity of 600 micromho $cm^{-1}$, and a rupture voltage of 210 volts.

EXAMPLE 84

The electrodeposition bath of Example 83 was applied to a substrate comprising a 2024T3 Phosanodized bare aluminum member at a deposition voltage of 75 V applied for 20 seconds. The resulting deposited coating was cured by heating at 347°F (175°C) for 20 minutes. The resultant cured film had the following properties:

| | |
|---|---|
| Film Thickness, mil: | 0.35-0.4 |
| Appearance: | glossy, slight orange peel |
| Pencil Hardness: | 7H |
| MEK rubs: | 300 + |
| Reverse Impact, in-lbs: | 40 + on 0.02 inch thick substrate |
| Skydrol Resistance: | Pass |
| Salt Spray Resistance: | Pass |

The following Table II shows the results of comparative tests similar to those whose results are reported in Table I above, for the primer coating of Example 84.

TABLE II

Bonded Properties - Adhesive and Primer System

| Adhesive: | FM$^R$ 73 Adhesive Film(1) | | FM$^R$ 300 Adhesive Film(1) | |
|---|---|---|---|---|
| Primer Coating: | Example 84 | Control BR$^R$ 127(1) | Example 84 | Control BR$^R$ 127(1) |
| | Test: Lap Shear | | | |
| | PSI at Failure (Failure Mode(1)) | | | |
| Temperature | | | | |
| Room Temp. | 5600 (C) | 5400 (C) | 3600 (C/A) | 5200 (C) |
| 250°F | 1500 (C) | 1600 (C) | - | - |
| 300°F | - | - | 2600 (C) | 2800 (C) |
| Room Temp. after 350°F aging for: | | | | |
| 80 hours | - | - | No change | 4800 (C) |
| 240 hours | - | - | 2000 (C) | 2300 (C/A) |

(1) As in Table I.

EXAMPLE 85

A. A self-cross-linking cathodic electrocoating composition based upon a structurally mixed aromatic novalac bisphenol-A hydroxypropyl carbamate resin which gave a further improvement in high temperature bonded properties was prepared from the following ingredients:

| | Parts by Weight | Equivalent | Solids |
|---|---|---|---|
| Epi-Rez$^R$ SU-8* | 446.0 | 2.00 | 446.0 |
| MIBK | 100.0 | - | - |
| N-methylaniline | 120.6 | 1.13 | 120.6 |
| Carbamate-Containing Amine of Example 82 | 329.2 | 0.83 | 247.6 |
| Butyl Cellosolve | 95.2 | - | - |
| Styrene Oxide | 16.5 | 0.14 | 16.5 |

*Celanese Corporationm structurally mixed aromatic novalac bisphenol-A Epoxy Resin.

B. Substantially the same preparation as used in part B of Example 83 was repeated, except that Epi-Rez$^R$ SU-8 was used in place of the DEN-485 of Example 83 and the amounts of the other reagents were adjusted as tabulated above. The analysis for the final electrocoating resin was 1.73 meq/g amine. The electrocoating resin was 75% solids.

C. An electrocoating bath was prepared by combining 350.0 parts of the electrocoating resin of part B of this Example with 7.62 parts of 90.8% formic acid and 5.39 parts of dibutyltindilaurate. 2385 Parts of deionized water was slowly added while rapidly mixing with a Cowels stirrer to produce a bath containing approximately 10% solids. The electrocoating bath had a pH of 4.1, a conductivity of 550 micromho $cm^{-1}$, and a rupture voltage of 220 volts.

EXAMPLE 86

The electrodepositon bath of Example 83 was applied to a substrate comprising a 2024T3 Phosanodized bare aluminum member at a deposition voltage of 75 V applied for 20 seconds. The resulting deposited coating was cured by heating at 175°C (347°F) for 20 minutes. The resultant cured film had the following properties:

| | |
|---|---|
| Film Thickness, mil: | 0.35-0.4 |
| Appearance: | glossy, slight orange peel |
| Pencil Hardness: | 7H |
| MEK rubs | 300 + |
| Reverse Impact, in-lbs: | 40 + on 0.02 inch thick substrate |
| Skydrol Resistance: | Pass |
| Salt Spray Resistance: | Pass |

The following Table III shows the results of comparative tests similar to those whose results are reported in the above Tables, for the primer coating of Example 86.

TABLE III

Bonded Properties - Adhesive and Primer System

| Adhesive: | FM^R 73 (1) | | FM^R 300 (1) | |
|---|---|---|---|---|
| Primer Coating: | Example 86 | Control BR^R 127 (1) | Example 86 | Control BR^R 127 (1) |
| Test: Lap Shear | | | | |
| Temperature | PSI at Failure (Failure Mode (1)) | | | |
| Room Temp. | 5100 (C) | 5400 (C) | 3850 (C/A) | 5200 (C) |
| 250°F | 1575 (C) | 1600 (C) | - | - |
| 300°F | - | - | 3000 (C) | 2800 (C) |
| Room Temp. after 350°F aging for: | | | | |
| 80 hours | - | - | No change | 4800 (C) |
| 240 hours | - | - | 2900 (C) | 2300 (C/A) |

(1) As in Table I.

The foregoing Examples 81-86 illustrate preparation and utilization of hydrophobic cathodic electrodeposited coatings in the present invention. The following Examples similarly illustrate the preparation and utilization of hydrophilic, water based coatings.

EXAMPLE 87

A hydroxyalkyl carbamate-containing amine for use in the following Examples 88 and 89 was prepared using the exact same ratio of reactants and the same procedure as in Example 82 except methanol was utilized as a solvent (40% by weight). After complete reaction (8 hours at room temperature) the methanol was removed by use of the water pump vacuum and with steam bath heating. The resulting product solution comprised diethylenetriamine bishdroxypropyl carbamate and was 73% solids in propylene carbonate (theroy 75% solids), had 2.16 meq/g secondary amine (theory 2.37 meq/g at 73% solids), and gave characteristic bands in the infrared for the hydroxypropyl carbamate group.

EXAMPLE 88

A. A self-cross-linking bishpenol-A hydroxypropyl carbamate resin which can be reduced with water to at least 15% solids as a clear solution with only 20% by total composition weight of cosolvents was prepared from the following ingredients:

| | Parts by Weight | Equivalents | Solids |
|---|---|---|---|
| EPON 828* | 150.9 | 0.82 | 150.9 |
| Carbamate-containing Amine of Example 87 | 380.0 | 0.82 | 277.4 |

*Shell Chemical Co. reaction product of epichlorohydrin and BP-A

The EPON 828 and the carbamate-containing amine of Example 87 were added to a suitable reactor under nitrogen equipped with a Cowels high speed stirrer. Upon stirring the temperature was allowed to reach 100°C (heat of exotherm) and was then maintained at this temperature by external cooling for one hour. After this, the mixture was stirred and heated at 70°C for 4 hours further. The final product had a solids content of 80%.

B. A sprayable aqueous composition was made up by mixing 191.4 parts of the resin obtained in part A of this Example with 191.4 parts of deionized water and 15.3 parts of ethylene glycol monobutyl ether cosolvent. The resulting clear solution contained only 13.5% by total weight organic cosolvents and was 38.5% solids. To this solution was added 14.5 parts of aqueous 1 Molar tetrabutyl ammonium hydroxide catalyst and the contents were well stirred. This composition was applied, by spraying, to aluminum panels. The panels were baked at 250°F (120°C) for 20 minutes and showed film thickness of 0.3 to 0.4 mil after cure. The coatings were smooth flossy, had 4H pencil hardness, passed 40 in-lb reverse impact test, and resisted greater than 300 water and methyl ethyl ketone (MEK) double rubs.

EXAMPLE 89

The composition of Example 88A was applied by spraying to a substrate comprising a 2024T3 Phosanodized bare aluminum member. The resulting deposited coating was cured by heating at 250°F (120°C) for 20 minutes. The resultant cured film had the following properties:

| | |
|---|---|
| Film Thickness, mil: | 0.25 - 0.3 |
| Appearance: | smooth, glossy |
| Pencil Hardness: | 4H |
| MEK rubs: | 300 + |
| Water rubs: | 300 + |
| Reverse Impact, in-lbs: | 40 + on 0.02 inch thick substrate |
| Skydrol Resistance: | Pass |
| Salt Spray Resistance: | Pass |

The following Table shows the results of comparative tests for the primer coating of Example 88.

TABLE IV

Bonded Properties - Adhesive and Primer System

| Adhesive: | $FM^R$ 73(1) | | $FM^R$ 300(1) | |
|---|---|---|---|---|
| Primer Coating: | Coating of Example 88 | Control $BR^R$ 127(1) | Coating of Example 88 | Control $BR^R$ 127(1) |
| | Test: Lap Shear | | | |
| Temperature | PSI at Failure (Failure Mode(1)) | | | |
| Room Temp. | 5500 (C) | 5685 (C) | 5400 (C) | 5350 (C) |
| 180°F | 4400 (C) | 4180 (C) | - | - |
| 250°F | 1700 (C) | 1900 (C) | - | - |
| 300°F | - | - | 1900 (C/A) | 2600 (C) |

TABLE IV (Continued)

| | | | | |
|---|---|---|---|---|
| Room Temp. Lap Shear after: | | | | |
| 3 days water boil | 4200 (C) | 4640 (C) | 4800 (C/A) | - |
| 10 days salt spray | 5700 (C) | 5770 (C) | 5600 (C) | 4330 (C) |
| 350°F Heat Aging: | | | | |
| for 80 hours | - | - | 4800 (C) | 4900 (C) |
| for 200 hours | - | - | 1200 (A) | 2800 (C) |
| | Test: Boeing M/M Peel | | | |
| | In. Lb./In.-Width (Failure Mode (1)) | | | |
| Room Temp. | 75 (C) | 73 (C) | 45 (C) | 54 (C) |
| -67°F | 58 (C) | 58 (C) | 27 (C/A) | 38 (C/A) |
| 10 days salt spray | 70 (C) | 75 (C) | 40 (C/A) | 56 (C) |

(1) As in Table 1

EXAMPLE 90

A. A self-cross-linking novalac hydroxypropyl carbamate resin which can be reduced with water to at least 12% soldis as a clear soltuion with 20% by total weight of cosolvent was prepared from the following ingredients:

| | Parts by Weight | Equivalents | Solids |
|---|---|---|---|
| EPN 1139* | 128.3 | 0.75 | 128.3 |
| Carbamate-containing Amine of Example 87 | 347.3 | 0.75 | 253.5 |

*Ciba Geigy Co. reaction product of phenol-formaldehyde condensate with epichlorohydrin

The EPN 1139 and carbamate-containing amine of Example 87 were reacted in the same manner as described in Example 88A controlling the exotherm by external cooling when necessary. The final product has a solids content of 80%.

B. A sprayable aqueous composition was prepared by dissolving 100 parts of the novalac-hydroxypropyl carbamate of part A of this Example in 100 parts of deionized water and 15.4 parts of 1 Molar tetrabutyl ammonium hydroxide catalyst. The resulting clear solution contained only 9% organic cosolvent and was 39% solids. Aluminum panels were sprayed and then baked at 250°F for 20 minutes. The resulting cured coatings were 0.5-0.6 mil thick, had 4H pencil hardness, passed 40 in-lb reverse impact, were smooth and glossy, and resisted greater than 300 MEK and water double rubs.

EXAMPLE 91

The composition of Example 90 was applied by spraying to a substrate comprising a 2024T3 Phosanodized bare aluminum member. The resulting deposited coating was cured by heating at 250°F (121°C) for 20 minutes. The resultant cured film had the following properties;

| | |
|---|---|
| Film Thickness, mil: | 0.25 - 0.3 |
| Appearance: | smooth, glossy |
| Pencil Hardness: | 5H |
| MEK rubs: | 300 + |
| Water rubs: | 300 + |
| Reverse Impace, in-lbs: | 40 + on 0.02 inch thick substrate |
| Skydrol Resistance: | Pass |
| Salt Spray Resistance: | Pass |

The following Table shows the results of comparative tests for the primer coating of Example 92.

TABLE V

Bonded Properties - Adhesive and Primer System

| Adhesive: | $FM^R$ 73(1) | | $FM^R$ 300(1) | |
|---|---|---|---|---|
| Primer Coating: | Example 91 | Control $BR^R$ 127(1) | Example 91 | Control $BR^R$ 127(1) |
| **Test: Lap Shear** | | | | |
| Temperature | PSI at Failure (Failure Mode(1)) | | | |
| Room Temp. | 6100 (C) | 5950 (C) | 5600 (C) | 5400 (C) |
| 250°F | 2200 (C) | 2300 (C) | - | - |
| 300°F | - | - | 2100 (C/A) | 3100 (C) |
| Room Temp. Lap Shear after: | | | | |
| 350°F Heat Aging: | | | | |
| at 80 hours | - | - | 4400 (C) | 4900 (C) |
| at 200 hours | - | - | 1900 (A) | 2800 (C) |
| **Test: Boeing M/M Peel** | | | | |
| | In. Lb./In.-Width (Failure Mode(1)) | | | |
| Room Temp. | 69 (C) | 66 (C) | 32 (C) | 35 (C) |
| -67°F | 50 (C/A) | 56 (C) | 27 (TC) | 27 (TC) |

(1) As in Table I.

EXAMPLE 92

A. A self-cross-linking tris(hydroxyphenyl)methane based (THPM) hydroxypropyl carbamate resin which shows excellent bonded properties is described below. The resin can be reduced with water to at least 10% solids as a clear solution with 20% by total weight of cosolvent. This resin was prepared from the following ingredients:

| | Parts by Weight | Equivalent | Solids |
|---|---|---|---|
| XD 7342.00L* | 98.0 | 0.594 | 98.0 |
| Carbamate-containing Amine of Example 82 | 236.7 | 0.594 | 178.0 |

*Dow Chemical Company - a THPM based epoxy resin

The XD 7342.00L and the carbamate-containing amine of Example 82 were added under nitrogen to a suitable reactor equipped with a Cowels high speed stirrer. Upon stirring all of the XD 7342.00L dissolved at 60°C (heat of shear and exotherm) and the reactor temperature reached 80°C before the

exotherm subsided. Thereafter, the mixture was stirred and heated at 90°C for 2 hours. The final product had a solids content of 82%.

B. A sprayable aqueous composition was prepared by mixing 316.8 parts of the resin of part A of this Example with 485.3 parts of deionized water and 66.9 parts of butyl Cellosolve cosolvent. The resulting clear solution contained 14% by total weight organic cosolvents and was 30% solids. To this solution was added 19.5 parts of 40% aqueous tetrabutyl ammonium hydroxide catalyst (3% on a solids to solids weight basis) and the contents were well stirred.

C. The composition of part B of this Example was applied by spraying to a substrate comprising a 2024T3 Phosanodized bare aluminum member. The resultant deposited coating was cured by heating at 250°F (121°C) for 20 minutes. The resultant cured film had the following properties:

| | |
|---|---|
| Film Thickness, mil: | 0.25 - 0.3 |
| Appearance: | smooth, glossy |
| Pencil Hardness: | 5H |
| MEK Rubs: | 300+ |
| Water Rubs: | 300+ |
| Reverse Impact, in-lbs: | 40+ on 0.02 inch thick substrate |
| Skydrol Resistance: | Pass |
| Salt Spray Resistance: | Pass |

The following Table shows the results of comparative tests for the primer coating of Example 92.

## TABLE VI

**Bonded Properties - Adhesive and Primer System**

| Adhesive: | FM[R] 73[(1)] | | FM[R] 300[(1)] | |
|---|---|---|---|---|
| Primer Coating: | Example 91 | Control BR[R] 127[(1)] | Example 91 | Control BR[R] 127[(1)] |
| **Test: Lap Shear** | | | | |
| Temperature | PSI at Failure (Failure Mode[(1)]) | | | |
| Room Temp. | 5880 (C) | 5810 (C) | 5360 (C) | 5340 (C) |
| 180°F | 4300 (C) | 4000 (C) | - | - |
| 250°F | 1290 (C/A) | 1580 (C/A | - | - |
| 300°F | - | - | 2350 (C) | 2700 (C) |
| **Test: Boeing M/M Peel** | | | | |
| | In. Lb./In.-Width | | | |
| Room Temp. | 71 (C) | 70 (C) | 43 (C) | 36 (C) |
| -67°F | 54 (C) | 57 (C) | 31 (C) | 33 (C) |

To summarize the above, the present invention refers to the following aspects.

According to a first aspect, there is described a hydroxyalkyl carbamate-containing amine having the formula:

$$R\text{-}NH\text{-}\underset{\underset{O}{\parallel}}{C}\text{-}O\text{-}\underset{\underset{R_1}{|}}{CH}\text{-}(\underset{\underset{R_3}{|}}{CH})_n\text{-}\underset{\underset{R_2}{|}}{CH}\text{-}OH$$

wherein R is an organic moiety having at least one unreacted secondary amine group, each of $R_1$, $R_2$ and $R_3$ is independently H, or a $C_1$ to $C_{20}$ alkyl, cycloalkyl or alkyl aromatic moiety or any of the foregoing carbon-containing moieties containing one or more heteroatoms, and n is 0 or 1.

A second aspect refers to a self-cross-linkable polymer containing at least two hydroxyalkyl carbamate groups per molecule.

A third aspect is a coating composition comprising:

(a) an hydroxyalkyl carbamate compound of the formula

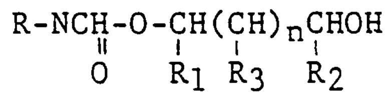

wherein R is a $C_1$ to $C_{20}$ organic moiety which may contain one or more constituents selected from the class consisting of heteroatoms and hydroxyl groups, and each of $R_1$, $R_2$ and $R_3$ is independently H or $CH_3$;

(b) an amino cross-linker;

(c) a polymer containing active sites which, at elevated temperatures, are reactive with the amino cross-linker (b); and

(d) optionally, an acid catalyst;

the compound (a), the cross-linker (b) and the polymer (c) being stable relative to each other in the composition while at ambient temperature, and reactive at elevated temperature.

A fourth aspect deals with a thermosettable coating composition comprising:

(a) a polymer selected from the class consisting of one or one or more of hydroxy group-containing polyurethane, hydroxy group-containing polyurea and hydroxy group-containing polyurethane/polyurea polymers;

(b) an amino cross-linker; and

(c) optionally, an acid catalyst;

wherein the polymer (a) is the reaction product of one or more reaction routes selected from the class consisting of (i) self-condensation of a poly-hydroxyalkyl carbamate compound, (ii) condensation of a poly-hydroxyalkyl carbamate compound with a polyol, and (iii) condensation of a polyhydroxyalkyl carbamate compound with a polyamine.

Specifically, the fourth aspect relates to a thermosettable coating composition comprising:

(a) a polymer selected from the class consisting of one or one or more of hydroxy group-containing polyurethane, hydroxy group-containing polyurea and hydroxy group-containing polyurethane/polyurea polymers;

(b) an amino cross-linker, which is selected from the class consisting of one or more of urea-formaldehyde, melamine formaldehyde, glycoluril-formaldehyde and benzoguanamine formaldehyde resins which have been at least partially alkylated and at least partially methylolated; and

(c) optionally, an acid catalyst;

wherein the polymer (a) is the reaction product of one or more reaction routes selected from the class consisting of (i) self-condensation of poly-hydroxyalkyl carbamate compound, (ii) condensation of a poly-hydroxyalkyl carbamate compound with a polyol, and (iii) condensation of polyhydroxyalkyl carbamate compound with a polyamine, said polyhydroxyalkyl carbamate compound having the formula:

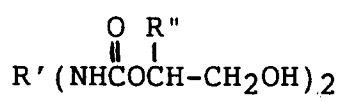

wherein each of R' and R'' is the same or different and comprises a suitable organic moiety.

According to a preferred embodiment of this aspect the composition further includes (d) a solvent which is compatible with components (a) - (c).

In a still further preferred embodiment of the composition of the fourth aspect, the polyhydroxyalkyl carbamate is hexamethylene bis(hydroxypropyl) carbamate.

The acid catalyst of the fourth aspect is preferably selected from the class consisting of one or more of Lewis or Bronsted acids.

The fourth aspect further comprises a method of forming a coating on a substrate comprising

(a) applying to the substrate a coating composition comprising:

(1) a polymer selected from the class consisting of one or more of hydroxy group-containing polyurethane, hydroxy group-containing polyurea and hydroxy group-containing polyurethane/polyurea polymers;

2) an amino cross-linker, which is selected from the class consisting of one or more of urea-formaldehyde, melamine formaldehyde, glycoluril-formaldehyde and benzoguanamine formaldehyde

resins which have been at least partially alkylated and at least partially methylolated; and

3) optionally, an acid catalyst; and

4) optionally, a solvent; and

b) heating the applied composition at an elevated temperature and for a time sufficient to cure it;

wherein the polymer (1) is the reaction product of one or more reaction routes selected from the class consisting of (i) self-condensation of a poly-hydroxyalkyl carbamate compound (ii) condensation of a poly-hydroxyalkyl carbamate compound with a polyol, and (iii) condensation of a polyhydroxyalkyl carbamate compound with a polyamine, said polyhydroxyalkyl carbamate compound having the formula:

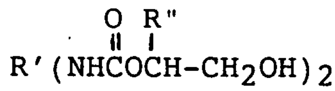

wherein each of R' and R" is the same or different and comprises a suitable organic moiety.

It is preferred that the applied composition is heated at a temperature of from about 93 °C to 204 °C (about 200 ° to about 400 °F).

A fifth aspect refers to a thermosettable composition comprising:

(a) a cross-linking agent containing at least two hydroxyalkyl carbamate groups;

(b) optionally, a cross-linking catalyst; the cross-linking agent (a) and the polymer (b) being stable relative to each other in the composition at ambient temperature and reactive with each other at elevated temperature.

A sixth aspect deals with a cathodically electrodepositable, self-cross-linkable polymer containing hydroxyalkyl carbamate groups and one or more tertiary amine groups per molecule.

A seventh aspect is directed to a cathodic electrocoating composition comprising:

(a) a hydrophobic cross-linking agent having at least two carbamate groups selected from the class consisting of one or both of (i) hydroxyalkyl carbamate groups, and (ii) alkyl carbamate groups obtained by capping the reaction product of a cyclic carbonate and a polyamine;

(b) a water-insoluble and hydrophobic amine group-containing polymer; and

(c) an acid disperser effective to provide cationic groups in the polymer (b) wherein the polymer is rendered water-dispersible; and

(d) optionally, a cross-linking catalyst: the cross-linking agent (a) and the polymer (b) being stable relative to each other in the composition while at ambient temperature, and reactive with each other at elevated temperature.

An eighth aspect refers to a hydrophilic, substantially epoxy-free and self-cross-linkable polymer containing hydroxyalkyl carbamate groups and one or more tertiary amine groups per molecule.

A ninth aspect deals with a self-cross-linkable acrylic polymer containing at least two hydroxyalkyl carbamate groups per molecule.

A tenth aspect relates to a bonding system for bonding workpiece surfaces to one another comprising a primer coating for application to said workpiece surfaces and a structural adhesive for application between the primer coatings, wherein said primer coating comprises a cured polymer having hydroxyalkyl carbamate groups and at least one tertiary amine group thereon and cross-linked by reaction of said hydroxyalkyl carbamate groups.

While the invention has been described with respect to specific preferred embodiments, it will be apparent to one skilled in the art that numerous variations may be made to the embodiments without departing from the spirit and scope of the invention.

## Claims

**1.** A coating composition comprising:

(a) an hydroxyalkyl carbamate compound of the formula

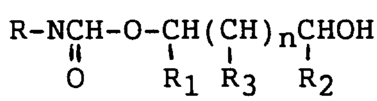

wherein R is a $C_1$ to $C_{20}$ organic moiety which may contain one or more constituents selected from the class consisting of heteroatoms and hydroxyl groups, and each of $R_1$, $R_2$ and $R_3$ is independently H or $CH_3$;
(b) an amino cross-linker;
(c) a polymer containing active sites which, at elevated temperatures, are reactive with the amino cross-linker (b); and
(d) optionally, an acid catalyst;
the compound (a), the cross-linker (b) and the polymer (c) being stable relative to each other in the composition while at ambient temperature, and reactive at elevated temperature.

**2.** A cathodically electrodepositable, self-cross-linkable polymer containing hydroxyalkyl carbamate groups and one or more tertiary amine groups per molecule.

**3.** A cathodic electrocoating composition comprising:
(a) a hydrophobic cross-linking agent having at least two carbamate groups selected from the class consisting of one or both of (i) hydroxyalkyl carbamate groups, and (ii) alkyl carbamate groups obtained by capping the reaction product of a cyclic carbonate and a polyamine;
(b) a water-insoluble and hydrophobic amine group-containing polymer; and
(c) an acid disperser effective to provide cationic groups in the polymer (b) wherein the polymer is rendered water-dispersible; and
(d) optionally, a cross-linking catalyst; the cross-linking agent (a) and the polymer (b) being stable relative to each other in the composition while at ambient temperature, and reactive with each other at elevated temperature.

**4.** A hydrophilic, substantially epoxy-free and self-cross-linkable polymer containing hydroxyalkyl carbamate groups and one or more tertiary amine groups per molecule.